# EUROPEAN PATENT SPECIFICATION

(11) **EP 3 265 571 B1**
(45) Date of publication and mention of the grant of the patent: **13.04.2022**
(21) Application number: 16710113.8
(22) Date of filing: 03.03.2016
(51) Int. Cl.: C12N 15/86, C07K 14/705

(54) **MULTIPLE VECTOR SYSTEM AND USES THEREOF**
MULTIVEKTORSYSTEM UND VERWENDUNGEN DAVON
SYSTÈME DE VECTEURS MULTIPLES ET LEURS UTILISATIONS

(30) Priority: 03.03.2015 US 201562127463 P
(43) Date of publication of application: 10.01.2018
(60) Divisional application: 22160356.6
(73) Proprietor: Fondazione Telethon, 00185 Roma (IT)
(72) Inventor: COLELLA, Pasqualina, 80078 Pozzuoli NA (IT); AURICCHIO, Alberto, 80078 Pozzuoli NA (IT); TRAPANI, Ivana, 80078 Pozzuoli NA (IT)
(74) Representative: D Young & Co LLP
(86) International application number: PCT/EP2016/054602
(87) International publication number: WO 2016/139321

(56) References cited:
- WO-A1-2014/170480
- TRAPANI I ET AL: "Improved dual AAV vectors with reduced expression of truncated proteins are safe and effective in the retina of a mouse model of Stargardt disease", HUMAN MOLECULAR GENETICS, vol. 24, no. 23, 29 September 2015 (2015-09-29), pages 6811-6825, XP055254224, gb ISSN: 0964-6906, DOI: 10.1093/hmg/ddv386
- TRAPANI I ET AL: "Effective delivery of large genes to the retina by dual AAV vectors", EMBO MOLECULAR MEDICINE, vol. 6, no. 2, 1 December 2013 (2013-12-01), pages 194-211, XP055127533, ISSN: 1757-4676, DOI: 10.1002/emmm.201302948
- COLELLA P ET AL: "Efficient gene delivery to the cone-enriched pig retina by dual AAV vectors", GENE THERAPY, vol. 21, no. 4, 1 April 2014 (2014-04-01), pages 450-456, XP055270423, GB ISSN: 0969-7128, DOI: 10.1038/gt.2014.8
- DYKA F M ET AL: "Dual Adeno-Associated Virus Vectors Result in Efficient In Vitro and In Vivo Expression of an Oversized Gene, MYO7A", HUMAN GENE THERAPY METHODS, vol. 25, no. 2, 1 April 2014 (2014-04-01), pages 166-177, XP055270425, ISSN: 1946-6536, DOI: 10.1089/hgtb.2013.212
- CHO S ET AL: "A degron created by SMN2 exon 7 skipping is a principal contributor to spinal muscular atrophy severity", GENES AND DEVELOPMENT., vol. 24, no. 5, 1 March 2010 (2010-03-01), pages 438-442, XP055270460, US ISSN: 0890-9369, DOI: 10.1101/gad.1884910

### SEQUENCE LISTING

<110> Fondazione Telethon
<120> Multiple vector system and uses thereof
<130> PCT 129062
<150> US62/127,463
   <151> 2015-03-03
<160> 78
<170> PatentIn version 3.5
<210> 1
   <211> 16
   <212> PRT
   <213> Artificial Sequence
<220>
   <223> synthetic
<400> 1
   Ala Cys Lys Asn Trp Phe Ser Ser Leu Ser His Phe Val Ile His Leu
   1               5                   10                  15
<210> 2
   <211> 35
   <212> PRT
   <213> Artificial Sequence
<220>
   <223> synthetic
<400> 2
   Ser Leu Ile Ser Leu Pro Leu Pro Thr Arg Val Lys Phe Ser Ser Leu
   1               5                   10                  15
   Leu Leu Ile Arg Ile Met Lys Ile Ile Thr Met Thr Phe Pro Lys Lys
               20                  25                  30
   Leu Arg Ser
           35
<210> 3
   <211> 16
   <212> **PRT**
   <213> **Artificial** Sequence
<220>
   <223> synthetic
<400> 3
   Phe Tyr Tyr Pro Ile Trp Phe Ala Arg Val Leu Leu Val His Tyr Gln
   1               5                   10                  15
<210> 4
   <211> 46
   <212> **PRT**
   <213> **Artificial** Sequence
<220>
   <223> synthetic
<400> 4
   Ser Asn Pro Phe Ser Ser Leu Phe Gly Ala Ser Leu Leu Ile Asp Ser
   1               5                   10                  15
   Val Ser Leu Lys Ser Asn Trp Asp Thr Ser Ser Ser Ser Cys Leu Ile
               20                  25                  30
   Ser Phe Phe Ser Ser Val Met Phe Ser Ser Thr Thr Arg Ser
           35                  40                  45
<210> 5
   <211> 39
   <212> PRT
   <213> Artificial Sequence
<220>
   <223> synthetic
<400> 5
   Cys Arg Gln Arg Phe Ser Cys His Leu Thr Ala Ser Tyr Pro Gln Ser
   1               5                   10                  15
   Thr Val Thr Pro Phe Leu Ala Phe Leu Arg Arg Asp Phe Phe Phe Leu
               20                  25                  30
   Arg His Asn Ser Ser Ala Asp
           35
<210> 6
   <211> 46
   <212> **PRT**
   <213> **Artificial** Sequence
<220>
   <223> synthetic
<400> 6
   Gly Ala Pro His Val Val Leu Phe Asp Phe Glu Leu Arg Ile Thr Asn
   1               5                   10                  15
   Pro Leu Ser His Ile Gln Ser Val Ser Leu Gln Ile Thr Leu Ile Phe
               20                  25                  30
   Cys Ser Leu Pro Ser Leu Ile Leu Ser Lys Phe Leu Gln Val
           35                  40                  45
<210> 7
   <211> 39
   <212> PRT
   <213> Artificial Sequence
<220>
   <223> synthetic
<400> 7
   Asn Thr Pro Leu Phe Ser Lys Ser Phe Ser Thr Thr Cys Gly Val Ala
   1               5                   10                  15
   Lys Lys Thr Leu Leu Leu Ala Gln Ile Ser Ser Leu Phe Phe Leu Leu
               20                  25                  30
   Leu Ser Ser Asn Ile Ala Val
           35
<210> 8
   <211> 45
   <212> **PRT**
   <213> **Artificial** Sequence
<220>
   <223> synthetic
<400> 8
   Pro Thr Val Lys Asn Ser Pro Lys Ile Phe Cys Leu Ser Ser Ser Pro
   1               5                   10                  15
   Tyr Leu Ala Phe Asn Leu Glu Tyr Leu Ser Leu Arg Ile Phe Ser Thr
               20                  25                  30
   Leu Ser Lys Cys Ser Asn Thr Leu Leu Thr Ser Leu Ser
           35                  40                  45
<210> 9
   <211> 30
   <212> **PRT**
   <213> **Artificial** Sequence
<220>
   <223> synthetic
<400> 9
   Ser Asn Gln Leu Lys Arg Leu Trp Leu Trp Leu Leu Glu Val Arg Ser
   1               5                   10                  15
   Phe Asp Arg Thr Leu Arg Arg Pro Trp Ile His Leu Pro Ser
               20                  25                  30
<210> 10
   <211> 50
   <212> PRT
   <213> Artificial Sequence
<220>
   <223> synthetic
<400> 10
   Ser Ile Ser Phe Val Ile Arg Ser His Ala Ser Ile Arg Met Gly Ala
   1               5                   10                  15
   Ser Asn Asp Phe Phe His Lys Leu Tyr Phe Thr Lys Cys Leu Thr Ser
               20                  25                  30
   Val Ile Leu Ser Lys Phe Leu Ile His Leu Leu Leu Arg Ser Thr Pro
           35                  40                  45
   Arg Val
       50
<210> 11
   <211> 22
   <212> DNA
   <213> Artificial Sequence
<220>
   <223> synthetic
<400> 11
   aggcatagga tgacaaaggg aa 22
<210> 12
   <211> 20
   <212> DNA
   <213> Artificial Sequence
<220>
   <223> synthetic
<400> 12
   ggcattcacc gcgtgcctta 20
<210> 13
   <211> 22
   <212> DNA
   <213> Artificial Sequence
<220>
   <223> synthetic
<400> 13
   agcctatcct ggattacttg aa 22
<210> 14
   <211> 9
   <212> PRT
   <213> Artificial Sequence
<220>
   <223> synthetic
<400> 14
   Ser Trp Asn Phe Lys Leu Tyr Val Met
   1               5
<210> 15
   <211> 14
   <212> **PRT**
   <213> **Artificial** Sequence
<220>
   <223> synthetic
<400> 15
   Met His Ser Trp Asn Phe Lys Leu Tyr Val Met Gly Ser Gly
   1               5                   10
<210> 16
   <211> 48
   <212> DNA
   <213> Artificial Sequence
<220>
   <223> synthetic
<400> 16
   gcctgcaaga actggttcag cagcctgagc cacttcgtga tccacctg 48
<210> 17
   <211> 158
   <212> DNA
   <213> Artificial Sequence
<220>
   <223> synthetic
<400> 17
   aggcatagga tgacaaaggg aacgataggc ataggatgac aaagggaaaa gcttaggcat        60
   aggatgacaa agggaaggta ccagatctgg cattcaccgc gtgccttacg atggcattca       120
   ccgcgtgcct taaagcttgg cattcaccgc gtgcctta                               158
<210> 18
   <211> 102
   <212> **DNA**
   <213> **Artificial** Sequence
<220>
   <223> synthetic
<400> 18
   agcctatcct ggattacttg aacgatagcc tatcctggat tacttgaaaa gcttagccta        60
   tcctggatta cttgaatcac agcctatcct ggattacttg aa                          102
<210> 19
   <211> 42
   <212> DNA
   <213> Artificial Sequence
<220>
   <223> synthetic
<400> 19
   atgcacagct ggaacttcaa gctgtacgtc atgggcagcg gc 42
<210> 20
   <211> 27
   <212> DNA
   <213> Artificial Sequence
<220>
   <223> synthetic
<400> 20
   agctggaact tcaagctgta cgtcatg 27
<210> 21
   <211> 136
   <212> DNA
   <213> Artificial Sequence
<220>
   <223> synthetic
<400> 21
   atgcacagct ggaacttcaa gctgtacgtc atgggcagcg gcggggtacc atgcacagct        60
   ggaacttcaa gctgtacgtc atgggcagcg gcggatgcac agctggaact tcaagctgta       120
   cgtcatgggc agcggc                                                       136
<210> 22
   <211> 77
   <212> DNA
   <213> Artificial Sequence
<220>
   <223> synthetic
<400> 22
   gggatttttc cgatttcggc ctattggtta aaaaatgagc tgatttaaca aaaatttaac 60
   gcgaatttta acaaaat 77
<210> 23
   <211> 77
   <212> **DNA**
   <213> Artificial Sequence
<220>
   <223> synthetic
<400> 23
   gggattttgc cgatttcggc ctattggtta aaaaatgagc tgatttaaca aaaatttaac 60
   gcgaatttta acaaaat 77
<210> 24
   <211> 287
   <212> DNA
   <213> Artificial Sequence
<220>
   <223> synthetic
<400> 24
   ccccgggtgc gcggcgtcgg tggtgccggc ggggggcgcc aggtcgcagg cggtgtaggg 60
   ctccaggcag gcggcgaagg ccatgacgtg cgctatgaag gtctgctcct gcacgccgtg 120
   aaccaggtgc gcctgcgggc cgcgcgcgaa caccgccacg tcctcgcctg cgtgggtctc 180
   ttcgtccagg ggcactgctg actgctgccg atactcgggg ctcccgctct cgctctcggt 240
   aacatccggc cgggcgccgt ccttgagcac atagcctgga ccgtttc 287
<210> 25
   <211> 288
   <212> DNA
   <213> Artificial Sequence
<220>
   <223> synthetic
<400> 25
   cgcagggcag cctctgtcat ctccatcagg gaggggtcca gtgtggagtc tcggtggatc 60
   tcgtatttca tgtctccagg ctcaaagaga cccatgagat gggtcacaga cgggtccagg 120
   gaagcctgca tgagctcagt gcggttccac acataccggg caccctggcg cttcgccagc 180
   cattcctgca ccagattctt cccgtccagc ctggtcccac cttggctgta gtcatctggg 240
   tactcagggt ctggggttcc catgcgaaac atgtactttc ggcctcca 288
<210> 26
   <211> 278
   <212> DNA
   <213> Artificial Sequence
<220>
   <223> synthetic
<400> 26
   gtgatcctag gtggaggccg aaagtacatg tttcgcatgg gaaccccaga ccctgagtac 60
   ccagatgact acagccaagg tgggaccagg ctggacggga agaatctggt gcaggaatgg 120
   ctggcgaagc gccagggtgc ccggtacgtg tggaaccgca ctgagctcat gcaggcttcc 180
   ctggacccgt ctgtgaccca tctcatgggt ctctttgagc ctggagacat gaaatacgag 240
   atccaccgag actccacact ggacccctcc ctgatgga 278
<210> 27
   <211> 82
   <212> DNA
   <213> Artificial Sequence
<220>
   <223> synthetic
<400> 27
   gtaagtatca aggttacaag acaggtttaa ggagaccaat agaaactggg cttgtcgaga 60
   cagagaagac tcttgcgttt ct 82
<210> 28
   <211> 51
   <212> DNA
   <213> Artificial Sequence
<220>
   <223> synthetic
<400> 28
   gataggcacc tattggtctt actgacatcc actttgcctt tctctccaca g 51
<210> 29
   <211> 130
   <212> DNA
   <213> Artificial Sequence
<220>
   <223> synthetic
<400> 29
   ctgcgcgctc gctcgctcac tgaggccgcc cgggcaaagc ccgggcgtcg ggcgaccttt        60
   ggtcgcccgg cctcagtgag cgagcgagcg cgcagagagg gagtggccaa ctccatcact       120
   aggggttcct                                                              130
<210> 30
   <211> 130
   <212> DNA
   <213> Artificial Sequence
<220>
   <223> synthetic
<400> 30
   aggaacccct agtgatggag ttggccactc cctctctgcg cgctcgctcg ctcactgagg        60
   ccgggcgacc aaaggtcgcc cgacgcccgg gctttgcccg ggcggcctca gtgagcgagc       120
   gagcgcgcag                                                              130
<210> 31
   <211> 175
   <212> **DNA**
   <213> Artificial Sequence
<220>
   <223> synthetic
<400> 31
   ctctcccccc tgtcgcgttc gctcgctcgc tggctcgttt gggggggtgg cagctcaaag        60
   agctgccaga cgacggccct ctggccgtcg cccccccaaa cgagccagcg agcgagcgaa       120
   cgcgacaggg gggagagtgc cacactctca agcaaggggg ttttgtaagc agtga            175
<210> 32
   <211> 175
   <212> **DNA**
   <213> **Artificial** Sequence
<220>
   <223> synthetic
<400> 32
   tcactgctta caaaaccccc ttgcttgaga gtgtggcact ctcccccctg tcgcgttcgc 60
   tcgctcgctg gctcgtttgg gggggcgacg gccagagggc cgtcgtctgg cagctctttg 120
   agctgccacc cccccaaacg agccagcgag cgagcgaacg cgacaggggg gagag 175
<210> 33
   <211> 153
   <212> **DNA**
   <213> Artificial Sequence
<220>
   <223> synthetic
<400> 33
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gac 153
<210> 34
   <211> 583
   <212> **DNA**
   <213> Artificial Sequence
<220>
   <223> synthetic
<400> 34
   tagttattaa tagtaatcaa ttacggggtc attagttcat agcccatata tggagttccg 60
   cgttacataa cttacggtaa atggcccgcc tggctgaccg cccaacgacc cccgcccatt 120
   gacgtcaata atgacgtatg ttcccatagt aacgccaata gggactttcc attgacgtca 180
   atgggtggag tatttacggt aaactgccca cttggcagta catcaagtgt atcatatgcc 240
   aagtccgccc cctattgacg tcaatgacgg taaatggccc gcctggcatt atgcccagta 300
   catgacctta cgggactttc ctacttggca gtacatctac gtattagtca tcgctattac 360
   catggtgatg cggttttggc agtacaccaa tgggcgtgga tagcggtttg actcacgggg 420
   atttccaagt ctccacccca ttgacgtcaa tgggagtttg ttttggcacc aaaatcaacg 480
   ggactttcca aaatgtcgta ataaccccgc cccgttgacg caaatgggcg gtaggcgtgt 540
   acggtgggag gtctatataa gcagagctcg tttagtgaac cgt 583
<210> 35
   <211> 133
   <212> **DNA**
   <213> Artificial Sequence
<220>
   <223> synthetic
<400> 35
   gtaagtatca aggttacaag acaggtttaa ggagaccaat agaaactggg cttgtcgaga      60
   cagagaagac tcttgcgttt ctgataggca cctattggtc ttactgacat ccactttgcc     120
   tttctctcca cag                                                        133
<210> 36
   <211> 299
   <212> DNA
   <213> Artificial Sequence
<220>
   <223> synthetic
<400> 36
   ctagtgggcc ccagaagcct ggtggttgtt tgtccttctc aggggaaaag tgaggcggcc      60
   ccttggagga aggggccggg cagaatgatc taatcggatt ccaagcagct caggggattg     120
   tctttttcta gcaccttctt gccactccta agcgtcctcc gtgaccccgg ctgggattta     180
   gcctggtgct gtgtcagccc cgggctccca ggggcttccc agtggtcccc aggaaccctc     240
   gacagggcca gggcgtctct ctcgtccagc aagggcaggg acgggccaca ggcaagggc      299
<210> 37
   <211> 365
   <212> DNA
   <213> Artificial Sequence
<220>
   <223> synthetic
<400> 37
   ctagttatta atagtaatca attacggggt cattagttca tagcccatat atggagttcc      60
   gcgttacata acttacggta aatggcccgc ctggctgacc gcccaacgac ccccgcccat     120
   tgacgtcaat aatgacgtat gttcccatag taacgccaat agggactttc cattgacgtc     180
   aatgggtgga gtatttacgg taaactgccc acttggcagt acatcaagtg tatcatatgc     240
   caagtacgcc ccctattgac gtcaatgacg gtaaatggcc cgcctggcat tatgcccagt     300
   acatgacctt atgggacttt cctacttggc agtacatcta cgtattagtc atcgctatta     360
   ccatg                                                                 365
<210> 38
   <211> 229
   <212> **DNA**
   <213> Artificial Sequence
<220>
   <223> synthetic
<400> 38
   tcgaggtgag ccccacgttc tgcttcactc tccccatctc ccccccctcc ccacccccaa 60
   ttttgtattt atttattttt taattatttt gtgcagcgat gggggcgggg cggggcgagg 120
   cggagaggtg cggcggcagc caatcggagc ggcgcgctcc gaaagtttcc ttttatggcg 180
   aggcggcggc ggcggcggct ctataaaaag cgaagcgcgc ggcgggcgg 229
<210> 39
   <211> 235
   <212> DNA
   <213> Artificial Sequence
<220>
   <223> synthetic
<400> 39
   agcacagtgt ctggcatgta gcaggaacta aaataatggc agtgattaat gttatgatat 60
   gcagacacaa cacagcaaga taagatgcaa tgtaccttct gggtcaaacc accctggcca 120
   ctcctccccg atacccaggg ttgatgtgct tgaattagac aggattaaag gcttactgga 180
   gctggaagcc ttgccccaac tcaggagttt agccccagac cttctgtcca ccagc 235
<210> 40
   <211> 22
   <212> DNA
   <213> Artificial Sequence
<220>
   <223> synthetic
<400> 40
   aaccacacaa cctactacct ca 22
<210> 41
   <211> 102
   <212> DNA
   <213> Artificial Sequence
<220>
   <223> synthetic
<400> 41
   aaccacacaa cctactacct cacgataacc acacaaccta ctacctcaaa gcttaaccac 60
   acaacctact acctcatcac aaccacacaa cctactacct ca 102
<210> 42
   <211> 105
   <212> **DNA**
   <213> **Artificial** Sequence
<220>
   <223> synthetic
<400> **42**
   agcctgatca gcctgcccct gcccacccgg gtgaagttca gcagcctgct gctgatccgg 60
   atcatgaaga tcatcaccat gaccttcccc aagaagctgc ggagc 105
<210> 43
   <211> 48
   <212> DNA
   <213> Artificial Sequence
<220>
   <223> synthetic
<400> 43
   ttctactacc ccatctggtt cgcccgggtg ctgctggtgc actaccag 48
<210> 44
   <211> 138
   <212> DNA
   <213> Artificial Sequence
<220>
   <223> synthetic
<400> 44
   agcaacccct tcagcagcct gttcggcgcc agcctgctga tcgacagcgt gagcctgaag 60
   agcaactggg acaccagcag cagcagctgc ctgatcagct tcttcagcag cgtgatgttc 120
   agcagcacca cccggagc 138
<210> 45
   <211> 117
   <212> **DNA**
   <213> Artificial Sequence
<220>
   <223> synthetic
<400> 45
   tgccggcagc ggttcagctg ccacctgacc gccagctacc cccagagcac cgtgaccccc 60
   ttcctggcct tcctgcggcg ggacttcttc ttcctgcggc acaacagcag cgccgac 117
<210> 46
   <211> 138
   <212> DNA
   <213> Artificial Sequence
<220>
   <223> synthetic
<400> 46
   ggcgcccccc acgtggtgct gttcgacttc gagctgcgga tcaccaaccc cctgagccac 60
   atccagagcg tgagcctgca gatcaccctg atcttctgca gcctgcccag cctgatcctg 120
   agcaagttcc tgcaggtg 138
<210> 47
   <211> 117
   <212> DNA
   <213> Artificial Sequence
<220>
   <223> synthetic
<400> 47
   aacacccccc tgttcagcaa gagcttcagc accacctgcg gcgtggccaa gaagaccctg 60
   ctgctggccc agatcagcag cctgttcttc ctgctgctga gcagcaacat cgccgtg 117
<210> 48
   <211> 135
   <212> **DNA**
   <213> Artificial Sequence
<220>
   <223> synthetic
<400> 48
   cccaccgtga agaacagccc caagatcttc tgcctgagca gcagccccta cctggccttc 60
   aacctggagt acctgagcct gcggatcttc agcaccctga gcaagtgcag caacaccctg 120
   ctgaccagcc tgagc 135
<210> 49
   <211> 90
   <212> DNA
   <213> Artificial Sequence
<220>
   <223> synthetic
<400> 49
   agcaaccagc tgaagcggct gtggctgtgg ctgctggagg tgcggagctt cgaccggacc 60
   ctgcggcggc cctggatcca cctgcccagc 90
<210> 50
   <211> 150
   <212> **DNA**
   <213> Artificial Sequence
<220>
   <223> synthetic
<400> 50
   agcatcagct tcgtgatccg gagccacgcc agcatccgga tgggcgccag caacgacttc 60
   ttccacaagc tgtacttcac caagtgcctg accagcgtga tcctgagcaa gttcctgatc 120
   cacctgctgc tgcggagcac cccccgggtg 150
<210> 51
   <211> 11
   <212> DNA
   <213> Artificial Sequence
<220>
   <223> synthetic
<400> 51
   tgaatgaatg a 11
<210> 52
   <211> 243
   <212> DNA
   <213> Artificial Sequence
<220>
   <223> synthetic
<400> 52
   ttcgagcaga catgataaga tacattgatg agtttggaca aaccacaact agaatgcagt 60
   gaaaaaaatg ctttatttgt gaaatttgtg atgctattgc tttatttgta accattataa 120
   gctgcaataa acaagttaac aacaacaatt gcattcattt tatgtttcag gttcaggggg 180
   agatgtggga ggttttttaa agcaagtaaa acctctacaa atgtggtaaa atcgataagg 240
   atc 243
<210> 53
   <211> 2918
   <212> DNA
   <213> Artificial Sequence
<220>
   <223> synthetic
<400> 53
   atgggcttcg tgagacagat acagcttttg ctctggaaga actggaccct gcggaaaagg 60
   caaaagattc gctttgtggt ggaactcgtg tggcctttat ctttatttct ggtcttgatc 120
   tggttaagga atgccaaccc gctctacagc catcatgaat gccatttccc caacaaggcg 180
   atgccctcag caggaatgct gccgtggctc caggggatct tctgcaatgt gaacaatccc 240
   tgttttcaaa gccccacccc aggagaatct cctggaattg tgtcaaacta taacaactcc 300
   atcttggcaa gggtatatcg agattttcaa gaactcctca tgaatgcacc agagagccag 360
   caccttggcc gtatttggac agagctacac atcttgtccc aattcatgga caccctccgg 420
   actcacccgg agagaattgc aggaagagga attcgaataa gggatatctt gaaagatgaa 480
   gaaacactga cactatttct cattaaaaac atcggcctgt ctgactcagt ggtctacctt 540
   ctgatcaact ctcaagtccg tccagagcag ttcgctcatg gagtcccgga cctggcgctg 600
   aaggacatcg cctgcagcga ggccctcctg gagcgcttca tcatcttcag ccagagacgc 660
   ggggcaaaga cggtgcgcta tgccctgtgc tccctctccc agggcaccct acagtggata 720
   gaagacactc tgtatgccaa cgtggacttc ttcaagctct tccgtgtgct tcccacactc 780
   ctagacagcc gttctcaagg tatcaatctg agatcttggg gaggaatatt atctgatatg 840
   tcaccaagaa ttcaagagtt tatccatcgg ccgagtatgc aggacttgct gtgggtgacc 900
   aggcccctca tgcagaatgg tggtccagag acctttacaa agctgatggg catcctgtct 960
   gacctcctgt gtggctaccc cgagggaggt ggctctcggg tgctctcctt caactggtat 1020
   gaagacaata actataaggc ctttctgggg attgactcca caaggaagga tcctatctat 1080
   tcttatgaca gaagaacaac atccttttgt aatgcattga tccagagcct ggagtcaaat 1140
   cctttaacca aaatcgcttg gagggcggca aagcctttgc tgatgggaaa aatcctgtac 1200
   actcctgatt cacctgcagc acgaaggata ctgaagaatg ccaactcaac ttttgaagaa 1260
   ctggaacacg ttaggaagtt ggtcaaagcc tgggaagaag tagggcccca gatctggtac 1320
   ttctttgaca acagcacaca gatgaacatg atcagagata ccctggggaa cccaacagta 1380
   aaagactttt tgaataggca gcttggtgaa gaaggtatta ctgctgaagc catcctaaac 1440
   ttcctctaca agggccctcg ggaaagccag gctgacgaca tggccaactt cgactggagg 1500
   gacatattta acatcactga tcgcaccctc cgccttgtca atcaatacct ggagtgcttg 1560
   gtcctggata agtttgaaag ctacaatgat gaaactcagc tcacccaacg tgccctctct 1620
   ctactggagg aaaacatgtt ctgggccgga gtggtattcc ctgacatgta tccctggacc 1680
   agctctctac caccccacgt gaagtataag atccgaatgg acatagacgt ggtggagaaa 1740
   accaataaga ttaaagacag gtattgggat tctggtccca gagctgatcc cgtggaagat 1800
   ttccggtaca tctggggcgg gtttgcctat ctgcaggaca tggttgaaca ggggatcaca 1860
   aggagccagg tgcaggcgga ggctccagtt ggaatctacc tccagcagat gccctacccc      1920
   tgcttcgtgg acgattcttt catgatcatc ctgaaccgct gtttccctat cttcatggtg      1980
   ctggcatgga tctactctgt ctccatgact gtgaagagca tcgtcttgga gaaggagttg      2040
   cgactgaagg agaccttgaa aaatcagggt gtctccaatg cagtgatttg gtgtacctgg      2100
   ttcctggaca gcttctccat catgtcgatg agcatcttcc tcctgacgat attcatcatg      2160
   catggaagaa tcctacatta cagcgaccca ttcatcctct tcctgttctt gttggctttc      2220
   tccactgcca ccatcatgct gtgctttctg ctcagcacct tcttctccaa ggccagtctg      2280
   gcagcagcct gtagtggtgt catctatttc accctctacc tgccacacat cctgtgcttc      2340
   gcctggcagg accgcatgac cgctgagctg aagaaggctg tgagcttact gtctccggtg      2400
   gcatttggat ttggcactga gtacctggtt cgctttgaag agcaaggcct ggggctgcag      2460
   tggagcaaca tcgggaacag tcccacggaa ggggacgaat tcagcttcct gctgtccatg      2520
   cagatgatgc tccttgatgc tgctgtctat ggcttactcg cttggtacct tgatcaggtg      2580
   tttccaggag actatggaac cccacttcct tggtactttc ttctacaaga gtcgtattgg      2640
   cttggcggtg aagggtgttc aaccagagaa gaaagagccc tggaaaagac cgagccccta      2700
   acagaggaaa cggaggatcc agagcaccca gaaggaatac acgactcctt ctttgaacgt      2760
   gagcatccag ggtgggttcc tggggtatgc gtgaagaatc tggtaaagat ttttgagccc      2820
   tgtggccggc cagctgtgga ccgtctgaac atcaccttct acgagaacca gatcaccgca      2880
   ttcctgggcc acaatggagc tgggaaaacc accacctt                              2918
<210> 54
   <211> 3945
   <212> **DNA**
   <213> **Artificial** Sequence
<220>
   <223> synthetic
<400> 54
   ctgcgcgctc gctcgctcac tgaggccgcc cgggcaaagc ccgggcgtcg ggcgaccttt       60
   ggtcgcccgg cctcagtgag cgagcgagcg cgcagagagg gagtggccaa ctccatcact      120
   aggggttcct tgtagttaat gattaacccg ccatgctact tatctacgta gccatgctct      180
   aggaagatct tcaatattgg ccattagcca tattattcat tggttatata gcataaatca      240
   atattggcta ttggccattg catacgttgt atctatatca taatatgtac atttatattg      300
   gctcatgtcc aatatgaccg ccatgttggc attgattatt gactagtggg ccccagaagc      360
   ctggtggttg tttgtccttc tcaggggaaa agtgaggcgg ccccttggag gaaggggccg      420
   ggcagaatga tctaatcgga ttccaagcag ctcaggggat tgtctttttc tagcaccttc      480
   ttgccactcc taagcgtcct ccgtgacccc ggctgggatt tagcctggtg ctgtgtcagc 540
   cccgggctcc caggggcttc ccagtggtcc ccaggaaccc tcgacagggc cagggcgtct 600
   ctctcgtcca gcaagggcag ggacgggcca caggcaaggg cgcggccgcc atgggcttcg 660
   tgagacagat acagcttttg ctctggaaga actggaccct gcggaaaagg caaaagattc 720
   gctttgtggt ggaactcgtg tggcctttat ctttatttct ggtcttgatc tggttaagga 780
   atgccaaccc gctctacagc catcatgaat gccatttccc caacaaggcg atgccctcag 840
   caggaatgct gccgtggctc caggggatct tctgcaatgt gaacaatccc tgttttcaaa 900
   gccccacccc aggagaatct cctggaattg tgtcaaacta taacaactcc atcttggcaa 960
   gggtatatcg agattttcaa gaactcctca tgaatgcacc agagagccag caccttggcc 1020
   gtatttggac agagctacac atcttgtccc aattcatgga caccctccgg actcacccgg 1080
   agagaattgc aggaagagga attcgaataa gggatatctt gaaagatgaa gaaacactga 1140
   cactatttct cattaaaaac atcggcctgt ctgactcagt ggtctacctt ctgatcaact 1200
   ctcaagtccg tccagagcag ttcgctcatg gagtcccgga cctggcgctg aaggacatcg 1260
   cctgcagcga ggccctcctg gagcgcttca tcatcttcag ccagagacgc ggggcaaaga 1320
   cggtgcgcta tgccctgtgc tccctctccc agggcaccct acagtggata gaagacactc 1380
   tgtatgccaa cgtggacttc ttcaagctct tccgtgtgct tcccacactc ctagacagcc 1440
   gttctcaagg tatcaatctg agatcttggg gaggaatatt atctgatatg tcaccaagaa 1500
   ttcaagagtt tatccatcgg ccgagtatgc aggacttgct gtgggtgacc aggcccctca 1560
   tgcagaatgg tggtccagag acctttacaa agctgatggg catcctgtct gacctcctgt 1620
   gtggctaccc cgagggaggt ggctctcggg tgctctcctt caactggtat gaagacaata 1680
   actataaggc ctttctgggg attgactcca caaggaagga tcctatctat tcttatgaca 1740
   gaagaacaac atccttttgt aatgcattga tccagagcct ggagtcaaat cctttaacca 1800
   aaatcgcttg gagggcggca aagcctttgc tgatgggaaa aatcctgtac actcctgatt 1860
   cacctgcagc acgaaggata ctgaagaatg ccaactcaac ttttgaagaa ctggaacacg 1920
   ttaggaagtt ggtcaaagcc tgggaagaag tagggcccca gatctggtac ttctttgaca 1980
   acagcacaca gatgaacatg atcagagata ccctggggaa cccaacagta aaagactttt 2040
   tgaataggca gcttggtgaa gaaggtatta ctgctgaagc catcctaaac ttcctctaca 2100
   agggccctcg ggaaagccag gctgacgaca tggccaactt cgactggagg gacatattta 2160
   acatcactga tcgcaccctc cgccttgtca atcaatacct ggagtgcttg gtcctggata 2220
   agtttgaaag ctacaatgat gaaactcagc tcacccaacg tgccctctct ctactggagg 2280
   aaaacatgtt ctgggccgga gtggtattcc ctgacatgta tccctggacc agctctctac 2340
   caccccacgt gaagtataag atccgaatgg acatagacgt ggtggagaaa accaataaga 2400
   ttaaagacag gtattgggat tctggtccca gagctgatcc cgtggaagat ttccggtaca 2460
   tctggggcgg gtttgcctat ctgcaggaca tggttgaaca ggggatcaca aggagccagg 2520
   tgcaggcgga ggctccagtt ggaatctacc tccagcagat gccctacccc tgcttcgtgg 2580
   acgattcttt catgatcatc ctgaaccgct gtttccctat cttcatggtg ctggcatgga 2640
   tctactctgt ctccatgact gtgaagagca tcgtcttgga gaaggagttg cgactgaagg 2700
   agaccttgaa aaatcagggt gtctccaatg cagtgatttg gtgtacctgg ttcctggaca 2760
   gcttctccat catgtcgatg agcatcttcc tcctgacgat attcatcatg catggaagaa 2820
   tcctacatta cagcgaccca ttcatcctct tcctgttctt gttggctttc tccactgcca 2880
   ccatcatgct gtgctttctg ctcagcacct tcttctccaa ggccagtctg gcagcagcct 2940
   gtagtggtgt catctatttc accctctacc tgccacacat cctgtgcttc gcctggcagg 3000
   accgcatgac cgctgagctg aagaaggctg tgagcttact gtctccggtg gcatttggat 3060
   ttggcactga gtacctggtt cgctttgaag agcaaggcct ggggctgcag tggagcaaca 3120
   tcgggaacag tcccacggaa ggggacgaat tcagcttcct gctgtccatg cagatgatgc 3180
   tccttgatgc tgctgtctat ggcttactcg cttggtacct tgatcaggtg tttccaggag 3240
   actatggaac cccacttcct tggtactttc ttctacaaga gtcgtattgg cttggcggtg 3300
   aagggtgttc aaccagagaa gaaagagccc tggaaaagac cgagccccta acagaggaaa 3360
   cggaggatcc agagcaccca gaaggaatac acgactcctt ctttgaacgt gagcatccag 3420
   ggtgggttcc tggggtatgc gtgaagaatc tggtaaagat ttttgagccc tgtggccggc 3480
   cagctgtgga ccgtctgaac atcaccttct acgagaacca gatcaccgca ttcctgggcc 3540
   acaatggagc tgggaaaacc accaccttgt aagtatcaag gttacaagac aggtttaagg 3600
   agaccaatag aaactgggct tgtcgagaca gagaagactc ttgcgtttct gggatttttc 3660
   cgatttcggc ctattggtta aaaaatgagc tgatttaaca aaaatttaac gcgaatttta 3720
   acaaaatatt aacgtttata atttcaggtg gcatctttcc cgcctgcaag aactggttca 3780
   gcagcctgag ccacttcgtg atccacctgc aattgaggaa cccctagtga tggagttggc 3840
   cactccctct ctgcgcgctc gctcgctcac tgaggccggg cgaccaaagg tcgcccgacg 3900
   cccgggcttt gcccgggcgg cctcagtgag cgagcgagcg cgcag 3945
<210> 55
   <211> 3904
   <212> **DNA**
   <213> Artificial Sequence
<220>
   <223> synthetic
<400> 55
   gtccatcctg acgggtctgt tgccaccaac ctctgggact gtgctcgttg ggggaaggga      60
   cattgaaacc agcctggatg cagtccggca gagccttggc atgtgtccac agcacaacat     120
   cctgttccac cacctcacgg tggctgagca catgctgttc tatgcccagc tgaaaggaaa     180
   gtcccaggag gaggcccagc tggagatgga agccatgttg gaggacacag gcctccacca     240
   caagcggaat gaagaggctc aggacctatc aggtggcatg cagagaaagc tgtcggttgc     300
   cattgccttt gtgggagatg ccaaggtggt gattctggac gaacccacct ctggggtgga     360
   cccttactcg agacgctcaa tctgggatct gctcctgaag tatcgctcag gcagaaccat     420
   catcatgtcc actcaccaca tggacgaggc cgacctcctt ggggaccgca ttgccatcat     480
   tgcccaggga aggctctact gctcaggcac cccactcttc ctgaagaact gctttggcac     540
   aggcttgtac ttaaccttgg tgcgcaagat gaaaaacatc cagagccaaa ggaaaggcag     600
   tgaggggacc tgcagctgct cgtctaaggg tttctccacc acgtgtccag cccacgtcga     660
   tgacctaact ccagaacaag tcctggatgg ggatgtaaat gagctgatgg atgtagttct     720
   ccaccatgtt ccagaggcaa agctggtgga gtgcattggt caagaactta tcttccttct     780
   tccaaataag aacttcaagc acagagcata tgccagcctt ttcagagagc tggaggagac     840
   gctggctgac cttggtctca gcagttttgg aatttctgac actcccctgg aagagatttt     900
   tctgaaggtc acggaggatt ctgattcagg acctctgttt gcgggtggcg ctcagcagaa     960
   aagagaaaac gtcaaccccc gacacccctg cttgggtccc agagagaagg ctggacagac    1020
   accccaggac tccaatgtct gctccccagg ggcgccggct gctcacccag agggccagcc    1080
   tcccccagag ccagagtgcc caggcccgca gctcaacacg gggacacagc tggtcctcca    1140
   gcatgtgcag gcgctgctgg tcaagagatt ccaacacacc atccgcagcc acaaggactt    1200
   cctggcgcag atcgtgctcc cggctacctt tgtgtttttg gctctgatgc tttctattgt    1260
   tatccctcct tttggcgaat accccgcttt gacccttcac ccctggatat atgggcagca    1320
   gtacaccttc ttcagcatgg atgaaccagg cagtgagcag ttcacggtac ttgcagacgt    1380
   cctcctgaat aagccaggct ttggcaaccg ctgcctgaag gaagggtggc ttccggagta    1440
   cccctgtggc aactcaacac cctggaagac tccttctgtg tccccaaaca tcacccagct    1500
   gttccagaag cagaaatgga cacaggtcaa cccttcacca tcctgcaggt gcagcaccag    1560
   ggagaagctc accatgctgc cagagtgccc cgagggtgcc gggggcctcc cgccccccca    1620
   gagaacacag cgcagcacgg aaattctaca agacctgacg gacaggaaca tctccgactt    1680
   cttggtaaaa acgtatcctg ctcttataag aagcagctta aagagcaaat tctgggtcaa    1740
   tgaacagagg tatggaggaa tttccattgg aggaaagctc ccagtcgtcc ccatcacggg    1800
   ggaagcactt gttgggtttt taagcgacct tggccggatc atgaatgtga gcgggggccc    1860
   tatcactaga gaggcctcta aagaaatacc tgatttcctt aaacatctag aaactgaaga    1920
   caacattaag gtgtggttta ataacaaagg ctggcatgcc ctggtcagct ttctcaatgt 1980
   ggcccacaac gccatcttac gggccagcct gcctaaggac agaagccccg aggagtatgg 2040
   aatcaccgtc attagccaac ccctgaacct gaccaaggag cagctctcag agattacagt 2100
   gctgaccact tcagtggatg ctgtggttgc catctgcgtg attttctcca tgtccttcgt 2160
   cccagccagc tttgtccttt atttgatcca ggagcgggtg aacaaatcca agcacctcca 2220
   gtttatcagt ggagtgagcc ccaccaccta ctgggtaacc aacttcctct gggacatcat 2280
   gaattattcc gtgagtgctg ggctggtggt gggcatcttc atcgggtttc agaagaaagc 2340
   ctacacttct ccagaaaacc ttcctgccct tgtggcactg ctcctgctgt atggatgggc 2400
   ggtcattccc atgatgtacc cagcatcctt cctgtttgat gtccccagca cagcctatgt 2460
   ggctttatct tgtgctaatc tgttcatcgg catcaacagc agtgctatta ccttcatctt 2520
   ggaattattt gagaataacc ggacgctgct caggttcaac gccgtgctga ggaagctgct 2580
   cattgtcttc ccccacttct gcctgggccg gggcctcatt gaccttgcac tgagccaggc 2640
   tgtgacagat gtctatgccc ggtttggtga ggagcactct gcaaatccgt tccactggga 2700
   cctgattggg aagaacctgt ttgccatggt ggtggaaggg gtggtgtact tcctcctgac 2760
   cctgctggtc cagcgccact tcttcctctc ccaatggatt gccgagccca ctaaggagcc 2820
   cattgttgat gaagatgatg atgtggctga agaaagacaa agaattatta ctggtggaaa 2880
   taaaactgac atcttaaggc tacatgaact aaccaagatt tatccaggca cctccagccc 2940
   agcagtggac aggctgtgtg tcggagttcg ccctggagag tgctttggcc tcctgggagt 3000
   gaatggtgcc ggcaaaacaa ccacattcaa gatgctcact ggggacacca cagtgacctc 3060
   aggggatgcc accgtagcag gcaagagtat tttaaccaat atttctgaag tccatcaaaa 3120
   tatgggctac tgtcctcagt ttgatgcaat cgatgagctg ctcacaggac gagaacatct 3180
   ttacctttat gcccggcttc gaggtgtacc agcagaagaa atcgaaaagg ttgcaaactg 3240
   gagtattaag agcctgggcc tgactgtcta cgccgactgc ctggctggca cgtacagtgg 3300
   gggcaacaag cggaaactct ccacagccat cgcactcatt ggctgcccac cgctggtgct 3360
   gctggatgag cccaccacag ggatggaccc ccaggcacgc cgcatgctgt ggaacgtcat 3420
   cgtgagcatc atcagagaag ggagggctgt ggtcctcaca tcccacagca tggaagaatg 3480
   tgaggcactg tgtacccggc tggccatcat ggtaaagggc gcctttcgat gtatgggcac 3540
   cattcagcat ctcaagtcca aatttggaga tggctatatc gtcacaatga agatcaaatc 3600
   cccgaaggac gacctgcttc ctgacctgaa ccctgtggag cagttcttcc aggggaactt 3660
   cccaggcagt gtgcagaggg agaggcacta caacatgctc cagttccagg tctcctcctc 3720
   ctccctggcg aggatcttcc agctcctcct ctcccacaag gacagcctgc tcatcgagga 3780
   gtactcagtc acacagacca cactggacca ggtgtttgta aattttgcta aacagcagac 3840
   tgaaagtcat gacctccctc tgcaccctcg agctgctgga gccagtcgac aagcccagga 3900
   ctga 3904
<210> 56
   <211> 4636
   <212> DNA
   <213> Artificial Sequence
<220>
   <223> synthetic
<400> 56
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatccggga tttttccgat ttcggcctat tggttaaaaa atgagctgat     180
   ttaacaaaaa tttaacgcga attttaacaa aatattaacg tttataattt caggtggcat     240
   ctttcgatag gcacctattg gtcttactga catccacttt gcctttctct ccacaggtcc     300
   atcctgacgg gtctgttgcc accaacctct gggactgtgc tcgttggggg aagggacatt     360
   gaaaccagcc tggatgcagt ccggcagagc cttggcatgt gtccacagca caacatcctg     420
   ttccaccacc tcacggtggc tgagcacatg ctgttctatg cccagctgaa aggaaagtcc     480
   caggaggagg cccagctgga gatggaagcc atgttggagg acacaggcct ccaccacaag     540
   cggaatgaag aggctcagga cctatcaggt ggcatgcaga gaaagctgtc ggttgccatt     600
   gcctttgtgg gagatgccaa ggtggtgatt ctggacgaac ccacctctgg ggtggaccct     660
   tactcgagac gctcaatctg ggatctgctc ctgaagtatc gctcaggcag aaccatcatc     720
   atgtccactc accacatgga cgaggccgac ctccttgggg accgcattgc catcattgcc     780
   cagggaaggc tctactgctc aggcacccca ctcttcctga agaactgctt tggcacaggc     840
   ttgtacttaa ccttggtgcg caagatgaaa aacatccaga gccaaaggaa aggcagtgag     900
   gggacctgca gctgctcgtc taagggtttc tccaccacgt gtccagccca cgtcgatgac     960
   ctaactccag aacaagtcct ggatggggat gtaaatgagc tgatggatgt agttctccac    1020
   catgttccag aggcaaagct ggtggagtgc attggtcaag aacttatctt ccttcttcca    1080
   aataagaact tcaagcacag agcatatgcc agccttttca gagagctgga ggagacgctg    1140
   gctgaccttg gtctcagcag ttttggaatt tctgacactc ccctggaaga gatttttctg    1200
   aaggtcacgg aggattctga ttcaggacct ctgtttgcgg gtggcgctca gcagaaaaga    1260
   gaaaacgtca acccccgaca cccctgcttg ggtcccagag agaaggctgg acagacaccc    1320
   caggactcca atgtctgctc cccaggggcg ccggctgctc acccagaggg ccagcctccc    1380
   ccagagccag agtgcccagg cccgcagctc aacacgggga cacagctggt cctccagcat    1440
   gtgcaggcgc tgctggtcaa gagattccaa cacaccatcc gcagccacaa ggacttcctg 1500
   gcgcagatcg tgctcccggc tacctttgtg tttttggctc tgatgctttc tattgttatc 1560
   cctccttttg gcgaataccc cgctttgacc cttcacccct ggatatatgg gcagcagtac 1620
   accttcttca gcatggatga accaggcagt gagcagttca cggtacttgc agacgtcctc 1680
   ctgaataagc caggctttgg caaccgctgc ctgaaggaag ggtggcttcc ggagtacccc 1740
   tgtggcaact caacaccctg gaagactcct tctgtgtccc caaacatcac ccagctgttc 1800
   cagaagcaga aatggacaca ggtcaaccct tcaccatcct gcaggtgcag caccagggag 1860
   aagctcacca tgctgccaga gtgccccgag ggtgccgggg gcctcccgcc cccccagaga 1920
   acacagcgca gcacggaaat tctacaagac ctgacggaca ggaacatctc cgacttcttg 1980
   gtaaaaacgt atcctgctct tataagaagc agcttaaaga gcaaattctg ggtcaatgaa 2040
   cagaggtatg gaggaatttc cattggagga aagctcccag tcgtccccat cacgggggaa 2100
   gcacttgttg ggtttttaag cgaccttggc cggatcatga atgtgagcgg gggccctatc 2160
   actagagagg cctctaaaga aatacctgat ttccttaaac atctagaaac tgaagacaac 2220
   attaaggtgt ggtttaataa caaaggctgg catgccctgg tcagctttct caatgtggcc 2280
   cacaacgcca tcttacgggc cagcctgcct aaggacagaa gccccgagga gtatggaatc 2340
   accgtcatta gccaacccct gaacctgacc aaggagcagc tctcagagat tacagtgctg 2400
   accacttcag tggatgctgt ggttgccatc tgcgtgattt tctccatgtc cttcgtccca 2460
   gccagctttg tcctttattt gatccaggag cgggtgaaca aatccaagca cctccagttt 2520
   atcagtggag tgagccccac cacctactgg gtaaccaact tcctctggga catcatgaat 2580
   tattccgtga gtgctgggct ggtggtgggc atcttcatcg ggtttcagaa gaaagcctac 2640
   acttctccag aaaaccttcc tgcccttgtg gcactgctcc tgctgtatgg atgggcggtc 2700
   attcccatga tgtacccagc atccttcctg tttgatgtcc ccagcacagc ctatgtggct 2760
   ttatcttgtg ctaatctgtt catcggcatc aacagcagtg ctattacctt catcttggaa 2820
   ttatttgaga ataaccggac gctgctcagg ttcaacgccg tgctgaggaa gctgctcatt 2880
   gtcttccccc acttctgcct gggccggggc ctcattgacc ttgcactgag ccaggctgtg 2940
   acagatgtct atgcccggtt tggtgaggag cactctgcaa atccgttcca ctgggacctg 3000
   attgggaaga acctgtttgc catggtggtg gaaggggtgg tgtacttcct cctgaccctg 3060
   ctggtccagc gccacttctt cctctcccaa tggattgccg agcccactaa ggagcccatt 3120
   gttgatgaag atgatgatgt ggctgaagaa agacaaagaa ttattactgg tggaaataaa 3180
   actgacatct taaggctaca tgaactaacc aagatttatc caggcacctc cagcccagca 3240
   gtggacaggc tgtgtgtcgg agttcgccct ggagagtgct ttggcctcct gggagtgaat 3300
   ggtgccggca aaacaaccac attcaagatg ctcactgggg acaccacagt gacctcaggg 3360
   gatgccaccg tagcaggcaa gagtatttta accaatattt ctgaagtcca tcaaaatatg 3420
   ggctactgtc ctcagtttga tgcaatcgat gagctgctca caggacgaga acatctttac 3480
   ctttatgccc ggcttcgagg tgtaccagca gaagaaatcg aaaaggttgc aaactggagt 3540
   attaagagcc tgggcctgac tgtctacgcc gactgcctgg ctggcacgta cagtgggggc 3600
   aacaagcgga aactctccac agccatcgca ctcattggct gcccaccgct ggtgctgctg 3660
   gatgagccca ccacagggat ggacccccag gcacgccgca tgctgtggaa cgtcatcgtg 3720
   agcatcatca gagaagggag ggctgtggtc ctcacatccc acagcatgga agaatgtgag 3780
   gcactgtgta cccggctggc catcatggta aagggcgcct ttcgatgtat gggcaccatt 3840
   cagcatctca agtccaaatt tggagatggc tatatcgtca caatgaagat caaatccccg 3900
   aaggacgacc tgcttcctga cctgaaccct gtggagcagt tcttccaggg gaacttccca 3960
   ggcagtgtgc agagggagag gcactacaac atgctccagt tccaggtctc ctcctcctcc 4020
   ctggcgagga tcttccagct cctcctctcc cacaaggaca gcctgctcat cgaggagtac 4080
   tcagtcacac agaccacact ggaccaggtg tttgtaaatt ttgctaaaca gcagactgaa 4140
   agtcatgacc tccctctgca ccctcgagct gctggagcca gtcgacaagc ccaggactga 4200
   gcggccgctt cgagcagaca tgataagata cattgatgag tttggacaaa ccacaactag 4260
   aatgcagtga aaaaaatgct ttatttgtga aatttgtgat gctattgctt tatttgtaac 4320
   cattataagc tgcaataaac aagttaacaa caacaattgc attcatttta tgtttcaggt 4380
   tcagggggag atgtgggagg ttttttaaag caagtaaaac ctctacaaat gtggtaaaat 4440
   cgataaggat cttcctagag catggctacg tagataagta gcatggcggg ttaatcatta 4500
   actacaagga acccctagtg atggagttgg ccactccctc tctgcgcgct cgctcgctca 4560
   ctgaggccgg gcgaccaaag gtcgcccgac gcccgggctt tgcccgggcg gcctcagtga 4620
   gcgagcgagc gcgcag 4636
<210> 57
   <211> 4540
   <212> **DNA**
   <213> **Artificial** Sequence
<220>
   <223> synthetic
<400> 57
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct tgtagttaat gattaacccg ccatgctact tatctacgta gccatgctct     180
   aggaagatct tcaatattgg ccattagcca tattattcat tggttatata gcataaatca     240
   atattggcta ttggccattg catacgttgt atctatatca taatatgtac atttatattg 300
   gctcatgtcc aatatgaccg ccatgttggc attgattatt gactagttat taatagtaat 360
   caattacggg gtcattagtt catagcccat atatggagtt ccgcgttaca taacttacgg 420
   taaatggccc gcctggctga ccgcccaacg acccccgccc attgacgtca ataatgacgt 480
   atgttcccat agtaacgcca atagggactt tccattgacg tcaatgggtg gagtatttac 540
   ggtaaactgc ccacttggca gtacatcaag tgtatcatat gccaagtccg ccccctattg 600
   acgtcaatga cggtaaatgg cccgcctggc attatgccca gtacatgacc ttacgggact 660
   ttcctacttg gcagtacatc tacgtattag tcatcgctat taccatggtg atgcggtttt 720
   ggcagtacac caatgggcgt ggatagcggt ttgactcacg gggatttcca agtctccacc 780
   ccattgacgt caatgggagt ttgttttggc accaaaatca acgggacttt ccaaaatgtc 840
   gtaataaccc cgccccgttg acgcaaatgg gcggtaggcg tgtacggtgg gaggtctata 900
   taagcagagc tcgtttagtg aaccgtcaga tcactagaag ctttattgcg gtagtttatc 960
   acagttaaat tgctaacgca gtcagtgctt ctgacacaac agtctcgaac ttaagctgca 1020
   gaagttggtc gtgaggcact gggcaggtaa gtatcaaggt tacaagacag gtttaaggag 1080
   accaatagaa actgggcttg tcgagacaga gaagactctt gcgtttctga taggcaccta 1140
   ttggtcttac tgacatccac tttgcctttc tctccacagg tgtccactcc cagttcaatt 1200
   acagctctta aggctagagt acttaatacg actcactata ggctagcctc gagaattcac 1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggattctgg tcccagagct gatcccgtgg aagatttccg gtacatctgg ggcgggtttg 3120
   cctatctgca ggacatggtt gaacagggga tcacaaggag ccaggtgcag gcggaggctc 3180
   cagttggaat ctacctccag cagatgccct acccctgctt cgtggacgat tctttcatga 3240
   tcatcctgaa ccgctgtttc cctatcttca tggtgctggc atggatctac tctgtctcca 3300
   tgactgtgaa gagcatcgtc ttggagaagg agttgcgact gaaggagacc ttgaaaaatc 3360
   agggtgtctc caatgcagtg atttggtgta cctggttcct ggacagcttc tccatcatgt 3420
   cgatgagcat cttcctcctg acgatattca tcatgcatgg aagaatccta cattacagcg 3480
   acccattcat cctcttcctg ttcttgttgg ctttctccac tgccaccatc atgctgtgct 3540
   ttctgctcag caccttcttc tccaaggcca gtctggcagc agcctgtagt ggtgtcatct 3600
   atttcaccct ctacctgcca cacatcctgt gcttcgcctg gcaggaccgc atgaccgctg 3660
   agctgaagaa ggctgtgagc ttactgtctc cggtggcatt tggatttggc actgagtacc 3720
   tggttcgctt tgaagagcaa ggcctggggc tgcagtggag caacatcggg aacagtccca 3780
   cggaagggga cgaattcagc ttcctgctgt ccatgcagat gatgctcctt gatgctgctg 3840
   tctatggctt actcgcttgg taccttgatc aggtgtttcc aggagactat ggaaccccac 3900
   ttccttggta ctttcttcta caagagtcgt attggcttgg cggtgaaggg tgttcaacca 3960
   gagaagaaag agccctggaa aagaccgagc ccctaacaga ggaaacggag gatccagagc 4020
   acccagaagg aatacacgac tccttctttg aacgtgagca tccagggtgg gttcctgggg     4080
   tatgcgtgaa gaatctggta aagatttttg agccctgtgg ccggccagct gtggaccgtc     4140
   tgaacatcac cttctacgag aaccagatca ccgcattcct gggccacaat ggagctggga     4200
   aaaccaccac cttgtaagta tcaaggttac aagacaggtt taaggagacc aatagaaact     4260
   gggcttgtcg agacagagaa gactcttgcg tttctgggat ttttccgatt tcggcctatt     4320
   ggttaaaaaa tgagctgatt taacaaaaat ttaacgcgaa ttttaacaaa atattaacgt     4380
   ttataatttc aggtggcatc tttccaattg aggaacccct agtgatggag ttggccactc     4440
   cctctctgcg cgctcgctcg ctcactgagg ccgggcgacc aaaggtcgcc cgacgcccgg     4500
   gctttgcccg ggcggcctca gtgagcgagc gagcgcgcag                           4540
<210> 58
   <211> 4702
   <212> DNA
   <213> Artificial Sequence
<220>
   <223> synthetic
<400> 58
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct ggatccggga tttttccgat ttcggcctat tggttaaaaa atgagctgat 180
   ttaacaaaaa tttaacgcga attttaacaa aatattaacg tttataattt caggtggcat 240
   ctttcgatag gcacctattg gtcttactga catccacttt gcctttctct ccacaggtcc 300
   atcctgacgg gtctgttgcc accaacctct gggactgtgc tcgttggggg aagggacatt 360
   gaaaccagcc tggatgcagt ccggcagagc cttggcatgt gtccacagca caacatcctg 420
   ttccaccacc tcacggtggc tgagcacatg ctgttctatg cccagctgaa aggaaagtcc 480
   caggaggagg cccagctgga gatggaagcc atgttggagg acacaggcct ccaccacaag 540
   cggaatgaag aggctcagga cctatcaggt ggcatgcaga gaaagctgtc ggttgccatt 600
   gcctttgtgg gagatgccaa ggtggtgatt ctggacgaac ccacctctgg ggtggaccct 660
   tactcgagac gctcaatctg ggatctgctc ctgaagtatc gctcaggcag aaccatcatc 720
   atgtccactc accacatgga cgaggccgac ctccttgggg accgcattgc catcattgcc 780
   cagggaaggc tctactgctc aggcacccca ctcttcctga agaactgctt tggcacaggc 840
   ttgtacttaa ccttggtgcg caagatgaaa aacatccaga gccaaaggaa aggcagtgag 900
   gggacctgca gctgctcgtc taagggtttc tccaccacgt gtccagccca cgtcgatgac 960
   ctaactccag aacaagtcct ggatggggat gtaaatgagc tgatggatgt agttctccac 1020
   catgttccag aggcaaagct ggtggagtgc attggtcaag aacttatctt ccttcttcca 1080
   aataagaact tcaagcacag agcatatgcc agccttttca gagagctgga ggagacgctg 1140
   gctgaccttg gtctcagcag ttttggaatt tctgacactc ccctggaaga gatttttctg 1200
   aaggtcacgg aggattctga ttcaggacct ctgtttgcgg gtggcgctca gcagaaaaga 1260
   gaaaacgtca acccccgaca cccctgcttg ggtcccagag agaaggctgg acagacaccc 1320
   caggactcca atgtctgctc cccaggggcg ccggctgctc acccagaggg ccagcctccc 1380
   ccagagccag agtgcccagg cccgcagctc aacacgggga cacagctggt cctccagcat 1440
   gtgcaggcgc tgctggtcaa gagattccaa cacaccatcc gcagccacaa ggacttcctg 1500
   gcgcagatcg tgctcccggc tacctttgtg tttttggctc tgatgctttc tattgttatc 1560
   cctccttttg gcgaataccc cgctttgacc cttcacccct ggatatatgg gcagcagtac 1620
   accttcttca gcatggatga accaggcagt gagcagttca cggtacttgc agacgtcctc 1680
   ctgaataagc caggctttgg caaccgctgc ctgaaggaag ggtggcttcc ggagtacccc 1740
   tgtggcaact caacaccctg gaagactcct tctgtgtccc caaacatcac ccagctgttc 1800
   cagaagcaga aatggacaca ggtcaaccct tcaccatcct gcaggtgcag caccagggag 1860
   aagctcacca tgctgccaga gtgccccgag ggtgccgggg gcctcccgcc cccccagaga 1920
   acacagcgca gcacggaaat tctacaagac ctgacggaca ggaacatctc cgacttcttg 1980
   gtaaaaacgt atcctgctct tataagaagc agcttaaaga gcaaattctg ggtcaatgaa 2040
   cagaggtatg gaggaatttc cattggagga aagctcccag tcgtccccat cacgggggaa 2100
   gcacttgttg ggtttttaag cgaccttggc cggatcatga atgtgagcgg gggccctatc 2160
   actagagagg cctctaaaga aatacctgat ttccttaaac atctagaaac tgaagacaac 2220
   attaaggtgt ggtttaataa caaaggctgg catgccctgg tcagctttct caatgtggcc 2280
   cacaacgcca tcttacgggc cagcctgcct aaggacagaa gccccgagga gtatggaatc 2340
   accgtcatta gccaacccct gaacctgacc aaggagcagc tctcagagat tacagtgctg 2400
   accacttcag tggatgctgt ggttgccatc tgcgtgattt tctccatgtc cttcgtccca 2460
   gccagctttg tcctttattt gatccaggag cgggtgaaca aatccaagca cctccagttt 2520
   atcagtggag tgagccccac cacctactgg gtaaccaact tcctctggga catcatgaat 2580
   tattccgtga gtgctgggct ggtggtgggc atcttcatcg ggtttcagaa gaaagcctac 2640
   acttctccag aaaaccttcc tgcccttgtg gcactgctcc tgctgtatgg atgggcggtc 2700
   attcccatga tgtacccagc atccttcctg tttgatgtcc ccagcacagc ctatgtggct 2760
   ttatcttgtg ctaatctgtt catcggcatc aacagcagtg ctattacctt catcttggaa 2820
   ttatttgaga ataaccggac gctgctcagg ttcaacgccg tgctgaggaa gctgctcatt 2880
   gtcttccccc acttctgcct gggccggggc ctcattgacc ttgcactgag ccaggctgtg 2940
   acagatgtct atgcccggtt tggtgaggag cactctgcaa atccgttcca ctgggacctg      3000
   attgggaaga acctgtttgc catggtggtg gaaggggtgg tgtacttcct cctgaccctg      3060
   ctggtccagc gccacttctt cctctcccaa tggattgccg agcccactaa ggagcccatt      3120
   gttgatgaag atgatgatgt ggctgaagaa agacaaagaa ttattactgg tggaaataaa      3180
   actgacatct taaggctaca tgaactaacc aagatttatc caggcacctc cagcccagca      3240
   gtggacaggc tgtgtgtcgg agttcgccct ggagagtgct ttggcctcct gggagtgaat      3300
   ggtgccggca aaacaaccac attcaagatg ctcactgggg acaccacagt gacctcaggg      3360
   gatgccaccg tagcaggcaa gagtatttta accaatattt ctgaagtcca tcaaaatatg      3420
   ggctactgtc ctcagtttga tgcaatcgat gagctgctca caggacgaga acatctttac      3480
   ctttatgccc ggcttcgagg tgtaccagca gaagaaatcg aaaaggttgc aaactggagt      3540
   attaagagcc tgggcctgac tgtctacgcc gactgcctgg ctggcacgta cagtgggggc      3600
   aacaagcgga aactctccac agccatcgca ctcattggct gcccaccgct ggtgctgctg      3660
   gatgagccca ccacagggat ggacccccag gcacgccgca tgctgtggaa cgtcatcgtg      3720
   agcatcatca gagaagggag ggctgtggtc ctcacatccc acagcatgga agaatgtgag      3780
   gcactgtgta cccggctggc catcatggta aagggcgcct ttcgatgtat gggcaccatt      3840
   cagcatctca agtccaaatt tggagatggc tatatcgtca caatgaagat caaatccccg      3900
   aaggacgacc tgcttcctga cctgaaccct gtggagcagt tcttccaggg gaacttccca      3960
   ggcagtgtgc agagggagag gcactacaac atgctccagt tccaggtctc ctcctcctcc      4020
   ctggcgagga tcttccagct cctcctctcc cacaaggaca gcctgctcat cgaggagtac      4080
   tcagtcacac agaccacact ggaccaggtg tttgtaaatt ttgctaaaca gcagactgaa      4140
   agtcatgacc tccctctgca ccctcgagct gctggagcca gtcgacaagc ccaggacgac      4200
   tacaaagacc atgacggtga ttataaagat catgacatcg actacaagga tgacgatgac      4260
   aagtgagcgg ccgcttcgag cagacatgat aagatacatt gatgagtttg gacaaaccac      4320
   aactagaatg cagtgaaaaa aatgctttat ttgtgaaatt tgtgatgcta ttgctttatt      4380
   tgtaaccatt ataagctgca ataaacaagt taacaacaac aattgcattc attttatgtt      4440
   tcaggttcag ggggagatgt gggaggtttt ttaaagcaag taaaacctct acaaatgtgg      4500
   taaaatcgat aaggatcttc ctagagcatg gctacgtaga taagtagcat ggcgggttaa      4560
   tcattaacta caaggaaccc ctagtgatgg agttggccac tccctctctg cgcgctcgct      4620
   cgctcactga ggccgggcga ccaaaggtcg cccgacgccc gggctttgcc cgggcggcct      4680
   cagtgagcga gcgagcgcgc ag                                              4702
<210> 59
   <211> 4718
   <212> **DNA**
   <213> **Artificial** Sequence
<220>
   <223> synthetic
<400> 59
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct tgtagttaat gattaacccg ccatgctact tatctacgta gccatgctct 180
   aggaagatct tcaatattgg ccattagcca tattattcat tggttatata gcataaatca 240
   atattggcta ttggccattg catacgttgt atctatatca taatatgtac atttatattg 300
   gctcatgtcc aatatgaccg ccatgttggc attgattatt gactagttat taatagtaat 360
   caattacggg gtcattagtt catagcccat atatggagtt ccgcgttaca taacttacgg 420
   taaatggccc gcctggctga ccgcccaacg acccccgccc attgacgtca ataatgacgt 480
   atgttcccat agtaacgcca atagggactt tccattgacg tcaatgggtg gagtatttac 540
   ggtaaactgc ccacttggca gtacatcaag tgtatcatat gccaagtccg ccccctattg 600
   acgtcaatga cggtaaatgg cccgcctggc attatgccca gtacatgacc ttacgggact 660
   ttcctacttg gcagtacatc tacgtattag tcatcgctat taccatggtg atgcggtttt 720
   ggcagtacac caatgggcgt ggatagcggt ttgactcacg gggatttcca agtctccacc 780
   ccattgacgt caatgggagt ttgttttggc accaaaatca acgggacttt ccaaaatgtc 840
   gtaataaccc cgccccgttg acgcaaatgg gcggtaggcg tgtacggtgg gaggtctata 900
   taagcagagc tcgtttagtg aaccgtcaga tcactagaag ctttattgcg gtagtttatc 960
   acagttaaat tgctaacgca gtcagtgctt ctgacacaac agtctcgaac ttaagctgca 1020
   gaagttggtc gtgaggcact gggcaggtaa gtatcaaggt tacaagacag gtttaaggag 1080
   accaatagaa actgggcttg tcgagacaga gaagactctt gcgtttctga taggcaccta 1140
   ttggtcttac tgacatccac tttgcctttc tctccacagg tgtccactcc cagttcaatt 1200
   acagctctta aggctagagt acttaatacg actcactata ggctagcctc gagaattcac 1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggattctgg tcccagagct gatcccgtgg aagatttccg gtacatctgg ggcgggtttg 3120
   cctatctgca ggacatggtt gaacagggga tcacaaggag ccaggtgcag gcggaggctc 3180
   cagttggaat ctacctccag cagatgccct acccctgctt cgtggacgat tctttcatga 3240
   tcatcctgaa ccgctgtttc cctatcttca tggtgctggc atggatctac tctgtctcca 3300
   tgactgtgaa gagcatcgtc ttggagaagg agttgcgact gaaggagacc ttgaaaaatc 3360
   agggtgtctc caatgcagtg atttggtgta cctggttcct ggacagcttc tccatcatgt 3420
   cgatgagcat cttcctcctg acgatattca tcatgcatgg aagaatccta cattacagcg 3480
   acccattcat cctcttcctg ttcttgttgg ctttctccac tgccaccatc atgctgtgct 3540
   ttctgctcag caccttcttc tccaaggcca gtctggcagc agcctgtagt ggtgtcatct 3600
   atttcaccct ctacctgcca cacatcctgt gcttcgcctg gcaggaccgc atgaccgctg 3660
   agctgaagaa ggctgtgagc ttactgtctc cggtggcatt tggatttggc actgagtacc 3720
   tggttcgctt tgaagagcaa ggcctggggc tgcagtggag caacatcggg aacagtccca 3780
   cggaagggga cgaattcagc ttcctgctgt ccatgcagat gatgctcctt gatgctgctg 3840
   tctatggctt actcgcttgg taccttgatc aggtgtttcc aggagactat ggaaccccac 3900
   ttccttggta ctttcttcta caagagtcgt attggcttgg cggtgaaggg tgttcaacca 3960
   gagaagaaag agccctggaa aagaccgagc ccctaacaga ggaaacggag gatccagagc 4020
   acccagaagg aatacacgac tccttctttg aacgtgagca tccagggtgg gttcctgggg 4080
   tatgcgtgaa gaatctggta aagatttttg agccctgtgg ccggccagct gtggaccgtc 4140
   tgaacatcac cttctacgag aaccagatca ccgcattcct gggccacaat ggagctggga 4200
   aaaccaccac cttgtaagta tcaaggttac aagacaggtt taaggagacc aatagaaact 4260
   gggcttgtcg agacagagaa gactcttgcg tttctccccg ggtgcgcggc gtcggtggtg 4320
   ccggcggggg gcgccaggtc gcaggcggtg tagggctcca ggcaggcggc gaaggccatg 4380
   acgtgcgcta tgaaggtctg ctcctgcacg ccgtgaacca ggtgcgcctg cgggccgcgc 4440
   gcgaacaccg ccacgtcctc gcctgcgtgg gtctcttcgt ccaggggcac tgctgactgc 4500
   tgccgatact cggggctccc gctctcgctc tcggtaacat ccggccgggc gccgtccttg 4560
   agcacatagc ctggaccgtt tccaattgag gaacccctag tgatggagtt ggccactccc 4620
   tctctgcgcg ctcgctcgct cactgaggcc gggcgaccaa aggtcgcccg acgcccgggc 4680
   tttgcccggg cggcctcagt gagcgagcga gcgcgcag 4718
<210> 60
   <211> 4880
   <212> DNA
   <213> Artificial Sequence
<220>
   <223> synthetic
<400> 60
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatcccccc gggtgcgcgg cgtcggtggt gccggcgggg ggcgccaggt     180
   cgcaggcggt gtagggctcc aggcaggcgg cgaaggccat gacgtgcgct atgaaggtct     240
   gctcctgcac gccgtgaacc aggtgcgcct gcgggccgcg cgcgaacacc gccacgtcct     300
   cgcctgcgtg ggtctcttcg tccaggggca ctgctgactg ctgccgatac tcggggctcc     360
   cgctctcgct ctcggtaaca tccggccggg cgccgtcctt gagcacatag cctggaccgt     420
   ttcgataggc acctattggt cttactgaca tccactttgc ctttctctcc acaggtccat 480
   cctgacgggt ctgttgccac caacctctgg gactgtgctc gttgggggaa gggacattga 540
   aaccagcctg gatgcagtcc ggcagagcct tggcatgtgt ccacagcaca acatcctgtt 600
   ccaccacctc acggtggctg agcacatgct gttctatgcc cagctgaaag gaaagtccca 660
   ggaggaggcc cagctggaga tggaagccat gttggaggac acaggcctcc accacaagcg 720
   gaatgaagag gctcaggacc tatcaggtgg catgcagaga aagctgtcgg ttgccattgc 780
   ctttgtggga gatgccaagg tggtgattct ggacgaaccc acctctgggg tggaccctta 840
   ctcgagacgc tcaatctggg atctgctcct gaagtatcgc tcaggcagaa ccatcatcat 900
   gtccactcac cacatggacg aggccgacct ccttggggac cgcattgcca tcattgccca 960
   gggaaggctc tactgctcag gcaccccact cttcctgaag aactgctttg gcacaggctt 1020
   gtacttaacc ttggtgcgca agatgaaaaa catccagagc caaaggaaag gcagtgaggg 1080
   gacctgcagc tgctcgtcta agggtttctc caccacgtgt ccagcccacg tcgatgacct 1140
   aactccagaa caagtcctgg atggggatgt aaatgagctg atggatgtag ttctccacca 1200
   tgttccagag gcaaagctgg tggagtgcat tggtcaagaa cttatcttcc ttcttccaaa 1260
   taagaacttc aagcacagag catatgccag ccttttcaga gagctggagg agacgctggc 1320
   tgaccttggt ctcagcagtt ttggaatttc tgacactccc ctggaagaga tttttctgaa 1380
   ggtcacggag gattctgatt caggacctct gtttgcgggt ggcgctcagc agaaaagaga 1440
   aaacgtcaac ccccgacacc cctgcttggg tcccagagag aaggctggac agacacccca 1500
   ggactccaat gtctgctccc caggggcgcc ggctgctcac ccagagggcc agcctccccc 1560
   agagccagag tgcccaggcc cgcagctcaa cacggggaca cagctggtcc tccagcatgt 1620
   gcaggcgctg ctggtcaaga gattccaaca caccatccgc agccacaagg acttcctggc 1680
   gcagatcgtg ctcccggcta cctttgtgtt tttggctctg atgctttcta ttgttatccc 1740
   tccttttggc gaataccccg ctttgaccct tcacccctgg atatatgggc agcagtacac 1800
   cttcttcagc atggatgaac caggcagtga gcagttcacg gtacttgcag acgtcctcct 1860
   gaataagcca ggctttggca accgctgcct gaaggaaggg tggcttccgg agtacccctg 1920
   tggcaactca acaccctgga agactccttc tgtgtcccca aacatcaccc agctgttcca 1980
   gaagcagaaa tggacacagg tcaacccttc accatcctgc aggtgcagca ccagggagaa 2040
   gctcaccatg ctgccagagt gccccgaggg tgccgggggc ctcccgcccc cccagagaac 2100
   acagcgcagc acggaaattc tacaagacct gacggacagg aacatctccg acttcttggt 2160
   aaaaacgtat cctgctctta taagaagcag cttaaagagc aaattctggg tcaatgaaca 2220
   gaggtatgga ggaatttcca ttggaggaaa gctcccagtc gtccccatca cgggggaagc 2280
   acttgttggg tttttaagcg accttggccg gatcatgaat gtgagcgggg gccctatcac    2340
   tagagaggcc tctaaagaaa tacctgattt ccttaaacat ctagaaactg aagacaacat    2400
   taaggtgtgg tttaataaca aaggctggca tgccctggtc agctttctca atgtggccca    2460
   caacgccatc ttacgggcca gcctgcctaa ggacagaagc cccgaggagt atggaatcac    2520
   cgtcattagc caacccctga acctgaccaa ggagcagctc tcagagatta cagtgctgac    2580
   cacttcagtg gatgctgtgg ttgccatctg cgtgattttc tccatgtcct tcgtcccagc    2640
   cagctttgtc ctttatttga tccaggagcg ggtgaacaaa tccaagcacc tccagtttat    2700
   cagtggagtg agccccacca cctactgggt aaccaacttc ctctgggaca tcatgaatta    2760
   ttccgtgagt gctgggctgg tggtgggcat cttcatcggg tttcagaaga aagcctacac    2820
   ttctccagaa aaccttcctg cccttgtggc actgctcctg ctgtatggat gggcggtcat    2880
   tcccatgatg tacccagcat ccttcctgtt tgatgtcccc agcacagcct atgtggcttt    2940
   atcttgtgct aatctgttca tcggcatcaa cagcagtgct attaccttca tcttggaatt    3000
   atttgagaat aaccggacgc tgctcaggtt caacgccgtg ctgaggaagc tgctcattgt    3060
   cttcccccac ttctgcctgg gccggggcct cattgacctt gcactgagcc aggctgtgac    3120
   agatgtctat gcccggtttg gtgaggagca ctctgcaaat ccgttccact gggacctgat    3180
   tgggaagaac ctgtttgcca tggtggtgga aggggtggtg tacttcctcc tgaccctgct    3240
   ggtccagcgc cacttcttcc tctcccaatg gattgccgag cccactaagg agcccattgt    3300
   tgatgaagat gatgatgtgg ctgaagaaag acaaagaatt attactggtg gaaataaaac    3360
   tgacatctta aggctacatg aactaaccaa gatttatcca ggcacctcca gcccagcagt    3420
   ggacaggctg tgtgtcggag ttcgccctgg agagtgcttt ggcctcctgg gagtgaatgg    3480
   tgccggcaaa acaaccacat tcaagatgct cactggggac accacagtga cctcagggga    3540
   tgccaccgta gcaggcaaga gtattttaac caatatttct gaagtccatc aaaatatggg    3600
   ctactgtcct cagtttgatg caatcgatga gctgctcaca ggacgagaac atctttacct    3660
   ttatgcccgg cttcgaggtg taccagcaga agaaatcgaa aaggttgcaa actggagtat    3720
   taagagcctg ggcctgactg tctacgccga ctgcctggct ggcacgtaca gtgggggcaa    3780
   caagcggaaa ctctccacag ccatcgcact cattggctgc ccaccgctgg tgctgctgga    3840
   tgagcccacc acagggatgg acccccaggc acgccgcatg ctgtggaacg tcatcgtgag    3900
   catcatcaga gaagggaggg ctgtggtcct cacatcccac agcatggaag aatgtgaggc    3960
   actgtgtacc cggctggcca tcatggtaaa gggcgccttt cgatgtatgg gcaccattca    4020
   gcatctcaag tccaaatttg gagatggcta tatcgtcaca atgaagatca aatccccgaa    4080
   ggacgacctg cttcctgacc tgaaccctgt ggagcagttc ttccagggga acttcccagg    4140
   cagtgtgcag agggagaggc actacaacat gctccagttc caggtctcct cctcctccct    4200
   ggcgaggatc ttccagctcc tcctctccca caaggacagc ctgctcatcg aggagtactc 4260
   agtcacacag accacactgg accaggtgtt tgtaaatttt gctaaacagc agactgaaag 4320
   tcatgacctc cctctgcacc ctcgagctgc tggagccagt cgacaagccc aggacgacta 4380
   caaagaccat gacggtgatt ataaagatca tgacatcgac tacaaggatg acgatgacaa 4440
   gtgagcggcc gcttcgagca gacatgataa gatacattga tgagtttgga caaaccacaa 4500
   ctagaatgca gtgaaaaaaa tgctttattt gtgaaatttg tgatgctatt gctttatttg 4560
   taaccattat aagctgcaat aaacaagtta acaacaacaa ttgcattcat tttatgtttc 4620
   aggttcaggg ggagatgtgg gaggtttttt aaagcaagta aaacctctac aaatgtggta 4680
   aaatcgataa ggatcttcct agagcatggc tacgtagata agtagcatgg cgggttaatc 4740
   attaactaca aggaacccct agtgatggag ttggccactc cctctctgcg cgctcgctcg 4800
   ctcactgagg ccgggcgacc aaaggtcgcc cgacgcccgg gctttgcccg ggcggcctca 4860
   gtgagcgagc gagcgcgcag 4880
<210> 61
   <211> 4719
   <212> **DNA**
   <213> **Artificial** Sequence
<220>
   <223> synthetic
<400> 61
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct tgtagttaat gattaacccg ccatgctact tatctacgta gccatgctct     180
   aggaagatct tcaatattgg ccattagcca tattattcat tggttatata gcataaatca     240
   atattggcta ttggccattg catacgttgt atctatatca taatatgtac atttatattg     300
   gctcatgtcc aatatgaccg ccatgttggc attgattatt gactagttat taatagtaat     360
   caattacggg gtcattagtt catagcccat atatggagtt ccgcgttaca taacttacgg     420
   taaatggccc gcctggctga ccgcccaacg acccccgccc attgacgtca ataatgacgt     480
   atgttcccat agtaacgcca atagggactt tccattgacg tcaatgggtg gagtatttac     540
   ggtaaactgc ccacttggca gtacatcaag tgtatcatat gccaagtccg ccccctattg     600
   acgtcaatga cggtaaatgg cccgcctggc attatgccca gtacatgacc ttacgggact     660
   ttcctacttg gcagtacatc tacgtattag tcatcgctat taccatggtg atgcggtttt     720
   ggcagtacac caatgggcgt ggatagcggt ttgactcacg gggatttcca agtctccacc     780
   ccattgacgt caatgggagt ttgttttggc accaaaatca acgggacttt ccaaaatgtc     840
   gtaataaccc cgccccgttg acgcaaatgg gcggtaggcg tgtacggtgg gaggtctata 900
   taagcagagc tcgtttagtg aaccgtcaga tcactagaag ctttattgcg gtagtttatc 960
   acagttaaat tgctaacgca gtcagtgctt ctgacacaac agtctcgaac ttaagctgca 1020
   gaagttggtc gtgaggcact gggcaggtaa gtatcaaggt tacaagacag gtttaaggag 1080
   accaatagaa actgggcttg tcgagacaga gaagactctt gcgtttctga taggcaccta 1140
   ttggtcttac tgacatccac tttgcctttc tctccacagg tgtccactcc cagttcaatt 1200
   acagctctta aggctagagt acttaatacg actcactata ggctagcctc gagaattcac 1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggattctgg tcccagagct gatcccgtgg aagatttccg gtacatctgg ggcgggtttg 3120
   cctatctgca ggacatggtt gaacagggga tcacaaggag ccaggtgcag gcggaggctc 3180
   cagttggaat ctacctccag cagatgccct acccctgctt cgtggacgat tctttcatga 3240
   tcatcctgaa ccgctgtttc cctatcttca tggtgctggc atggatctac tctgtctcca 3300
   tgactgtgaa gagcatcgtc ttggagaagg agttgcgact gaaggagacc ttgaaaaatc 3360
   agggtgtctc caatgcagtg atttggtgta cctggttcct ggacagcttc tccatcatgt 3420
   cgatgagcat cttcctcctg acgatattca tcatgcatgg aagaatccta cattacagcg 3480
   acccattcat cctcttcctg ttcttgttgg ctttctccac tgccaccatc atgctgtgct 3540
   ttctgctcag caccttcttc tccaaggcca gtctggcagc agcctgtagt ggtgtcatct 3600
   atttcaccct ctacctgcca cacatcctgt gcttcgcctg gcaggaccgc atgaccgctg 3660
   agctgaagaa ggctgtgagc ttactgtctc cggtggcatt tggatttggc actgagtacc 3720
   tggttcgctt tgaagagcaa ggcctggggc tgcagtggag caacatcggg aacagtccca    3780
   cggaagggga cgaattcagc ttcctgctgt ccatgcagat gatgctcctt gatgctgctg    3840
   tctatggctt actcgcttgg taccttgatc aggtgtttcc aggagactat ggaaccccac 3900
   ttccttggta ctttcttcta caagagtcgt attggcttgg cggtgaaggg tgttcaacca 3960
   gagaagaaag agccctggaa aagaccgagc ccctaacaga ggaaacggag gatccagagc 4020
   acccagaagg aatacacgac tccttctttg aacgtgagca tccagggtgg gttcctgggg 4080
   tatgcgtgaa gaatctggta aagatttttg agccctgtgg ccggccagct gtggaccgtc 4140
   tgaacatcac cttctacgag aaccagatca ccgcattcct gggccacaat ggagctggga 4200
   aaaccaccac cttgtaagta tcaaggttac aagacaggtt taaggagacc aatagaaact 4260
   gggcttgtcg agacagagaa gactcttgcg tttctcgcag ggcagcctct gtcatctcca 4320
   tcagggaggg gtccagtgtg gagtctcggt ggatctcgta tttcatgtct ccaggctcaa 4380
   agagacccat gagatgggtc acagacgggt ccagggaagc ctgcatgagc tcagtgcggt 4440
   tccacacata ccgggcaccc tggcgcttcg ccagccattc ctgcaccaga ttcttcccgt 4500
   ccagcctggt cccaccttgg ctgtagtcat ctgggtactc agggtctggg gttcccatgc 4560
   gaaacatgta ctttcggcct ccacaattga ggaaccccta gtgatggagt tggccactcc 4620
   ctctctgcgc gctcgctcgc tcactgaggc cgggcgacca aaggtcgccc gacgcccggg 4680
   ctttgcccgg gcggcctcag tgagcgagcg agcgcgcag 4719
<210> 62
   <211> 4881
   <212> **DNA**
   <213> Artificial Sequence
<220>
   <223> synthetic
<400> 62
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatcccgca gggcagcctc tgtcatctcc atcagggagg ggtccagtgt     180
   ggagtctcgg tggatctcgt atttcatgtc tccaggctca aagagaccca tgagatgggt     240
   cacagacggg tccagggaag cctgcatgag ctcagtgcgg ttccacacat accgggcacc     300
   ctggcgcttc gccagccatt cctgcaccag attcttcccg tccagcctgg tcccaccttg     360
   gctgtagtca tctgggtact cagggtctgg ggttcccatg cgaaacatgt actttcggcc     420
   tccagatagg cacctattgg tcttactgac atccactttg cctttctctc cacaggtcca     480
   tcctgacggg tctgttgcca ccaacctctg ggactgtgct cgttggggga agggacattg     540
   aaaccagcct ggatgcagtc cggcagagcc ttggcatgtg tccacagcac aacatcctgt     600
   tccaccacct cacggtggct gagcacatgc tgttctatgc ccagctgaaa ggaaagtccc     660
   aggaggaggc ccagctggag atggaagcca tgttggagga cacaggcctc caccacaagc     720
   ggaatgaaga ggctcaggac ctatcaggtg gcatgcagag aaagctgtcg gttgccattg     780
   cctttgtggg agatgccaag gtggtgattc tggacgaacc cacctctggg gtggaccctt     840
   actcgagacg ctcaatctgg gatctgctcc tgaagtatcg ctcaggcaga accatcatca     900
   tgtccactca ccacatggac gaggccgacc tccttgggga ccgcattgcc atcattgccc     960
   agggaaggct ctactgctca ggcaccccac tcttcctgaa gaactgcttt ggcacaggct    1020
   tgtacttaac cttggtgcgc aagatgaaaa acatccagag ccaaaggaaa ggcagtgagg    1080
   ggacctgcag ctgctcgtct aagggtttct ccaccacgtg tccagcccac gtcgatgacc    1140
   taactccaga acaagtcctg gatggggatg taaatgagct gatggatgta gttctccacc    1200
   atgttccaga ggcaaagctg gtggagtgca ttggtcaaga acttatcttc cttcttccaa    1260
   ataagaactt caagcacaga gcatatgcca gccttttcag agagctggag gagacgctgg    1320
   ctgaccttgg tctcagcagt tttggaattt ctgacactcc cctggaagag atttttctga    1380
   aggtcacgga ggattctgat tcaggacctc tgtttgcggg tggcgctcag cagaaaagag    1440
   aaaacgtcaa cccccgacac ccctgcttgg gtcccagaga gaaggctgga cagacacccc    1500
   aggactccaa tgtctgctcc ccaggggcgc cggctgctca cccagagggc cagcctcccc    1560
   cagagccaga gtgcccaggc ccgcagctca acacggggac acagctggtc ctccagcatg    1620
   tgcaggcgct gctggtcaag agattccaac acaccatccg cagccacaag gacttcctgg    1680
   cgcagatcgt gctcccggct acctttgtgt ttttggctct gatgctttct attgttatcc    1740
   ctccttttgg cgaatacccc gctttgaccc ttcacccctg gatatatggg cagcagtaca    1800
   ccttcttcag catggatgaa ccaggcagtg agcagttcac ggtacttgca gacgtcctcc    1860
   tgaataagcc aggctttggc aaccgctgcc tgaaggaagg gtggcttccg gagtacccct    1920
   gtggcaactc aacaccctgg aagactcctt ctgtgtcccc aaacatcacc cagctgttcc    1980
   agaagcagaa atggacacag gtcaaccctt caccatcctg caggtgcagc accagggaga    2040
   agctcaccat gctgccagag tgccccgagg gtgccggggg cctcccgccc ccccagagaa    2100
   cacagcgcag cacggaaatt ctacaagacc tgacggacag gaacatctcc gacttcttgg    2160
   taaaaacgta tcctgctctt ataagaagca gcttaaagag caaattctgg gtcaatgaac    2220
   agaggtatgg aggaatttcc attggaggaa agctcccagt cgtccccatc acgggggaag    2280
   cacttgttgg gtttttaagc gaccttggcc ggatcatgaa tgtgagcggg ggccctatca    2340
   ctagagaggc ctctaaagaa atacctgatt tccttaaaca tctagaaact gaagacaaca    2400
   ttaaggtgtg gtttaataac aaaggctggc atgccctggt cagctttctc aatgtggccc    2460
   acaacgccat cttacgggcc agcctgccta aggacagaag ccccgaggag tatggaatca    2520
   ccgtcattag ccaacccctg aacctgacca aggagcagct ctcagagatt acagtgctga    2580
   ccacttcagt ggatgctgtg gttgccatct gcgtgatttt ctccatgtcc ttcgtcccag    2640
   ccagctttgt cctttatttg atccaggagc gggtgaacaa atccaagcac ctccagttta    2700
   tcagtggagt gagccccacc acctactggg taaccaactt cctctgggac atcatgaatt    2760
   attccgtgag tgctgggctg gtggtgggca tcttcatcgg gtttcagaag aaagcctaca    2820
   cttctccaga aaaccttcct gcccttgtgg cactgctcct gctgtatgga tgggcggtca    2880
   ttcccatgat gtacccagca tccttcctgt ttgatgtccc cagcacagcc tatgtggctt    2940
   tatcttgtgc taatctgttc atcggcatca acagcagtgc tattaccttc atcttggaat    3000
   tatttgagaa taaccggacg ctgctcaggt tcaacgccgt gctgaggaag ctgctcattg    3060
   tcttccccca cttctgcctg ggccggggcc tcattgacct tgcactgagc caggctgtga    3120
   cagatgtcta tgcccggttt ggtgaggagc actctgcaaa tccgttccac tgggacctga    3180
   ttgggaagaa cctgtttgcc atggtggtgg aaggggtggt gtacttcctc ctgaccctgc    3240
   tggtccagcg ccacttcttc ctctcccaat ggattgccga gcccactaag gagcccattg    3300
   ttgatgaaga tgatgatgtg gctgaagaaa gacaaagaat tattactggt ggaaataaaa    3360
   ctgacatctt aaggctacat gaactaacca agatttatcc aggcacctcc agcccagcag 3420
   tggacaggct gtgtgtcgga gttcgccctg gagagtgctt tggcctcctg ggagtgaatg 3480
   gtgccggcaa aacaaccaca ttcaagatgc tcactgggga caccacagtg acctcagggg 3540
   atgccaccgt agcaggcaag agtattttaa ccaatatttc tgaagtccat caaaatatgg 3600
   gctactgtcc tcagtttgat gcaatcgatg agctgctcac aggacgagaa catctttacc 3660
   tttatgcccg gcttcgaggt gtaccagcag aagaaatcga aaaggttgca aactggagta 3720
   ttaagagcct gggcctgact gtctacgccg actgcctggc tggcacgtac agtgggggca 3780
   acaagcggaa actctccaca gccatcgcac tcattggctg cccaccgctg gtgctgctgg 3840
   atgagcccac cacagggatg gacccccagg cacgccgcat gctgtggaac gtcatcgtga 3900
   gcatcatcag agaagggagg gctgtggtcc tcacatccca cagcatggaa gaatgtgagg 3960
   cactgtgtac ccggctggcc atcatggtaa agggcgcctt tcgatgtatg ggcaccattc 4020
   agcatctcaa gtccaaattt ggagatggct atatcgtcac aatgaagatc aaatccccga 4080
   aggacgacct gcttcctgac ctgaaccctg tggagcagtt cttccagggg aacttcccag 4140
   gcagtgtgca gagggagagg cactacaaca tgctccagtt ccaggtctcc tcctcctccc 4200
   tggcgaggat cttccagctc ctcctctccc acaaggacag cctgctcatc gaggagtact 4260
   cagtcacaca gaccacactg gaccaggtgt ttgtaaattt tgctaaacag cagactgaaa 4320
   gtcatgacct ccctctgcac cctcgagctg ctggagccag tcgacaagcc caggacgact 4380
   acaaagacca tgacggtgat tataaagatc atgacatcga ctacaaggat gacgatgaca 4440
   agtgagcggc cgcttcgagc agacatgata agatacattg atgagtttgg acaaaccaca 4500
   actagaatgc agtgaaaaaa atgctttatt tgtgaaattt gtgatgctat tgctttattt 4560
   gtaaccatta taagctgcaa taaacaagtt aacaacaaca attgcattca ttttatgttt 4620
   caggttcagg gggagatgtg ggaggttttt taaagcaagt aaaacctcta caaatgtggt 4680
   aaaatcgata aggatcttcc tagagcatgg ctacgtagat aagtagcatg gcgggttaat 4740
   cattaactac aaggaacccc tagtgatgga gttggccact ccctctctgc gcgctcgctc 4800
   gctcactgag gccgggcgac caaaggtcgc ccgacgcccg ggctttgccc gggcggcctc 4860
   agtgagcgag cgagcgcgca g 4881
<210> 63
   <211> 4709
   <212> **DNA**
   <213> Artificial Sequence
<220>
   <223> synthetic
<400> 63
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct tgtagttaat gattaacccg ccatgctact tatctacgta gccatgctct 180
   aggaagatct tcaatattgg ccattagcca tattattcat tggttatata gcataaatca 240
   atattggcta ttggccattg catacgttgt atctatatca taatatgtac atttatattg 300
   gctcatgtcc aatatgaccg ccatgttggc attgattatt gactagttat taatagtaat 360
   caattacggg gtcattagtt catagcccat atatggagtt ccgcgttaca taacttacgg 420
   taaatggccc gcctggctga ccgcccaacg acccccgccc attgacgtca ataatgacgt 480
   atgttcccat agtaacgcca atagggactt tccattgacg tcaatgggtg gagtatttac 540
   ggtaaactgc ccacttggca gtacatcaag tgtatcatat gccaagtccg ccccctattg 600
   acgtcaatga cggtaaatgg cccgcctggc attatgccca gtacatgacc ttacgggact 660
   ttcctacttg gcagtacatc tacgtattag tcatcgctat taccatggtg atgcggtttt 720
   ggcagtacac caatgggcgt ggatagcggt ttgactcacg gggatttcca agtctccacc 780
   ccattgacgt caatgggagt ttgttttggc accaaaatca acgggacttt ccaaaatgtc 840
   gtaataaccc cgccccgttg acgcaaatgg gcggtaggcg tgtacggtgg gaggtctata 900
   taagcagagc tcgtttagtg aaccgtcaga tcactagaag ctttattgcg gtagtttatc 960
   acagttaaat tgctaacgca gtcagtgctt ctgacacaac agtctcgaac ttaagctgca 1020
   gaagttggtc gtgaggcact gggcaggtaa gtatcaaggt tacaagacag gtttaaggag 1080
   accaatagaa actgggcttg tcgagacaga gaagactctt gcgtttctga taggcaccta 1140
   ttggtcttac tgacatccac tttgcctttc tctccacagg tgtccactcc cagttcaatt 1200
   acagctctta aggctagagt acttaatacg actcactata ggctagcctc gagaattcac 1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggattctgg tcccagagct gatcccgtgg aagatttccg gtacatctgg ggcgggtttg 3120
   cctatctgca ggacatggtt gaacagggga tcacaaggag ccaggtgcag gcggaggctc 3180
   cagttggaat ctacctccag cagatgccct acccctgctt cgtggacgat tctttcatga 3240
   tcatcctgaa ccgctgtttc cctatcttca tggtgctggc atggatctac tctgtctcca 3300
   tgactgtgaa gagcatcgtc ttggagaagg agttgcgact gaaggagacc ttgaaaaatc 3360
   agggtgtctc caatgcagtg atttggtgta cctggttcct ggacagcttc tccatcatgt 3420
   cgatgagcat cttcctcctg acgatattca tcatgcatgg aagaatccta cattacagcg 3480
   acccattcat cctcttcctg ttcttgttgg ctttctccac tgccaccatc atgctgtgct 3540
   ttctgctcag caccttcttc tccaaggcca gtctggcagc agcctgtagt ggtgtcatct 3600
   atttcaccct ctacctgcca cacatcctgt gcttcgcctg gcaggaccgc atgaccgctg 3660
   agctgaagaa ggctgtgagc ttactgtctc cggtggcatt tggatttggc actgagtacc 3720
   tggttcgctt tgaagagcaa ggcctggggc tgcagtggag caacatcggg aacagtccca 3780
   cggaagggga cgaattcagc ttcctgctgt ccatgcagat gatgctcctt gatgctgctg 3840
   tctatggctt actcgcttgg taccttgatc aggtgtttcc aggagactat ggaaccccac     3900
   ttccttggta ctttcttcta caagagtcgt attggcttgg cggtgaaggg tgttcaacca     3960
   gagaagaaag agccctggaa aagaccgagc ccctaacaga ggaaacggag gatccagagc     4020
   acccagaagg aatacacgac tccttctttg aacgtgagca tccagggtgg gttcctgggg     4080
   tatgcgtgaa gaatctggta aagatttttg agccctgtgg ccggccagct gtggaccgtc     4140
   tgaacatcac cttctacgag aaccagatca ccgcattcct gggccacaat ggagctggga     4200
   aaaccaccac cttgtaagta tcaaggttac aagacaggtt taaggagacc aatagaaact     4260
   gggcttgtcg agacagagaa gactcttgcg tttctgtgat cctaggtgga ggccgaaagt     4320
   acatgtttcg catgggaacc ccagaccctg agtacccaga tgactacagc caaggtggga     4380
   ccaggctgga cgggaagaat ctggtgcagg aatggctggc gaagcgccag ggtgcccggt     4440
   acgtgtggaa ccgcactgag ctcatgcagg cttccctgga cccgtctgtg acccatctca     4500
   tgggtctctt tgagcctgga gacatgaaat acgagatcca ccgagactcc acactggacc     4560
   cctccctgat ggacaattga ggaaccccta gtgatggagt tggccactcc ctctctgcgc     4620
   gctcgctcgc tcactgaggc cgggcgacca aaggtcgccc gacgcccggg ctttgcccgg     4680
   gcggcctcag tgagcgagcg agcgcgcag                                       4709
<210> 64
   <211> 4871
   <212> **DNA**
   <213> **Artificial** Sequence
<220>
   <223> synthetic
<400> 64
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct ggatccgtga tcctaggtgg aggccgaaag tacatgtttc gcatgggaac 180
   cccagaccct gagtacccag atgactacag ccaaggtggg accaggctgg acgggaagaa 240
   tctggtgcag gaatggctgg cgaagcgcca gggtgcccgg tacgtgtgga accgcactga 300
   gctcatgcag gcttccctgg acccgtctgt gacccatctc atgggtctct ttgagcctgg 360
   agacatgaaa tacgagatcc accgagactc cacactggac ccctccctga tggagatagg 420
   cacctattgg tcttactgac atccactttg cctttctctc cacaggtcca tcctgacggg 480
   tctgttgcca ccaacctctg ggactgtgct cgttggggga agggacattg aaaccagcct 540
   ggatgcagtc cggcagagcc ttggcatgtg tccacagcac aacatcctgt tccaccacct 600
   cacggtggct gagcacatgc tgttctatgc ccagctgaaa ggaaagtccc aggaggaggc 660
   ccagctggag atggaagcca tgttggagga cacaggcctc caccacaagc ggaatgaaga 720
   ggctcaggac ctatcaggtg gcatgcagag aaagctgtcg gttgccattg cctttgtggg 780
   agatgccaag gtggtgattc tggacgaacc cacctctggg gtggaccctt actcgagacg 840
   ctcaatctgg gatctgctcc tgaagtatcg ctcaggcaga accatcatca tgtccactca 900
   ccacatggac gaggccgacc tccttgggga ccgcattgcc atcattgccc agggaaggct 960
   ctactgctca ggcaccccac tcttcctgaa gaactgcttt ggcacaggct tgtacttaac 1020
   cttggtgcgc aagatgaaaa acatccagag ccaaaggaaa ggcagtgagg ggacctgcag 1080
   ctgctcgtct aagggtttct ccaccacgtg tccagcccac gtcgatgacc taactccaga 1140
   acaagtcctg gatggggatg taaatgagct gatggatgta gttctccacc atgttccaga 1200
   ggcaaagctg gtggagtgca ttggtcaaga acttatcttc cttcttccaa ataagaactt 1260
   caagcacaga gcatatgcca gccttttcag agagctggag gagacgctgg ctgaccttgg 1320
   tctcagcagt tttggaattt ctgacactcc cctggaagag atttttctga aggtcacgga 1380
   ggattctgat tcaggacctc tgtttgcggg tggcgctcag cagaaaagag aaaacgtcaa 1440
   cccccgacac ccctgcttgg gtcccagaga gaaggctgga cagacacccc aggactccaa 1500
   tgtctgctcc ccaggggcgc cggctgctca cccagagggc cagcctcccc cagagccaga 1560
   gtgcccaggc ccgcagctca acacggggac acagctggtc ctccagcatg tgcaggcgct 1620
   gctggtcaag agattccaac acaccatccg cagccacaag gacttcctgg cgcagatcgt 1680
   gctcccggct acctttgtgt ttttggctct gatgctttct attgttatcc ctccttttgg 1740
   cgaatacccc gctttgaccc ttcacccctg gatatatggg cagcagtaca ccttcttcag 1800
   catggatgaa ccaggcagtg agcagttcac ggtacttgca gacgtcctcc tgaataagcc 1860
   aggctttggc aaccgctgcc tgaaggaagg gtggcttccg gagtacccct gtggcaactc 1920
   aacaccctgg aagactcctt ctgtgtcccc aaacatcacc cagctgttcc agaagcagaa 1980
   atggacacag gtcaaccctt caccatcctg caggtgcagc accagggaga agctcaccat 2040
   gctgccagag tgccccgagg gtgccggggg cctcccgccc ccccagagaa cacagcgcag 2100
   cacggaaatt ctacaagacc tgacggacag gaacatctcc gacttcttgg taaaaacgta 2160
   tcctgctctt ataagaagca gcttaaagag caaattctgg gtcaatgaac agaggtatgg 2220
   aggaatttcc attggaggaa agctcccagt cgtccccatc acgggggaag cacttgttgg 2280
   gtttttaagc gaccttggcc ggatcatgaa tgtgagcggg ggccctatca ctagagaggc 2340
   ctctaaagaa atacctgatt tccttaaaca tctagaaact gaagacaaca ttaaggtgtg 2400
   gtttaataac aaaggctggc atgccctggt cagctttctc aatgtggccc acaacgccat 2460
   cttacgggcc agcctgccta aggacagaag ccccgaggag tatggaatca ccgtcattag 2520
   ccaacccctg aacctgacca aggagcagct ctcagagatt acagtgctga ccacttcagt 2580
   ggatgctgtg gttgccatct gcgtgatttt ctccatgtcc ttcgtcccag ccagctttgt 2640
   cctttatttg atccaggagc gggtgaacaa atccaagcac ctccagttta tcagtggagt 2700
   gagccccacc acctactggg taaccaactt cctctgggac atcatgaatt attccgtgag 2760
   tgctgggctg gtggtgggca tcttcatcgg gtttcagaag aaagcctaca cttctccaga 2820
   aaaccttcct gcccttgtgg cactgctcct gctgtatgga tgggcggtca ttcccatgat 2880
   gtacccagca tccttcctgt ttgatgtccc cagcacagcc tatgtggctt tatcttgtgc 2940
   taatctgttc atcggcatca acagcagtgc tattaccttc atcttggaat tatttgagaa 3000
   taaccggacg ctgctcaggt tcaacgccgt gctgaggaag ctgctcattg tcttccccca 3060
   cttctgcctg ggccggggcc tcattgacct tgcactgagc caggctgtga cagatgtcta 3120
   tgcccggttt ggtgaggagc actctgcaaa tccgttccac tgggacctga ttgggaagaa 3180
   cctgtttgcc atggtggtgg aaggggtggt gtacttcctc ctgaccctgc tggtccagcg 3240
   ccacttcttc ctctcccaat ggattgccga gcccactaag gagcccattg ttgatgaaga 3300
   tgatgatgtg gctgaagaaa gacaaagaat tattactggt ggaaataaaa ctgacatctt 3360
   aaggctacat gaactaacca agatttatcc aggcacctcc agcccagcag tggacaggct 3420
   gtgtgtcgga gttcgccctg gagagtgctt tggcctcctg ggagtgaatg gtgccggcaa 3480
   aacaaccaca ttcaagatgc tcactgggga caccacagtg acctcagggg atgccaccgt 3540
   agcaggcaag agtattttaa ccaatatttc tgaagtccat caaaatatgg gctactgtcc 3600
   tcagtttgat gcaatcgatg agctgctcac aggacgagaa catctttacc tttatgcccg 3660
   gcttcgaggt gtaccagcag aagaaatcga aaaggttgca aactggagta ttaagagcct 3720
   gggcctgact gtctacgccg actgcctggc tggcacgtac agtgggggca acaagcggaa 3780
   actctccaca gccatcgcac tcattggctg cccaccgctg gtgctgctgg atgagcccac 3840
   cacagggatg gacccccagg cacgccgcat gctgtggaac gtcatcgtga gcatcatcag 3900
   agaagggagg gctgtggtcc tcacatccca cagcatggaa gaatgtgagg cactgtgtac 3960
   ccggctggcc atcatggtaa agggcgcctt tcgatgtatg ggcaccattc agcatctcaa 4020
   gtccaaattt ggagatggct atatcgtcac aatgaagatc aaatccccga aggacgacct 4080
   gcttcctgac ctgaaccctg tggagcagtt cttccagggg aacttcccag gcagtgtgca 4140
   gagggagagg cactacaaca tgctccagtt ccaggtctcc tcctcctccc tggcgaggat 4200
   cttccagctc ctcctctccc acaaggacag cctgctcatc gaggagtact cagtcacaca 4260
   gaccacactg gaccaggtgt ttgtaaattt tgctaaacag cagactgaaa gtcatgacct 4320
   ccctctgcac cctcgagctg ctggagccag tcgacaagcc caggacgact acaaagacca 4380
   tgacggtgat tataaagatc atgacatcga ctacaaggat gacgatgaca agtgagcggc 4440
   cgcttcgagc agacatgata agatacattg atgagtttgg acaaaccaca actagaatgc      4500
   agtgaaaaaa atgctttatt tgtgaaattt gtgatgctat tgctttattt gtaaccatta      4560
   taagctgcaa taaacaagtt aacaacaaca attgcattca ttttatgttt caggttcagg      4620
   gggagatgtg ggaggttttt taaagcaagt aaaacctcta caaatgtggt aaaatcgata      4680
   aggatcttcc tagagcatgg ctacgtagat aagtagcatg gcgggttaat cattaactac      4740
   aaggaacccc tagtgatgga gttggccact ccctctctgc gcgctcgctc gctcactgag      4800
   gccgggcgac caaaggtcgc ccgacgcccg ggctttgccc gggcggcctc agtgagcgag      4860
   cgagcgcgca g                                                           4871
<210> 65
   <211> 4073
   <212> DNA
   <213> Artificial Sequence
<220>
   <223> synthetic
<400> 65
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct tgtagttaat gattaacccg ccatgctact tatctacgta gccatgctct     180
   aggaagatct tcaatattgg ccattagcca tattattcat tggttatata gcataaatca     240
   atattggcta ttggccattg catacgttgt atctatatca taatatgtac atttatattg     300
   gctcatgtcc aatatgaccg ccatgttggc attgattatt gactagtggg ccccagaagc     360
   ctggtggttg tttgtccttc tcaggggaaa agtgaggcgg ccccttggag gaaggggccg     420
   ggcagaatga tctaatcgga ttccaagcag ctcaggggat tgtctttttc tagcaccttc     480
   ttgccactcc taagcgtcct ccgtgacccc ggctgggatt tagcctggtg ctgtgtcagc     540
   cccgggctcc caggggcttc ccagtggtcc ccaggaaccc tcgacagggc cagggcgtct     600
   ctctcgtcca gcaagggcag ggacgggcca caggcaaggg cgcggccgcc atgggcttcg     660
   tgagacagat acagcttttg ctctggaaga actggaccct gcggaaaagg caaaagattc     720
   gctttgtggt ggaactcgtg tggcctttat ctttatttct ggtcttgatc tggttaagga     780
   atgccaaccc gctctacagc catcatgaat gccatttccc caacaaggcg atgccctcag     840
   caggaatgct gccgtggctc caggggatct tctgcaatgt gaacaatccc tgttttcaaa     900
   gccccacccc aggagaatct cctggaattg tgtcaaacta taacaactcc atcttggcaa     960
   gggtatatcg agattttcaa gaactcctca tgaatgcacc agagagccag caccttggcc    1020
   gtatttggac agagctacac atcttgtccc aattcatgga caccctccgg actcacccgg    1080
   agagaattgc aggaagagga attcgaataa gggatatctt gaaagatgaa gaaacactga    1140
   cactatttct cattaaaaac atcggcctgt ctgactcagt ggtctacctt ctgatcaact 1200
   ctcaagtccg tccagagcag ttcgctcatg gagtcccgga cctggcgctg aaggacatcg 1260
   cctgcagcga ggccctcctg gagcgcttca tcatcttcag ccagagacgc ggggcaaaga 1320
   cggtgcgcta tgccctgtgc tccctctccc agggcaccct acagtggata gaagacactc 1380
   tgtatgccaa cgtggacttc ttcaagctct tccgtgtgct tcccacactc ctagacagcc 1440
   gttctcaagg tatcaatctg agatcttggg gaggaatatt atctgatatg tcaccaagaa 1500
   ttcaagagtt tatccatcgg ccgagtatgc aggacttgct gtgggtgacc aggcccctca 1560
   tgcagaatgg tggtccagag acctttacaa agctgatggg catcctgtct gacctcctgt 1620
   gtggctaccc cgagggaggt ggctctcggg tgctctcctt caactggtat gaagacaata 1680
   actataaggc ctttctgggg attgactcca caaggaagga tcctatctat tcttatgaca 1740
   gaagaacaac atccttttgt aatgcattga tccagagcct ggagtcaaat cctttaacca 1800
   aaatcgcttg gagggcggca aagcctttgc tgatgggaaa aatcctgtac actcctgatt 1860
   cacctgcagc acgaaggata ctgaagaatg ccaactcaac ttttgaagaa ctggaacacg 1920
   ttaggaagtt ggtcaaagcc tgggaagaag tagggcccca gatctggtac ttctttgaca 1980
   acagcacaca gatgaacatg atcagagata ccctggggaa cccaacagta aaagactttt 2040
   tgaataggca gcttggtgaa gaaggtatta ctgctgaagc catcctaaac ttcctctaca 2100
   agggccctcg ggaaagccag gctgacgaca tggccaactt cgactggagg gacatattta 2160
   acatcactga tcgcaccctc cgccttgtca atcaatacct ggagtgcttg gtcctggata 2220
   agtttgaaag ctacaatgat gaaactcagc tcacccaacg tgccctctct ctactggagg 2280
   aaaacatgtt ctgggccgga gtggtattcc ctgacatgta tccctggacc agctctctac 2340
   caccccacgt gaagtataag atccgaatgg acatagacgt ggtggagaaa accaataaga 2400
   ttaaagacag gtattgggat tctggtccca gagctgatcc cgtggaagat ttccggtaca 2460
   tctggggcgg gtttgcctat ctgcaggaca tggttgaaca ggggatcaca aggagccagg 2520
   tgcaggcgga ggctccagtt ggaatctacc tccagcagat gccctacccc tgcttcgtgg 2580
   acgattcttt catgatcatc ctgaaccgct gtttccctat cttcatggtg ctggcatgga 2640
   tctactctgt ctccatgact gtgaagagca tcgtcttgga gaaggagttg cgactgaagg 2700
   agaccttgaa aaatcagggt gtctccaatg cagtgatttg gtgtacctgg ttcctggaca 2760
   gcttctccat catgtcgatg agcatcttcc tcctgacgat attcatcatg catggaagaa 2820
   tcctacatta cagcgaccca ttcatcctct tcctgttctt gttggctttc tccactgcca 2880
   ccatcatgct gtgctttctg ctcagcacct tcttctccaa ggccagtctg gcagcagcct 2940
   gtagtggtgt catctatttc accctctacc tgccacacat cctgtgcttc gcctggcagg 3000
   accgcatgac cgctgagctg aagaaggctg tgagcttact gtctccggtg gcatttggat     3060
   ttggcactga gtacctggtt cgctttgaag agcaaggcct ggggctgcag tggagcaaca     3120
   tcgggaacag tcccacggaa ggggacgaat tcagcttcct gctgtccatg cagatgatgc     3180
   tccttgatgc tgctgtctat ggcttactcg cttggtacct tgatcaggtg tttccaggag     3240
   actatggaac cccacttcct tggtactttc ttctacaaga gtcgtattgg cttggcggtg     3300
   aagggtgttc aaccagagaa gaaagagccc tggaaaagac cgagccccta acagaggaaa     3360
   cggaggatcc agagcaccca gaaggaatac acgactcctt ctttgaacgt gagcatccag     3420
   ggtgggttcc tggggtatgc gtgaagaatc tggtaaagat ttttgagccc tgtggccggc     3480
   cagctgtgga ccgtctgaac atcaccttct acgagaacca gatcaccgca ttcctgggcc     3540
   acaatggagc tgggaaaacc accaccttgt aagtatcaag gttacaagac aggtttaagg     3600
   agaccaatag aaactgggct tgtcgagaca gagaagactc ttgcgtttct ccccgggtgc     3660
   gcggcgtcgg tggtgccggc ggggggcgcc aggtcgcagg cggtgtaggg ctccaggcag     3720
   gcggcgaagg ccatgacgtg cgctatgaag gtctgctcct gcacgccgtg aaccaggtgc     3780
   gcctgcgggc cgcgcgcgaa caccgccacg tcctcgcctg cgtgggtctc ttcgtccagg     3840
   ggcactgctg actgctgccg atactcgggg ctcccgctct cgctctcggt aacatccggc     3900
   cgggcgccgt ccttgagcac atagcctgga ccgtttccaa ttgaggaacc cctagtgatg     3960
   gagttggcca ctccctctct gcgcgctcgc tcgctcactg aggccgggcg accaaaggtc     4020
   gcccgacgcc cgggctttgc ccgggcggcc tcagtgagcg agcgagcgcg cag            4073
<210> 66
   <211> 4074
   <212> DNA
   <213> Artificial Sequence
<220>
   <223> synthetic
<400> 66
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct tgtagttaat gattaacccg ccatgctact tatctacgta gccatgctct     180
   aggaagatct tcaatattgg ccattagcca tattattcat tggttatata gcataaatca     240
   atattggcta ttggccattg catacgttgt atctatatca taatatgtac atttatattg     300
   gctcatgtcc aatatgaccg ccatgttggc attgattatt gactagtggg ccccagaagc     360
   ctggtggttg tttgtccttc tcaggggaaa agtgaggcgg ccccttggag gaaggggccg     420
   ggcagaatga tctaatcgga ttccaagcag ctcaggggat tgtctttttc tagcaccttc     480
   ttgccactcc taagcgtcct ccgtgacccc ggctgggatt tagcctggtg ctgtgtcagc     540
   cccgggctcc caggggcttc ccagtggtcc ccaggaaccc tcgacagggc cagggcgtct 600
   ctctcgtcca gcaagggcag ggacgggcca caggcaaggg cgcggccgcc atgggcttcg 660
   tgagacagat acagcttttg ctctggaaga actggaccct gcggaaaagg caaaagattc 720
   gctttgtggt ggaactcgtg tggcctttat ctttatttct ggtcttgatc tggttaagga 780
   atgccaaccc gctctacagc catcatgaat gccatttccc caacaaggcg atgccctcag 840
   caggaatgct gccgtggctc caggggatct tctgcaatgt gaacaatccc tgttttcaaa 900
   gccccacccc aggagaatct cctggaattg tgtcaaacta taacaactcc atcttggcaa 960
   gggtatatcg agattttcaa gaactcctca tgaatgcacc agagagccag caccttggcc 1020
   gtatttggac agagctacac atcttgtccc aattcatgga caccctccgg actcacccgg 1080
   agagaattgc aggaagagga attcgaataa gggatatctt gaaagatgaa gaaacactga 1140
   cactatttct cattaaaaac atcggcctgt ctgactcagt ggtctacctt ctgatcaact 1200
   ctcaagtccg tccagagcag ttcgctcatg gagtcccgga cctggcgctg aaggacatcg 1260
   cctgcagcga ggccctcctg gagcgcttca tcatcttcag ccagagacgc ggggcaaaga 1320
   cggtgcgcta tgccctgtgc tccctctccc agggcaccct acagtggata gaagacactc 1380
   tgtatgccaa cgtggacttc ttcaagctct tccgtgtgct tcccacactc ctagacagcc 1440
   gttctcaagg tatcaatctg agatcttggg gaggaatatt atctgatatg tcaccaagaa 1500
   ttcaagagtt tatccatcgg ccgagtatgc aggacttgct gtgggtgacc aggcccctca 1560
   tgcagaatgg tggtccagag acctttacaa agctgatggg catcctgtct gacctcctgt 1620
   gtggctaccc cgagggaggt ggctctcggg tgctctcctt caactggtat gaagacaata 1680
   actataaggc ctttctgggg attgactcca caaggaagga tcctatctat tcttatgaca 1740
   gaagaacaac atccttttgt aatgcattga tccagagcct ggagtcaaat cctttaacca 1800
   aaatcgcttg gagggcggca aagcctttgc tgatgggaaa aatcctgtac actcctgatt 1860
   cacctgcagc acgaaggata ctgaagaatg ccaactcaac ttttgaagaa ctggaacacg 1920
   ttaggaagtt ggtcaaagcc tgggaagaag tagggcccca gatctggtac ttctttgaca 1980
   acagcacaca gatgaacatg atcagagata ccctggggaa cccaacagta aaagactttt 2040
   tgaataggca gcttggtgaa gaaggtatta ctgctgaagc catcctaaac ttcctctaca 2100
   agggccctcg ggaaagccag gctgacgaca tggccaactt cgactggagg gacatattta 2160
   acatcactga tcgcaccctc cgccttgtca atcaatacct ggagtgcttg gtcctggata 2220
   agtttgaaag ctacaatgat gaaactcagc tcacccaacg tgccctctct ctactggagg 2280
   aaaacatgtt ctgggccgga gtggtattcc ctgacatgta tccctggacc agctctctac 2340
   caccccacgt gaagtataag atccgaatgg acatagacgt ggtggagaaa accaataaga 2400
   ttaaagacag gtattgggat tctggtccca gagctgatcc cgtggaagat ttccggtaca 2460
   tctggggcgg gtttgcctat ctgcaggaca tggttgaaca ggggatcaca aggagccagg 2520
   tgcaggcgga ggctccagtt ggaatctacc tccagcagat gccctacccc tgcttcgtgg 2580
   acgattcttt catgatcatc ctgaaccgct gtttccctat cttcatggtg ctggcatgga 2640
   tctactctgt ctccatgact gtgaagagca tcgtcttgga gaaggagttg cgactgaagg 2700
   agaccttgaa aaatcagggt gtctccaatg cagtgatttg gtgtacctgg ttcctggaca 2760
   gcttctccat catgtcgatg agcatcttcc tcctgacgat attcatcatg catggaagaa 2820
   tcctacatta cagcgaccca ttcatcctct tcctgttctt gttggctttc tccactgcca 2880
   ccatcatgct gtgctttctg ctcagcacct tcttctccaa ggccagtctg gcagcagcct 2940
   gtagtggtgt catctatttc accctctacc tgccacacat cctgtgcttc gcctggcagg 3000
   accgcatgac cgctgagctg aagaaggctg tgagcttact gtctccggtg gcatttggat 3060
   ttggcactga gtacctggtt cgctttgaag agcaaggcct ggggctgcag tggagcaaca 3120
   tcgggaacag tcccacggaa ggggacgaat tcagcttcct gctgtccatg cagatgatgc 3180
   tccttgatgc tgctgtctat ggcttactcg cttggtacct tgatcaggtg tttccaggag 3240
   actatggaac cccacttcct tggtactttc ttctacaaga gtcgtattgg cttggcggtg 3300
   aagggtgttc aaccagagaa gaaagagccc tggaaaagac cgagccccta acagaggaaa 3360
   cggaggatcc agagcaccca gaaggaatac acgactcctt ctttgaacgt gagcatccag 3420
   ggtgggttcc tggggtatgc gtgaagaatc tggtaaagat ttttgagccc tgtggccggc 3480
   cagctgtgga ccgtctgaac atcaccttct acgagaacca gatcaccgca ttcctgggcc 3540
   acaatggagc tgggaaaacc accaccttgt aagtatcaag gttacaagac aggtttaagg 3600
   agaccaatag aaactgggct tgtcgagaca gagaagactc ttgcgtttct cgcagggcag 3660
   cctctgtcat ctccatcagg gaggggtcca gtgtggagtc tcggtggatc tcgtatttca 3720
   tgtctccagg ctcaaagaga cccatgagat gggtcacaga cgggtccagg gaagcctgca 3780
   tgagctcagt gcggttccac acataccggg caccctggcg cttcgccagc cattcctgca 3840
   ccagattctt cccgtccagc ctggtcccac cttggctgta gtcatctggg tactcagggt 3900
   ctggggttcc catgcgaaac atgtactttc ggcctccaca attgaggaac ccctagtgat 3960
   ggagttggcc actccctctc tgcgcgctcg ctcgctcact gaggccgggc gaccaaaggt 4020
   cgcccgacgc ccgggctttg cccgggcggc ctcagtgagc gagcgagcgc gcag 4074
<210> 67
   <211> 4636
   <212> DNA
   <213> Artificial Sequence
<220>
   <223> synthetic
<400> 67
   ctctcccccc tgtcgcgttc gctcgctcgc tggctcgttt gggggggtgg cagctcaaag      60
   agctgccaga cgacggccct ctggccgtcg cccccccaaa cgagccagcg agcgagcgaa     120
   cgcgacaggg gggagagtgc cacactctca agcaaggggg ttttgtaagc agtgagctag     180
   cctgaattcc agcacactgg cggccgttac tagtggatct tcaatattgg ccattagcca     240
   tattattcat tggttatata gcataaatca atattggcta ttggccattg catacgttgt     300
   atctatatca taatatgtac atttatattg gctcatgtcc aatatgaccg ccatgttggc     360
   attgattatt gactagttat taatagtaat caattacggg gtcattagtt catagcccat     420
   atatggagtt ccgcgttaca taacttacgg taaatggccc gcctggctga ccgcccaacg     480
   acccccgccc attgacgtca ataatgacgt atgttcccat agtaacgcca atagggactt     540
   tccattgacg tcaatgggtg gagtatttac ggtaaactgc ccacttggca gtacatcaag     600
   tgtatcatat gccaagtccg ccccctattg acgtcaatga cggtaaatgg cccgcctggc     660
   attatgccca gtacatgacc ttacgggact ttcctacttg gcagtacatc tacgtattag     720
   tcatcgctat taccatggtg atgcggtttt ggcagtacac caatgggcgt ggatagcggt     780
   ttgactcacg gggatttcca agtctccacc ccattgacgt caatgggagt ttgttttggc     840
   accaaaatca acgggacttt ccaaaatgtc gtaataaccc cgccccgttg acgcaaatgg     900
   gcggtaggcg tgtacggtgg gaggtctata taagcagagc tcgtttagtg aaccgtcaga     960
   tcactagaag ctttattgcg gtagtttatc acagttaaat tgctaacgca gtcagtgctt    1020
   ctgacacaac agtctcgaac ttaagctgca gaagttggtc gtgaggcact gggcaggtaa    1080
   gtatcaaggt tacaagacag gtttaaggag accaatagaa actgggcttg tcgagacaga    1140
   gaagactctt gcgtttctga taggcaccta ttggtcttac tgacatccac tttgcctttc    1200
   tctccacagg tgtccactcc cagttcaatt acagctctta aggctagagt acttaatacg    1260
   actcactata ggctagcctc gagaattcac gcgtggtacc tctagagtcg acccgggcgg    1320
   ccgccatggg cttcgtgaga cagatacagc ttttgctctg gaagaactgg accctgcgga    1380
   aaaggcaaaa gattcgcttt gtggtggaac tcgtgtggcc tttatcttta tttctggtct    1440
   tgatctggtt aaggaatgcc aacccgctct acagccatca tgaatgccat ttccccaaca    1500
   aggcgatgcc ctcagcagga atgctgccgt ggctccaggg gatcttctgc aatgtgaaca    1560
   atccctgttt tcaaagcccc accccaggag aatctcctgg aattgtgtca aactataaca    1620
   actccatctt ggcaagggta tatcgagatt ttcaagaact cctcatgaat gcaccagaga    1680
   gccagcacct tggccgtatt tggacagagc tacacatctt gtcccaattc atggacaccc    1740
   tccggactca cccggagaga attgcaggaa gaggaattcg aataagggat atcttgaaag    1800
   atgaagaaac actgacacta tttctcatta aaaacatcgg cctgtctgac tcagtggtct 1860
   accttctgat caactctcaa gtccgtccag agcagttcgc tcatggagtc ccggacctgg 1920
   cgctgaagga catcgcctgc agcgaggccc tcctggagcg cttcatcatc ttcagccaga 1980
   gacgcggggc aaagacggtg cgctatgccc tgtgctccct ctcccagggc accctacagt 2040
   ggatagaaga cactctgtat gccaacgtgg acttcttcaa gctcttccgt gtgcttccca 2100
   cactcctaga cagccgttct caaggtatca atctgagatc ttggggagga atattatctg 2160
   atatgtcacc aagaattcaa gagtttatcc atcggccgag tatgcaggac ttgctgtggg 2220
   tgaccaggcc cctcatgcag aatggtggtc cagagacctt tacaaagctg atgggcatcc 2280
   tgtctgacct cctgtgtggc taccccgagg gaggtggctc tcgggtgctc tccttcaact 2340
   ggtatgaaga caataactat aaggcctttc tggggattga ctccacaagg aaggatccta 2400
   tctattctta tgacagaaga acaacatcct tttgtaatgc attgatccag agcctggagt 2460
   caaatccttt aaccaaaatc gcttggaggg cggcaaagcc tttgctgatg ggaaaaatcc 2520
   tgtacactcc tgattcacct gcagcacgaa ggatactgaa gaatgccaac tcaacttttg 2580
   aagaactgga acacgttagg aagttggtca aagcctggga agaagtaggg ccccagatct 2640
   ggtacttctt tgacaacagc acacagatga acatgatcag agataccctg gggaacccaa 2700
   cagtaaaaga ctttttgaat aggcagcttg gtgaagaagg tattactgct gaagccatcc 2760
   taaacttcct ctacaagggc cctcgggaaa gccaggctga cgacatggcc aacttcgact 2820
   ggagggacat atttaacatc actgatcgca ccctccgcct tgtcaatcaa tacctggagt 2880
   gcttggtcct ggataagttt gaaagctaca atgatgaaac tcagctcacc caacgtgccc 2940
   tctctctact ggaggaaaac atgttctggg ccggagtggt attccctgac atgtatccct 3000
   ggaccagctc tctaccaccc cacgtgaagt ataagatccg aatggacata gacgtggtgg 3060
   agaaaaccaa taagattaaa gacaggtatt gggactacaa agaccatgac ggtgattata 3120
   aagatcatga catcgactac aaggatgacg atgacaagga ttctggtccc agagctgatc 3180
   ccgtggaaga tttccggtac atctggggcg ggtttgccta tctgcaggac atggttgaac 3240
   aggggatcac aaggagccag gtgcaggcgg aggctccagt tggaatctac ctccagcaga 3300
   tgccctaccc ctgcttcgtg gacgattctt tcatgatcat cctgaaccgc tgtttcccta 3360
   tcttcatggt gctggcatgg atctactctg tctccatgac tgtgaagagc atcgtcttgg 3420
   agaaggagtt gcgactgaag gagaccttga aaaatcaggg tgtctccaat gcagtgattt 3480
   ggtgtacctg gttcctggac agcttctcca tcatgtcgat gagcatcttc ctcctgacga 3540
   tattcatcat gcatggaaga atcctacatt acagcgaccc attcatcctc ttcctgttct 3600
   tgttggcttt ctccactgcc accatcatgc tgtgctttct gctcagcacc ttcttctcca 3660
   aggccagtct ggcagcagcc tgtagtggtg tcatctattt caccctctac ctgccacaca 3720
   tcctgtgctt cgcctggcag gaccgcatga ccgctgagct gaagaaggct gtgagcttac 3780
   tgtctccggt ggcatttgga tttggcactg agtacctggt tcgctttgaa gagcaaggcc 3840
   tggggctgca gtggagcaac atcgggaaca gtcccacgga aggggacgaa ttcagcttcc 3900
   tgctgtccat gcagatgatg ctccttgatg ctgctgtcta tggcttactc gcttggtacc 3960
   ttgatcaggt gtttccagga gactatggaa ccccacttcc ttggtacttt cttctacaag 4020
   agtcgtattg gcttggcggt gaagggtgtt caaccagaga agaaagagcc ctggaaaaga 4080
   ccgagcccct aacagaggaa acggaggatc cagagcaccc agaaggaata cacgactcct 4140
   tctttgaacg tgagcatcca gggtgggttc ctggggtatg cgtgaagaat ctggtaaaga 4200
   tttttgagcc ctgtggccgg ccagctgtgg accgtctgaa catcaccttc tacgagaacc 4260
   agatcaccgc attcctgggc cacaatggag ctgggaaaac caccaccttg taagtatcaa 4320
   ggttacaaga caggtttaag gagaccaata gaaactgggc ttgtcgagac agagaagact 4380
   cttgcgtttc tgggattttt ccgatttcgg cctattggtt aaaaaatgag ctgatttaac 4440
   aaaaatttaa cgcgaatttt aacaaaatat taacgtttat aatttcaggt ggcatctttc 4500
   caattgagga acccctagtg atggagttgg ccactccctc tctgcgcgct cgctcgctca 4560
   ctgaggccgg gcgaccaaag gtcgcccgac gcccgggctt tgcccgggcg gcctcagtga 4620
   gcgagcgagc gcgcag 4636
<210> 68
   <211> 4731
   <212> DNA
   <213> Artificial Sequence
<220>
   <223> synthetic
<400> 68
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatccggga tttttccgat ttcggcctat tggttaaaaa atgagctgat     180
   ttaacaaaaa tttaacgcga attttaacaa aatattaacg tttataattt caggtggcat     240
   ctttcgatag gcacctattg gtcttactga catccacttt gcctttctct ccacaggtcc     300
   atcctgacgg gtctgttgcc accaacctct gggactgtgc tcgttggggg aagggacatt     360
   gaaaccagcc tggatgcagt ccggcagagc cttggcatgt gtccacagca caacatcctg     420
   ttccaccacc tcacggtggc tgagcacatg ctgttctatg cccagctgaa aggaaagtcc     480
   caggaggagg cccagctgga gatggaagcc atgttggagg acacaggcct ccaccacaag     540
   cggaatgaag aggctcagga cctatcaggt ggcatgcaga gaaagctgtc ggttgccatt     600
   gcctttgtgg gagatgccaa ggtggtgatt ctggacgaac ccacctctgg ggtggaccct 660
   tactcgagac gctcaatctg ggatctgctc ctgaagtatc gctcaggcag aaccatcatc 720
   atgtccactc accacatgga cgaggccgac ctccttgggg accgcattgc catcattgcc 780
   cagggaaggc tctactgctc aggcacccca ctcttcctga agaactgctt tggcacaggc 840
   ttgtacttaa ccttggtgcg caagatgaaa aacatccaga gccaaaggaa aggcagtgag 900
   gggacctgca gctgctcgtc taagggtttc tccaccacgt gtccagccca cgtcgatgac 960
   ctaactccag aacaagtcct ggatggggat gtaaatgagc tgatggatgt agttctccac 1020
   catgttccag aggcaaagct ggtggagtgc attggtcaag aacttatctt ccttcttcca 1080
   aataagaact tcaagcacag agcatatgcc agccttttca gagagctgga ggagacgctg 1140
   gctgaccttg gtctcagcag ttttggaatt tctgacactc ccctggaaga gatttttctg 1200
   aaggtcacgg aggattctga ttcaggacct ctgtttgcgg gtggcgctca gcagaaaaga 1260
   gaaaacgtca acccccgaca cccctgcttg ggtcccagag agaaggctgg acagacaccc 1320
   caggactcca atgtctgctc cccaggggcg ccggctgctc acccagaggg ccagcctccc 1380
   ccagagccag agtgcccagg cccgcagctc aacacgggga cacagctggt cctccagcat 1440
   gtgcaggcgc tgctggtcaa gagattccaa cacaccatcc gcagccacaa ggacttcctg 1500
   gcgcagatcg tgctcccggc tacctttgtg tttttggctc tgatgctttc tattgttatc 1560
   cctccttttg gcgaataccc cgctttgacc cttcacccct ggatatatgg gcagcagtac 1620
   accttcttca gcatggatga accaggcagt gagcagttca cggtacttgc agacgtcctc 1680
   ctgaataagc caggctttgg caaccgctgc ctgaaggaag ggtggcttcc ggagtacccc 1740
   tgtggcaact caacaccctg gaagactcct tctgtgtccc caaacatcac ccagctgttc 1800
   cagaagcaga aatggacaca ggtcaaccct tcaccatcct gcaggtgcag caccagggag 1860
   aagctcacca tgctgccaga gtgccccgag ggtgccgggg gcctcccgcc cccccagaga 1920
   acacagcgca gcacggaaat tctacaagac ctgacggaca ggaacatctc cgacttcttg 1980
   gtaaaaacgt atcctgctct tataagaagc agcttaaaga gcaaattctg ggtcaatgaa 2040
   cagaggtatg gaggaatttc cattggagga aagctcccag tcgtccccat cacgggggaa 2100
   gcacttgttg ggtttttaag cgaccttggc cggatcatga atgtgagcgg gggccctatc 2160
   actagagagg cctctaaaga aatacctgat ttccttaaac atctagaaac tgaagacaac 2220
   attaaggtgt ggtttaataa caaaggctgg catgccctgg tcagctttct caatgtggcc 2280
   cacaacgcca tcttacgggc cagcctgcct aaggacagaa gccccgagga gtatggaatc 2340
   accgtcatta gccaacccct gaacctgacc aaggagcagc tctcagagat tacagtgctg 2400
   accacttcag tggatgctgt ggttgccatc tgcgtgattt tctccatgtc cttcgtccca 2460
   gccagctttg tcctttattt gatccaggag cgggtgaaca aatccaagca cctccagttt 2520
   atcagtggag tgagccccac cacctactgg gtaaccaact tcctctggga catcatgaat 2580
   tattccgtga gtgctgggct ggtggtgggc atcttcatcg ggtttcagaa gaaagcctac 2640
   acttctccag aaaaccttcc tgcccttgtg gcactgctcc tgctgtatgg atgggcggtc 2700
   attcccatga tgtacccagc atccttcctg tttgatgtcc ccagcacagc ctatgtggct 2760
   ttatcttgtg ctaatctgtt catcggcatc aacagcagtg ctattacctt catcttggaa 2820
   ttatttgaga ataaccggac gctgctcagg ttcaacgccg tgctgaggaa gctgctcatt 2880
   gtcttccccc acttctgcct gggccggggc ctcattgacc ttgcactgag ccaggctgtg 2940
   acagatgtct atgcccggtt tggtgaggag cactctgcaa atccgttcca ctgggacctg 3000
   attgggaaga acctgtttgc catggtggtg gaaggggtgg tgtacttcct cctgaccctg 3060
   ctggtccagc gccacttctt cctctcccaa tggattgccg agcccactaa ggagcccatt 3120
   gttgatgaag atgatgatgt ggctgaagaa agacaaagaa ttattactgg tggaaataaa 3180
   actgacatct taaggctaca tgaactaacc aagatttatc caggcacctc cagcccagca 3240
   gtggacaggc tgtgtgtcgg agttcgccct ggagagtgct ttggcctcct gggagtgaat 3300
   ggtgccggca aaacaaccac attcaagatg ctcactgggg acaccacagt gacctcaggg 3360
   gatgccaccg tagcaggcaa gagtatttta accaatattt ctgaagtcca tcaaaatatg 3420
   ggctactgtc ctcagtttga tgcaatcgat gagctgctca caggacgaga acatctttac 3480
   ctttatgccc ggcttcgagg tgtaccagca gaagaaatcg aaaaggttgc aaactggagt 3540
   attaagagcc tgggcctgac tgtctacgcc gactgcctgg ctggcacgta cagtgggggc 3600
   aacaagcgga aactctccac agccatcgca ctcattggct gcccaccgct ggtgctgctg 3660
   gatgagccca ccacagggat ggacccccag gcacgccgca tgctgtggaa cgtcatcgtg 3720
   agcatcatca gagaagggag ggctgtggtc ctcacatccc acagcatgga agaatgtgag 3780
   gcactgtgta cccggctggc catcatggta aagggcgcct ttcgatgtat gggcaccatt 3840
   cagcatctca agtccaaatt tggagatggc tatatcgtca caatgaagat caaatccccg 3900
   aaggacgacc tgcttcctga cctgaaccct gtggagcagt tcttccaggg gaacttccca 3960
   ggcagtgtgc agagggagag gcactacaac atgctccagt tccaggtctc ctcctcctcc 4020
   ctggcgagga tcttccagct cctcctctcc cacaaggaca gcctgctcat cgaggagtac 4080
   tcagtcacac agaccacact ggaccaggtg tttgtaaatt ttgctaaaca gcagactgaa 4140
   agtcatgacc tccctctgca ccctcgagct gctggagcca gtcgacaagc ccaggacgac 4200
   tacaaagacc atgacggtga ttataaagat catgacatcg actacaagga tgacgatgac 4260
   aagtgagcgg ccgcttcgag cagacatgat aagatacatt gatgagtttg gacaaaccac 4320
   aactagaatg cagtgaaaaa aatgctttat ttgtgaaatt tgtgatgcta ttgctttatt 4380
   tgtaaccatt ataagctgca ataaacaagt taacaacaac aattgcattc attttatgtt     4440
   tcaggttcag ggggagatgt gggaggtttt ttaaagcaag taaaacctct acaaatgtgg     4500
   taaaatcgat aaggatcttc ctagagcatg gctacatctg cagaattcag gctagctcac     4560
   tgcttacaaa acccccttgc ttgagagtgt ggcactctcc cccctgtcgc gttcgctcgc     4620
   tcgctggctc gtttgggggg gcgacggcca gagggccgtc gtctggcagc tctttgagct     4680
   gccacccccc caaacgagcc agcgagcgag cgaacgcgac aggggggaga g              4731
<210> 69
   <211> 4420
   <212> DNA
   <213> Artificial Sequence
<220>
   <223> synthetic
<400> 69
   ctctcccccc tgtcgcgttc gctcgctcgc tggctcgttt gggggggtgg cagctcaaag      60
   agctgccaga cgacggccct ctggccgtcg cccccccaaa cgagccagcg agcgagcgaa     120
   cgcgacaggg gggagagtgc cacactctca agcaaggggg ttttgtaagc agtgagctag     180
   cgtgccacct ggtcgacatt gattattgac tagttattaa tagtaatcaa ttacggggtc     240
   attagttcat agcccatata tggagttccg cgttacataa cttacggtaa atggcccgcc     300
   tggctgaccg cccaacgacc cccgcccatt gacgtcaata atgacgtatg ttcccatagt     360
   aacgccaata gggactttcc attgacgtca atgggtggag tatttacggt aaactgccca     420
   cttggcagta catcaagtgt atcatatgcc aagtacgccc cctattgacg tcaatgacgg     480
   taaatggccc gcctggcatt atgcccagta catgacctta tgggactttc ctacttggca     540
   gtacatctac gtattagtca tcgctattac catggtcgag gtgagcccca cgttctgctt     600
   cactctcccc atctcccccc cctccccacc cccaattttg tatttattta ttttttaatt      660
   attttgtgca gcgatggggg cggggcgggg cgaggcggag aggtgcggcg gcagccaatc     720
   ggagcggcgc gctccgaaag tttcctttta tggcgaggcg gcggcggcgg cggctctata     780
   aaaagcgaag cgcgcggcgg gcggctgcag aagttggtcg tgaggcactg ggcaggtaag     840
   tatcaaggtt acaagacagg tttaaggaga ccaatagaaa ctgggcttgt cgagacagag     900
   aagactcttg cgtttctgat aggcacctat tggtcttact gacatccact ttgcctttct     960
   ctccacaggt gtccaggcgg ccgccatggt gattcttcag cagggggacc atgtgtggat    1020
   ggacctgaga ttggggcagg agttcgacgt gcccatcggg gcggtggtga agctctgcga    1080
   ctctgggcag gtccaggtgg tggatgatga agacaatgaa cactggatct ctccgcagaa    1140
   cgcaacgcac atcaagccta tgcaccccac gtcggtccac ggcgtggagg acatgatccg    1200
   cctgggggac ctcaacgagg cgggcatctt gcgcaacctg cttatccgct accgggacca    1260
   cctcatctac acgtatacgg gctccatcct ggtggctgtg aacccctacc agctgctctc 1320
   catctactcg ccagagcaca tccgccagta taccaacaag aagattgggg agatgccccc 1380
   ccacatcttt gccattgctg acaactgcta cttcaacatg aaacgcaaca gccgagacca 1440
   gtgctgcatc atcagtgggg aatctggggc cgggaagacg gagagcacaa agctgatcct 1500
   gcagttcctg gcagccatca gtgggcagca ctcgtggatt gagcagcagg tcttggaggc 1560
   cacccccatt ctggaagcat ttgggaatgc caagaccatc cgcaatgaca actcaagccg 1620
   tttcggaaag tacatcgaca tccacttcaa caagcggggc gccatcgagg gcgcgaagat 1680
   tgagcagtac ctgctggaaa agtcacgtgt ctgtcgccag gccctggatg aaaggaacta 1740
   ccacgtgttc tactgcatgc tggagggcat gagtgaggat cagaagaaga agctgggctt 1800
   gggccaggcc tctgactaca actacttggc catgggtaac tgcataacct gtgagggccg 1860
   ggtggacagc caggagtacg ccaacatccg ctccgccatg aaggtgctca tgttcactga 1920
   caccgagaac tgggagatct cgaagctcct ggctgccatc ctgcacctgg gcaacctgca 1980
   gtatgaggca cgcacatttg aaaacctgga tgcctgtgag gttctcttct ccccatcgct 2040
   ggccacagct gcatccctgc ttgaggtgaa ccccccagac ctgatgagct gcctgactag 2100
   ccgcaccctc atcacccgcg gggagacggt gtccacccca ctgagcaggg aacaggcact 2160
   ggacgtgcgc gacgccttcg taaaggggat ctacgggcgg ctgttcgtgt ggattgtgga 2220
   caagatcaac gcagcaattt acaagcctcc ctcccaggat gtgaagaact ctcgcaggtc 2280
   catcggcctc ctggacatct ttgggtttga gaactttgct gtgaacagct ttgagcagct 2340
   ctgcatcaac ttcgccaatg agcacctgca gcagttcttt gtgcggcacg tgttcaagct 2400
   ggagcaggag gaatatgacc tggagagcat tgactggctg cacatcgagt tcactgacaa 2460
   ccaggatgcc ctggacatga ttgccaacaa gcccatgaac atcatctccc tcatcgatga 2520
   ggagagcaag ttccccaagg gcacagacac caccatgtta cacaagctga actcccagca 2580
   caagctcaac gccaactaca tcccccccaa gaacaaccat gagacccagt ttggcatcaa 2640
   ccattttgca ggcatcgtct actatgagac ccaaggcttc ctggagaaga accgagacac 2700
   cctgcatggg gacattatcc agctggtcca ctcctccagg aacaagttca tcaagcagat 2760
   cttccaggcc gatgtcgcca tgggcgccga gaccaggaag cgctcgccca cacttagcag 2820
   ccagttcaag cggtcactgg agctgctgat gcgcacgctg ggtgcctgcc agcccttctt 2880
   tgtgcgatgc atcaagccca atgagttcaa gaagcccatg ctgttcgacc ggcacctgtg 2940
   cgtgcgccag ctgcggtact caggaatgat ggagaccatc cgaatccgcc gagctggcta 3000
   ccccatccgc tacagcttcg tagagtttgt ggagcggtac cgtgtgctgc tgccaggtgt 3060
   gaagccggcc tacaagcagg gcgacctccg cgggacttgc cagcgcatgg ctgaggctgt 3120
   gctgggcacc cacgatgact ggcagatagg caaaaccaag atctttctga aggaccacca 3180
   tgacatgctg ctggaagtgg agcgggacaa agccatcacc gacagagtca tcctccttca 3240
   gaaagtcatc cggggattca aagacaggtc taactttctg aagctgaaga acgctgccac 3300
   actgatccag aggcactggc ggggtcacaa ctgtaggaag aactacgggc tgatgcgtct 3360
   gggcttcctg cggctgcagg ccctgcaccg ctcccggaag ctgcaccagc agtaccgcct 3420
   ggcccgccag cgcatcatcc agttccaggc ccgctgccgc gcctatctgg tgcgcaaggc 3480
   cttccgccac cgcctctggg ctgtgctcac cgtgcaggcc tatgcccggg gcatgatcgc 3540
   ccgcaggctg caccaacgcc tcagggctga gtatctgtgg cgcctcgagg ctgagaaaat 3600
   gcggctggcg gaggaagaga agcttcggaa ggagatgagc gccaagaagg ccaaggagga 3660
   ggccgagcgc aagcatcagg agcgcctggc ccagctggct cgtgaggacg ctgagcggga 3720
   gctgaaggag aaggaggccg ctcggcggaa gaaggagctc ctggagcaga tggaaagggc 3780
   ccgccatgag cctgtcaatc actcagacat ggtggacaag atgtttggct tcctggggac 3840
   ttcaggtggc ctgccaggcc aggagggcca ggcacctagt ggctttgagg acctggagcg 3900
   agggcggagg gagatggtgg aggaggacct ggatgcagcc ctgcccctgc ctgacgagga 3960
   tgaggaggac ctctctgagt ataaatttgc caagttcgcg gccacctact tccaggggac 4020
   aactacgcac tcctacaccc ggcggccact caaacagcca ctgctctacc atgacgacga 4080
   gggtgaccag ctggtaagta tcaaggttac aagacaggtt taaggagacc aatagaaact 4140
   gggcttgtcg agacagagaa gactcttgcg tttctgggat ttttccgatt tcggcctatt 4200
   ggttaaaaaa tgagctgatt taacaaaaat ttaacgcgaa ttttaacaaa atattaacgt 4260
   ttataatttc aggtggcatc tttccaattg aggaacccct agtgatggag ttggccactc 4320
   cctctctgcg cgctcgctcg ctcactgagg ccgggcgacc aaaggtcgcc cgacgcccgg 4380
   gctttgcccg ggcggcctca gtgagcgagc gagcgcgcag 4420
<210> 70
   <211> 4367
   <212> DNA
   <213> Artificial Sequence
<220>
   <223> synthetic
<400> 70
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatccggga ttttgccgat ttcggcctat tggttaaaaa atgagctgat     180
   ttaacaaaaa tttaacgcga attttaacaa aatattaacg tttataattt caggtggcat     240
   ctttcgatag gcacctattg gtcttactga catccacttt gcctttctct ccacaggcag     300
   ccctggcggt ctggatcacc atcctccgct tcatggggga cctccctgag cccaagtacc      360
   acacagccat gagtgatggc agtgagaaga tccctgtgat gaccaagatt tatgagaccc      420
   tgggcaagaa gacgtacaag agggagctgc aggccctgca gggcgagggc gaggcccagc      480
   tccccgaggg ccagaagaag agcagtgtga ggcacaagct ggtgcatttg actctgaaaa      540
   agaagtccaa gctcacagag gaggtgacca agaggctgca tgacggggag tccacagtgc      600
   agggcaacag catgctggag gaccggccca cctccaacct ggagaagctg cacttcatca      660
   tcggcaatgg catcctgcgg ccagcactcc gggacgagat ctactgccag atcagcaagc      720
   agctgaccca caacccctcc aagagcagct atgcccgggg ctggattctc gtgtctctct      780
   gcgtgggctg tttcgccccc tccgagaagt ttgtcaagta cctgcggaac ttcatccacg      840
   ggggcccgcc cggctacgcc ccgtactgtg aggagcgcct gagaaggacc tttgtcaatg      900
   ggacacggac acagccgccc agctggctgg agctgcaggc caccaagtcc aagaagccaa      960
   tcatgttgcc cgtgacattc atggatggga ccaccaagac cctgctgacg gactcggcaa     1020
   ccacggccaa ggagctctgc aacgcgctgg ccgacaagat ctctctcaag gaccggttcg     1080
   ggttctccct ctacattgcc ctgtttgaca aggtgtcctc cctgggcagc ggcagtgacc     1140
   acgtcatgga cgccatctcc cagtgcgagc agtacgccaa ggagcagggc gcccaggagc     1200
   gcaacgcccc ctggaggctc ttcttccgca aagaggtctt cacgccctgg cacagcccct     1260
   ccgaggacaa cgtggccacc aacctcatct accagcaggt ggtgcgagga gtcaagtttg     1320
   gggagtacag gtgtgagaag gaggacgacc tggctgagct ggcctcccag cagtactttg     1380
   tagactatgg ctctgagatg atcctggagc gcctcctgaa cctcgtgccc acctacatcc     1440
   ccgaccgcga gatcacgccc ctgaagacgc tggagaagtg ggcccagctg gccatcgccg     1500
   cccacaagaa ggggatttat gcccagagga gaactgatgc ccagaaggtc aaagaggatg     1560
   tggtcagtta tgcccgcttc aagtggccct tgctcttctc caggttttat gaagcctaca     1620
   aattctcagg ccccagtctc cccaagaacg acgtcatcgt ggccgtcaac tggacgggtg     1680
   tgtactttgt ggatgagcag gagcaggtac ttctggagct gtccttccca gagatcatgg     1740
   ccgtgtccag cagcagggag tgccgtgtct ggctctcact gggctgctct gatcttggct     1800
   gtgctgcgcc tcactcaggc tgggcaggac tgaccccggc ggggccctgt tctccgtgtt     1860
   ggtcctgcag gggagcgaaa acgacggccc ccagcttcac gctggccacc atcaaggggg     1920
   acgaatacac cttcacctcc agtaatgctg aggacattcg tgacctggtg gtcaccttcc     1980
   tagaggggct ccggaagaga tctaagtatg ttgtggccct gcaggataac cccaaccccg     2040
   caggcgagga gtcaggcttc ctcagctttg ccaagggaga cctcatcatc ctggaccatg     2100
   acacgggcga gcaggtcatg aactcgggct gggccaacgg catcaatgag aggaccaagc     2160
   agcgtgggga cttccccacc gactgtgtgt acgtcatgcc cactgtcacc atgccacctc 2220
   gtgagattgt ggccctggtc accatgactc ccgatcagag gcaggacgtt gtccggctct 2280
   tgcagctgcg aacggcggag cccgaggtgc gtgccaagcc ctacacgctg gaggagtttt 2340
   cctatgacta cttcaggccc ccacccaagc acacgctgag ccgtgtcatg gtgtccaagg 2400
   cccgaggcaa ggaccggctg tggagccaca cgcgggaacc gctcaagcag gcgctgctca 2460
   agaagctcct gggcagtgag gagctctcgc aggaggcctg cctggccttc attgctgtgc 2520
   tcaagtacat gggcgactac ccgtccaaga ggacacgctc cgtcaatgag ctcaccgacc 2580
   agatctttga gggtcccctg aaagccgagc ccctgaagga cgaggcatat gtgcagatcc 2640
   tgaagcagct gaccgacaac cacatcaggt acagcgagga gcggggttgg gagctgctct 2700
   ggctgtgcac gggccttttc ccacccagca acatcctcct gccccacgtg cagcgcttcc 2760
   tgcagtcccg aaagcactgc ccactcgcca tcgactgcct gcaacggctc cagaaagccc 2820
   tgagaaacgg gtcccggaag taccctccgc acctggtgga ggtggaggcc atccagcaca 2880
   agaccaccca gattttccac aaggtctact tccctgatga cactgacgag gccttcgaag 2940
   tggagtccag caccaaggcc aaggacttct gccagaacat cgccaccagg ctgctcctca 3000
   agtcctcaga gggattcagc ctctttgtca aaattgcaga caaggtcatc agcgttcctg 3060
   agaatgactt cttctttgac tttgttcgac acttgacaga ctggataaag aaagctcggc 3120
   ccatcaagga cggaattgtg ccctcactca cctaccaggt gttcttcatg aagaagctgt 3180
   ggaccaccac ggtgccaggg aaggatccca tggccgattc catcttccac tattaccagg 3240
   agttgcccaa gtatctccga ggctaccaca agtgcacgcg ggaggaggtg ctgcagctgg 3300
   gggcgctgat ctacagggtc aagttcgagg aggacaagtc ctacttcccc agcatcccca 3360
   agctgctgcg ggagctggtg ccccaggacc ttatccggca ggtctcacct gatgactgga 3420
   agcggtccat cgtcgcctac ttcaacaagc acgcagggaa gtccaaggag gaggccaagc 3480
   tggccttcct gaagctcatc ttcaagtggc ccacctttgg ctcagccttc ttcgaggtga 3540
   agcaaactac ggagccaaac ttccctgaga tcctcctaat tgccatcaac aagtatgggg 3600
   tcagcctcat cgatcccaaa acgaaggata tcctcaccac tcatcccttc accaagatct 3660
   ccaactggag cagcggcaac acctacttcc acatcaccat tgggaacttg gtgcgcggga 3720
   gcaaactgct ctgcgagacg tcactgggct acaagatgga tgacctcctg acttcctaca 3780
   ttagccagat gctcacagcc atgagcaaac agcggggctc caggagcggc aagatgtatg 3840
   atgttcctga ttatgctagc ctctgaccgc ggcctgctgc cggctctgcg gcctcttccg 3900
   cgtcttcgag atctgcctcg actgtgcctt ctagttgcca gccatctgtt gtttgcccct 3960
   cccccgtgcc ttccttgacc ctggaaggtg ccactcccac tgtcctttcc taataaaatg 4020
   aggaaattgc atcgcattgt ctgagtaggt gtcattctat tctggggggt ggggtggggc 4080
   aggacagcaa gggggaggat tgggaagaca atagcaggca tgctggggac tcgagcaatt     4140
   cccgataagg atcttcctag agcatggcta catctgcaga attcaggcta gctcactgct     4200
   tacaaaaccc ccttgcttga gagtgtggca ctctcccccc tgtcgcgttc gctcgctcgc     4260
   tggctcgttt gggggggcga cggccagagg gccgtcgtct ggcagctctt tgagctgcca     4320
   cccccccaaa cgagccagcg agcgagcgaa cgcgacaggg gggagag                   4367
<210> 71
   <211> 4738
   <212> DNA
   <213> Artificial Sequence
<220>
   <223> synthetic
<400> 71
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct tgtagttaat gattaacccg ccatgctact tatctacgta gccatgctct     180
   aggaagatct tcaatattgg ccattagcca tattattcat tggttatata gcataaatca     240
   atattggcta ttggccattg catacgttgt atctatatca taatatgtac atttatattg     300
   gctcatgtcc aatatgaccg ccatgttggc attgattatt gactagttat taatagtaat     360
   caattacggg gtcattagtt catagcccat atatggagtt ccgcgttaca taacttacgg     420
   taaatggccc gcctggctga ccgcccaacg acccccgccc attgacgtca ataatgacgt     480
   atgttcccat agtaacgcca atagggactt tccattgacg tcaatgggtg gagtatttac     540
   ggtaaactgc ccacttggca gtacatcaag tgtatcatat gccaagtccg ccccctattg     600
   acgtcaatga cggtaaatgg cccgcctggc attatgccca gtacatgacc ttacgggact     660
   ttcctacttg gcagtacatc tacgtattag tcatcgctat taccatggtg atgcggtttt     720
   ggcagtacac caatgggcgt ggatagcggt ttgactcacg gggatttcca agtctccacc     780
   ccattgacgt caatgggagt ttgttttggc accaaaatca acgggacttt ccaaaatgtc     840
   gtaataaccc cgccccgttg acgcaaatgg gcggtaggcg tgtacggtgg gaggtctata     900
   taagcagagc tcgtttagtg aaccgtcaga tcactagaag ctttattgcg gtagtttatc     960
   acagttaaat tgctaacgca gtcagtgctt ctgacacaac agtctcgaac ttaagctgca    1020
   gaagttggtc gtgaggcact gggcaggtaa gtatcaaggt tacaagacag gtttaaggag    1080
   accaatagaa actgggcttg tcgagacaga gaagactctt gcgtttctga taggcaccta    1140
   ttggtcttac tgacatccac tttgcctttc tctccacagg tgtccactcc cagttcaatt    1200
   acagctctta aggctagagt acttaatacg actcactata ggctagcctc gagaattcac    1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggactacaa agaccatgac ggtgattata aagatcatga catcgactac aaggatgacg 3120
   atgacaagga ttctggtccc agagctgatc ccgtggaaga tttccggtac atctggggcg 3180
   ggtttgccta tctgcaggac atggttgaac aggggatcac aaggagccag gtgcaggcgg 3240
   aggctccagt tggaatctac ctccagcaga tgccctaccc ctgcttcgtg gacgattctt 3300
   tcatgatcat cctgaaccgc tgtttcccta tcttcatggt gctggcatgg atctactctg 3360
   tctccatgac tgtgaagagc atcgtcttgg agaaggagtt gcgactgaag gagaccttga 3420
   aaaatcaggg tgtctccaat gcagtgattt ggtgtacctg gttcctggac agcttctcca 3480
   tcatgtcgat gagcatcttc ctcctgacga tattcatcat gcatggaaga atcctacatt 3540
   acagcgaccc attcatcctc ttcctgttct tgttggcttt ctccactgcc accatcatgc 3600
   tgtgctttct gctcagcacc ttcttctcca aggccagtct ggcagcagcc tgtagtggtg 3660
   tcatctattt caccctctac ctgccacaca tcctgtgctt cgcctggcag gaccgcatga 3720
   ccgctgagct gaagaaggct gtgagcttac tgtctccggt ggcatttgga tttggcactg 3780
   agtacctggt tcgctttgaa gagcaaggcc tggggctgca gtggagcaac atcgggaaca 3840
   gtcccacgga aggggacgaa ttcagcttcc tgctgtccat gcagatgatg ctccttgatg 3900
   ctgctgtcta tggcttactc gcttggtacc ttgatcaggt gtttccagga gactatggaa 3960
   ccccacttcc ttggtacttt cttctacaag agtcgtattg gcttggcggt gaagggtgtt 4020
   caaccagaga agaaagagcc ctggaaaaga ccgagcccct aacagaggaa acggaggatc 4080
   cagagcaccc agaaggaata cacgactcct tctttgaacg tgagcatcca gggtgggttc 4140
   ctggggtatg cgtgaagaat ctggtaaaga tttttgagcc ctgtggccgg ccagctgtgg 4200
   accgtctgaa catcaccttc tacgagaacc agatcaccgc attcctgggc cacaatggag 4260
   ctgggaaaac caccaccttg taagtatcaa ggttacaaga caggtttaag gagaccaata 4320
   gaaactgggc ttgtcgagac agagaagact cttgcgtttc tgggattttt ccgatttcgg 4380
   cctattggtt aaaaaatgag ctgatttaac aaaaatttaa cgcgaatttt aacaaaatat 4440
   taacgtttat aatttcaggt ggcatctttc caattcgccc ttagatctag cctatcctgg 4500
   attacttgaa cgatagccta tcctggatta cttgaaaagc ttagcctatc ctggattact 4560
   tgaatcacag cctatcctgg attacttgaa agatctaagg gcgaattgag gaacccctag 4620
   tgatggagtt ggccactccc tctctgcgcg ctcgctcgct cactgaggcc gggcgaccaa 4680
   aggtcgcccg acgcccgggc tttgcccggg cggcctcagt gagcgagcga gcgcgcag 4738
<210> 72
   <211> 4770
   <212> DNA
   <213> Artificial Sequence
<220>
   <223> synthetic
<400> 72
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct tgtagttaat gattaacccg ccatgctact tatctacgta gccatgctct 180
   aggaagatct tcaatattgg ccattagcca tattattcat tggttatata gcataaatca 240
   atattggcta ttggccattg catacgttgt atctatatca taatatgtac atttatattg 300
   gctcatgtcc aatatgaccg ccatgttggc attgattatt gactagttat taatagtaat 360
   caattacggg gtcattagtt catagcccat atatggagtt ccgcgttaca taacttacgg 420
   taaatggccc gcctggctga ccgcccaacg acccccgccc attgacgtca ataatgacgt 480
   atgttcccat agtaacgcca atagggactt tccattgacg tcaatgggtg gagtatttac 540
   ggtaaactgc ccacttggca gtacatcaag tgtatcatat gccaagtccg ccccctattg 600
   acgtcaatga cggtaaatgg cccgcctggc attatgccca gtacatgacc ttacgggact 660
   ttcctacttg gcagtacatc tacgtattag tcatcgctat taccatggtg atgcggtttt 720
   ggcagtacac caatgggcgt ggatagcggt ttgactcacg gggatttcca agtctccacc 780
   ccattgacgt caatgggagt ttgttttggc accaaaatca acgggacttt ccaaaatgtc 840
   gtaataaccc cgccccgttg acgcaaatgg gcggtaggcg tgtacggtgg gaggtctata 900
   taagcagagc tcgtttagtg aaccgtcaga tcactagaag ctttattgcg gtagtttatc 960
   acagttaaat tgctaacgca gtcagtgctt ctgacacaac agtctcgaac ttaagctgca 1020
   gaagttggtc gtgaggcact gggcaggtaa gtatcaaggt tacaagacag gtttaaggag 1080
   accaatagaa actgggcttg tcgagacaga gaagactctt gcgtttctga taggcaccta 1140
   ttggtcttac tgacatccac tttgcctttc tctccacagg tgtccactcc cagttcaatt 1200
   acagctctta aggctagagt acttaatacg actcactata ggctagcctc gagaattcac 1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggactacaa agaccatgac ggtgattata aagatcatga catcgactac aaggatgacg 3120
   atgacaagga ttctggtccc agagctgatc ccgtggaaga tttccggtac atctggggcg 3180
   ggtttgccta tctgcaggac atggttgaac aggggatcac aaggagccag gtgcaggcgg 3240
   aggctccagt tggaatctac ctccagcaga tgccctaccc ctgcttcgtg gacgattctt 3300
   tcatgatcat cctgaaccgc tgtttcccta tcttcatggt gctggcatgg atctactctg 3360
   tctccatgac tgtgaagagc atcgtcttgg agaaggagtt gcgactgaag gagaccttga 3420
   aaaatcaggg tgtctccaat gcagtgattt ggtgtacctg gttcctggac agcttctcca 3480
   tcatgtcgat gagcatcttc ctcctgacga tattcatcat gcatggaaga atcctacatt 3540
   acagcgaccc attcatcctc ttcctgttct tgttggcttt ctccactgcc accatcatgc 3600
   tgtgctttct gctcagcacc ttcttctcca aggccagtct ggcagcagcc tgtagtggtg 3660
   tcatctattt caccctctac ctgccacaca tcctgtgctt cgcctggcag gaccgcatga 3720
   ccgctgagct gaagaaggct gtgagcttac tgtctccggt ggcatttgga tttggcactg 3780
   agtacctggt tcgctttgaa gagcaaggcc tggggctgca gtggagcaac atcgggaaca      3840
   gtcccacgga aggggacgaa ttcagcttcc tgctgtccat gcagatgatg ctccttgatg      3900
   ctgctgtcta tggcttactc gcttggtacc ttgatcaggt gtttccagga gactatggaa      3960
   ccccacttcc ttggtacttt cttctacaag agtcgtattg gcttggcggt gaagggtgtt      4020
   caaccagaga agaaagagcc ctggaaaaga ccgagcccct aacagaggaa acggaggatc      4080
   cagagcaccc agaaggaata cacgactcct tctttgaacg tgagcatcca gggtgggttc      4140
   ctggggtatg cgtgaagaat ctggtaaaga tttttgagcc ctgtggccgg ccagctgtgg      4200
   accgtctgaa catcaccttc tacgagaacc agatcaccgc attcctgggc cacaatggag      4260
   ctgggaaaac caccaccttg taagtatcaa ggttacaaga caggtttaag gagaccaata      4320
   gaaactgggc ttgtcgagac agagaagact cttgcgtttc tgggattttt ccgatttcgg      4380
   cctattggtt aaaaaatgag ctgatttaac aaaaatttaa cgcgaatttt aacaaaatat      4440
   taacgtttat aatttcaggt ggcatctttc caattgaggc ataggatgac aaagggaacg      4500
   ataggcatag gatgacaaag ggaaaagctt aggcatagga tgacaaaggg aaggtaccag      4560
   atctggcatt caccgcgtgc cttacgatgg cattcaccgc gtgccttaaa gcttggcatt      4620
   caccgcgtgc cttacaattg aggaacccct agtgatggag ttggccactc cctctctgcg      4680
   cgctcgctcg ctcactgagg ccgggcgacc aaaggtcgcc cgacgcccgg gctttgcccg      4740
   ggcggcctca gtgagcgagc gagcgcgcag                                       4770
<210> 73
   <211> 4656
   <212> DNA
   <213> Artificial Sequence
<220>
   <223> synthetic
<400> 73
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct tgtagttaat gattaacccg ccatgctact tatctacgta gccatgctct     180
   aggaagatct tcaatattgg ccattagcca tattattcat tggttatata gcataaatca     240
   atattggcta ttggccattg catacgttgt atctatatca taatatgtac atttatattg     300
   gctcatgtcc aatatgaccg ccatgttggc attgattatt gactagttat taatagtaat     360
   caattacggg gtcattagtt catagcccat atatggagtt ccgcgttaca taacttacgg     420
   taaatggccc gcctggctga ccgcccaacg acccccgccc attgacgtca ataatgacgt     480
   atgttcccat agtaacgcca atagggactt tccattgacg tcaatgggtg gagtatttac     540
   ggtaaactgc ccacttggca gtacatcaag tgtatcatat gccaagtccg ccccctattg     600
   acgtcaatga cggtaaatgg cccgcctggc attatgccca gtacatgacc ttacgggact 660
   ttcctacttg gcagtacatc tacgtattag tcatcgctat taccatggtg atgcggtttt 720
   ggcagtacac caatgggcgt ggatagcggt ttgactcacg gggatttcca agtctccacc 780
   ccattgacgt caatgggagt ttgttttggc accaaaatca acgggacttt ccaaaatgtc 840
   gtaataaccc cgccccgttg acgcaaatgg gcggtaggcg tgtacggtgg gaggtctata 900
   taagcagagc tcgtttagtg aaccgtcaga tcactagaag ctttattgcg gtagtttatc 960
   acagttaaat tgctaacgca gtcagtgctt ctgacacaac agtctcgaac ttaagctgca 1020
   gaagttggtc gtgaggcact gggcaggtaa gtatcaaggt tacaagacag gtttaaggag 1080
   accaatagaa actgggcttg tcgagacaga gaagactctt gcgtttctga taggcaccta 1140
   ttggtcttac tgacatccac tttgcctttc tctccacagg tgtccactcc cagttcaatt 1200
   acagctctta aggctagagt acttaatacg actcactata ggctagcctc gagaattcac 1260
   gcgtggtacc tctagagtcg acccgggcgg ccgccatggg cttcgtgaga cagatacagc 1320
   ttttgctctg gaagaactgg accctgcgga aaaggcaaaa gattcgcttt gtggtggaac 1380
   tcgtgtggcc tttatcttta tttctggtct tgatctggtt aaggaatgcc aacccgctct 1440
   acagccatca tgaatgccat ttccccaaca aggcgatgcc ctcagcagga atgctgccgt 1500
   ggctccaggg gatcttctgc aatgtgaaca atccctgttt tcaaagcccc accccaggag 1560
   aatctcctgg aattgtgtca aactataaca actccatctt ggcaagggta tatcgagatt 1620
   ttcaagaact cctcatgaat gcaccagaga gccagcacct tggccgtatt tggacagagc 1680
   tacacatctt gtcccaattc atggacaccc tccggactca cccggagaga attgcaggaa 1740
   gaggaattcg aataagggat atcttgaaag atgaagaaac actgacacta tttctcatta 1800
   aaaacatcgg cctgtctgac tcagtggtct accttctgat caactctcaa gtccgtccag 1860
   agcagttcgc tcatggagtc ccggacctgg cgctgaagga catcgcctgc agcgaggccc 1920
   tcctggagcg cttcatcatc ttcagccaga gacgcggggc aaagacggtg cgctatgccc 1980
   tgtgctccct ctcccagggc accctacagt ggatagaaga cactctgtat gccaacgtgg 2040
   acttcttcaa gctcttccgt gtgcttccca cactcctaga cagccgttct caaggtatca 2100
   atctgagatc ttggggagga atattatctg atatgtcacc aagaattcaa gagtttatcc 2160
   atcggccgag tatgcaggac ttgctgtggg tgaccaggcc cctcatgcag aatggtggtc 2220
   cagagacctt tacaaagctg atgggcatcc tgtctgacct cctgtgtggc taccccgagg 2280
   gaggtggctc tcgggtgctc tccttcaact ggtatgaaga caataactat aaggcctttc 2340
   tggggattga ctccacaagg aaggatccta tctattctta tgacagaaga acaacatcct 2400
   tttgtaatgc attgatccag agcctggagt caaatccttt aaccaaaatc gcttggaggg 2460
   cggcaaagcc tttgctgatg ggaaaaatcc tgtacactcc tgattcacct gcagcacgaa 2520
   ggatactgaa gaatgccaac tcaacttttg aagaactgga acacgttagg aagttggtca 2580
   aagcctggga agaagtaggg ccccagatct ggtacttctt tgacaacagc acacagatga 2640
   acatgatcag agataccctg gggaacccaa cagtaaaaga ctttttgaat aggcagcttg 2700
   gtgaagaagg tattactgct gaagccatcc taaacttcct ctacaagggc cctcgggaaa 2760
   gccaggctga cgacatggcc aacttcgact ggagggacat atttaacatc actgatcgca 2820
   ccctccgcct tgtcaatcaa tacctggagt gcttggtcct ggataagttt gaaagctaca 2880
   atgatgaaac tcagctcacc caacgtgccc tctctctact ggaggaaaac atgttctggg 2940
   ccggagtggt attccctgac atgtatccct ggaccagctc tctaccaccc cacgtgaagt 3000
   ataagatccg aatggacata gacgtggtgg agaaaaccaa taagattaaa gacaggtatt 3060
   gggactacaa agaccatgac ggtgattata aagatcatga catcgactac aaggatgacg 3120
   atgacaagga ttctggtccc agagctgatc ccgtggaaga tttccggtac atctggggcg 3180
   ggtttgccta tctgcaggac atggttgaac aggggatcac aaggagccag gtgcaggcgg 3240
   aggctccagt tggaatctac ctccagcaga tgccctaccc ctgcttcgtg gacgattctt 3300
   tcatgatcat cctgaaccgc tgtttcccta tcttcatggt gctggcatgg atctactctg 3360
   tctccatgac tgtgaagagc atcgtcttgg agaaggagtt gcgactgaag gagaccttga 3420
   aaaatcaggg tgtctccaat gcagtgattt ggtgtacctg gttcctggac agcttctcca 3480
   tcatgtcgat gagcatcttc ctcctgacga tattcatcat gcatggaaga atcctacatt 3540
   acagcgaccc attcatcctc ttcctgttct tgttggcttt ctccactgcc accatcatgc 3600
   tgtgctttct gctcagcacc ttcttctcca aggccagtct ggcagcagcc tgtagtggtg 3660
   tcatctattt caccctctac ctgccacaca tcctgtgctt cgcctggcag gaccgcatga 3720
   ccgctgagct gaagaaggct gtgagcttac tgtctccggt ggcatttgga tttggcactg 3780
   agtacctggt tcgctttgaa gagcaaggcc tggggctgca gtggagcaac atcgggaaca 3840
   gtcccacgga aggggacgaa ttcagcttcc tgctgtccat gcagatgatg ctccttgatg 3900
   ctgctgtcta tggcttactc gcttggtacc ttgatcaggt gtttccagga gactatggaa 3960
   ccccacttcc ttggtacttt cttctacaag agtcgtattg gcttggcggt gaagggtgtt 4020
   caaccagaga agaaagagcc ctggaaaaga ccgagcccct aacagaggaa acggaggatc 4080
   cagagcaccc agaaggaata cacgactcct tctttgaacg tgagcatcca gggtgggttc 4140
   ctggggtatg cgtgaagaat ctggtaaaga tttttgagcc ctgtggccgg ccagctgtgg 4200
   accgtctgaa catcaccttc tacgagaacc agatcaccgc attcctgggc cacaatggag 4260
   ctgggaaaac caccaccttg taagtatcaa ggttacaaga caggtttaag gagaccaata 4320
   gaaactgggc ttgtcgagac agagaagact cttgcgtttc tgggattttt ccgatttcgg 4380
   cctattggtt aaaaaatgag ctgatttaac aaaaatttaa cgcgaatttt aacaaaatat 4440
   taacgtttat aatttcaggt ggcatctttc ccgcctgcaa gaactggttc agcagcctga 4500
   gccacttcgt gatccacctg caattgagga acccctagtg atggagttgg ccactccctc 4560
   tctgcgcgct cgctcgctca ctgaggccgg gcgaccaaag gtcgcccgac gcccgggctt 4620
   tgcccgggcg gcctcagtga gcgagcgagc gcgcag 4656
<210> 74
   <211> 4719
   <212> DNA
   <213> Artificial Sequence
<220>
   <223> synthetic
<400> 74
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct ggatccggga tttttccgat ttcggcctat tggttaaaaa atgagctgat 180
   ttaacaaaaa tttaacgcga attttaacaa aatattaacg tttataattt caggtggcat 240
   ctttcaagct ttgaatgaat gagataggca cctattggtc ttactgacat ccactttgcc 300
   tttctctcca caggtccatc ctgacgggtc tgttgccacc aacctctggg actgtgctcg 360
   ttgggggaag ggacattgaa accagcctgg atgcagtccg gcagagcctt ggcatgtgtc 420
   cacagcacaa catcctgttc caccacctca cggtggctga gcacatgctg ttctatgccc 480
   agctgaaagg aaagtcccag gaggaggccc agctggagat ggaagccatg ttggaggaca 540
   caggcctcca ccacaagcgg aatgaagagg ctcaggacct atcaggtggc atgcagagaa 600
   agctgtcggt tgccattgcc tttgtgggag atgccaaggt ggtgattctg gacgaaccca 660
   cctctggggt ggacccttac tcgagacgct caatctggga tctgctcctg aagtatcgct 720
   caggcagaac catcatcatg tccactcacc acatggacga ggccgacctc cttggggacc 780
   gcattgccat cattgcccag ggaaggctct actgctcagg caccccactc ttcctgaaga 840
   actgctttgg cacaggcttg tacttaacct tggtgcgcaa gatgaaaaac atccagagcc 900
   aaaggaaagg cagtgagggg acctgcagct gctcgtctaa gggtttctcc accacgtgtc 960
   cagcccacgt cgatgaccta actccagaac aagtcctgga tggggatgta aatgagctga 1020
   tggatgtagt tctccaccat gttccagagg caaagctggt ggagtgcatt ggtcaagaac 1080
   ttatcttcct tcttccaaat aagaacttca agcacagagc atatgccagc cttttcagag 1140
   agctggagga gacgctggct gaccttggtc tcagcagttt tggaatttct gacactcccc 1200
   tggaagagat ttttctgaag gtcacggagg attctgattc aggacctctg tttgcgggtg 1260
   gcgctcagca gaaaagagaa aacgtcaacc cccgacaccc ctgcttgggt cccagagaga 1320
   aggctggaca gacaccccag gactccaatg tctgctcccc aggggcgccg gctgctcacc 1380
   cagagggcca gcctccccca gagccagagt gcccaggccc gcagctcaac acggggacac 1440
   agctggtcct ccagcatgtg caggcgctgc tggtcaagag attccaacac accatccgca 1500
   gccacaagga cttcctggcg cagatcgtgc tcccggctac ctttgtgttt ttggctctga 1560
   tgctttctat tgttatccct ccttttggcg aataccccgc tttgaccctt cacccctgga 1620
   tatatgggca gcagtacacc ttcttcagca tggatgaacc aggcagtgag cagttcacgg 1680
   tacttgcaga cgtcctcctg aataagccag gctttggcaa ccgctgcctg aaggaagggt 1740
   ggcttccgga gtacccctgt ggcaactcaa caccctggaa gactccttct gtgtccccaa 1800
   acatcaccca gctgttccag aagcagaaat ggacacaggt caacccttca ccatcctgca 1860
   ggtgcagcac cagggagaag ctcaccatgc tgccagagtg ccccgagggt gccgggggcc 1920
   tcccgccccc ccagagaaca cagcgcagca cggaaattct acaagacctg acggacagga 1980
   acatctccga cttcttggta aaaacgtatc ctgctcttat aagaagcagc ttaaagagca 2040
   aattctgggt caatgaacag aggtatggag gaatttccat tggaggaaag ctcccagtcg 2100
   tccccatcac gggggaagca cttgttgggt ttttaagcga ccttggccgg atcatgaatg 2160
   tgagcggggg ccctatcact agagaggcct ctaaagaaat acctgatttc cttaaacatc 2220
   tagaaactga agacaacatt aaggtgtggt ttaataacaa aggctggcat gccctggtca 2280
   gctttctcaa tgtggcccac aacgccatct tacgggccag cctgcctaag gacagaagcc 2340
   ccgaggagta tggaatcacc gtcattagcc aacccctgaa cctgaccaag gagcagctct 2400
   cagagattac agtgctgacc acttcagtgg atgctgtggt tgccatctgc gtgattttct 2460
   ccatgtcctt cgtcccagcc agctttgtcc tttatttgat ccaggagcgg gtgaacaaat 2520
   ccaagcacct ccagtttatc agtggagtga gccccaccac ctactgggta accaacttcc 2580
   tctgggacat catgaattat tccgtgagtg ctgggctggt ggtgggcatc ttcatcgggt 2640
   ttcagaagaa agcctacact tctccagaaa accttcctgc ccttgtggca ctgctcctgc 2700
   tgtatggatg ggcggtcatt cccatgatgt acccagcatc cttcctgttt gatgtcccca 2760
   gcacagccta tgtggcttta tcttgtgcta atctgttcat cggcatcaac agcagtgcta 2820
   ttaccttcat cttggaatta tttgagaata accggacgct gctcaggttc aacgccgtgc 2880
   tgaggaagct gctcattgtc ttcccccact tctgcctggg ccggggcctc attgaccttg 2940
   cactgagcca ggctgtgaca gatgtctatg cccggtttgg tgaggagcac tctgcaaatc 3000
   cgttccactg ggacctgatt gggaagaacc tgtttgccat ggtggtggaa ggggtggtgt 3060
   acttcctcct gaccctgctg gtccagcgcc acttcttcct ctcccaatgg attgccgagc 3120
   ccactaagga gcccattgtt gatgaagatg atgatgtggc tgaagaaaga caaagaatta 3180
   ttactggtgg aaataaaact gacatcttaa ggctacatga actaaccaag atttatccag 3240
   gcacctccag cccagcagtg gacaggctgt gtgtcggagt tcgccctgga gagtgctttg 3300
   gcctcctggg agtgaatggt gccggcaaaa caaccacatt caagatgctc actggggaca 3360
   ccacagtgac ctcaggggat gccaccgtag caggcaagag tattttaacc aatatttctg 3420
   aagtccatca aaatatgggc tactgtcctc agtttgatgc aatcgatgag ctgctcacag 3480
   gacgagaaca tctttacctt tatgcccggc ttcgaggtgt accagcagaa gaaatcgaaa 3540
   aggttgcaaa ctggagtatt aagagcctgg gcctgactgt ctacgccgac tgcctggctg 3600
   gcacgtacag tgggggcaac aagcggaaac tctccacagc catcgcactc attggctgcc 3660
   caccgctggt gctgctggat gagcccacca cagggatgga cccccaggca cgccgcatgc 3720
   tgtggaacgt catcgtgagc atcatcagag aagggagggc tgtggtcctc acatcccaca 3780
   gcatggaaga atgtgaggca ctgtgtaccc ggctggccat catggtaaag ggcgcctttc 3840
   gatgtatggg caccattcag catctcaagt ccaaatttgg agatggctat atcgtcacaa 3900
   tgaagatcaa atccccgaag gacgacctgc ttcctgacct gaaccctgtg gagcagttct 3960
   tccaggggaa cttcccaggc agtgtgcaga gggagaggca ctacaacatg ctccagttcc 4020
   aggtctcctc ctcctccctg gcgaggatct tccagctcct cctctcccac aaggacagcc 4080
   tgctcatcga ggagtactca gtcacacaga ccacactgga ccaggtgttt gtaaattttg 4140
   ctaaacagca gactgaaagt catgacctcc ctctgcaccc tcgagctgct ggagccagtc 4200
   gacaagccca ggacgactac aaagaccatg acggtgatta taaagatcat gacatcgact 4260
   acaaggatga cgatgacaag tgagcggccg cttcgagcag acatgataag atacattgat 4320
   gagtttggac aaaccacaac tagaatgcag tgaaaaaaat gctttatttg tgaaatttgt 4380
   gatgctattg ctttatttgt aaccattata agctgcaata aacaagttaa caacaacaat 4440
   tgcattcatt ttatgtttca ggttcagggg gagatgtggg aggtttttta aagcaagtaa 4500
   aacctctaca aatgtggtaa aatcgataag gatcttccta gagcatggct acgtagataa 4560
   gtagcatggc gggttaatca ttaactacaa ggaaccccta gtgatggagt tggccactcc 4620
   ctctctgcgc gctcgctcgc tcactgaggc cgggcgacca aaggtcgccc gacgcccggg 4680
   ctttgcccgg gcggcctcag tgagcgagcg agcgcgcag 4719
<210> 75
   <211> 4758
   <212> DNA
   <213> Artificial Sequence
<220>
   <223> synthetic
<400> 75
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct ggatccggga tttttccgat ttcggcctat tggttaaaaa atgagctgat 180
   ttaacaaaaa tttaacgcga attttaacaa aatattaacg tttataattt caggtggcat 240
   ctttcaagct tatgcacagc tggaacttca agctgtacgt catgggcagc ggcggggtac 300
   cgataggcac ctattggtct tactgacatc cactttgcct ttctctccac aggtccatcc 360
   tgacgggtct gttgccacca acctctggga ctgtgctcgt tgggggaagg gacattgaaa 420
   ccagcctgga tgcagtccgg cagagccttg gcatgtgtcc acagcacaac atcctgttcc 480
   accacctcac ggtggctgag cacatgctgt tctatgccca gctgaaagga aagtcccagg 540
   aggaggccca gctggagatg gaagccatgt tggaggacac aggcctccac cacaagcgga 600
   atgaagaggc tcaggaccta tcaggtggca tgcagagaaa gctgtcggtt gccattgcct 660
   ttgtgggaga tgccaaggtg gtgattctgg acgaacccac ctctggggtg gacccttact 720
   cgagacgctc aatctgggat ctgctcctga agtatcgctc aggcagaacc atcatcatgt 780
   ccactcacca catggacgag gccgacctcc ttggggaccg cattgccatc attgcccagg 840
   gaaggctcta ctgctcaggc accccactct tcctgaagaa ctgctttggc acaggcttgt 900
   acttaacctt ggtgcgcaag atgaaaaaca tccagagcca aaggaaaggc agtgagggga 960
   cctgcagctg ctcgtctaag ggtttctcca ccacgtgtcc agcccacgtc gatgacctaa 1020
   ctccagaaca agtcctggat ggggatgtaa atgagctgat ggatgtagtt ctccaccatg 1080
   ttccagaggc aaagctggtg gagtgcattg gtcaagaact tatcttcctt cttccaaata 1140
   agaacttcaa gcacagagca tatgccagcc ttttcagaga gctggaggag acgctggctg 1200
   accttggtct cagcagtttt ggaatttctg acactcccct ggaagagatt tttctgaagg 1260
   tcacggagga ttctgattca ggacctctgt ttgcgggtgg cgctcagcag aaaagagaaa 1320
   acgtcaaccc ccgacacccc tgcttgggtc ccagagagaa ggctggacag acaccccagg 1380
   actccaatgt ctgctcccca ggggcgccgg ctgctcaccc agagggccag cctcccccag 1440
   agccagagtg cccaggcccg cagctcaaca cggggacaca gctggtcctc cagcatgtgc 1500
   aggcgctgct ggtcaagaga ttccaacaca ccatccgcag ccacaaggac ttcctggcgc 1560
   agatcgtgct cccggctacc tttgtgtttt tggctctgat gctttctatt gttatccctc 1620
   cttttggcga ataccccgct ttgacccttc acccctggat atatgggcag cagtacacct 1680
   tcttcagcat ggatgaacca ggcagtgagc agttcacggt acttgcagac gtcctcctga 1740
   ataagccagg ctttggcaac cgctgcctga aggaagggtg gcttccggag tacccctgtg 1800
   gcaactcaac accctggaag actccttctg tgtccccaaa catcacccag ctgttccaga 1860
   agcagaaatg gacacaggtc aacccttcac catcctgcag gtgcagcacc agggagaagc 1920
   tcaccatgct gccagagtgc cccgagggtg ccgggggcct cccgcccccc cagagaacac 1980
   agcgcagcac ggaaattcta caagacctga cggacaggaa catctccgac ttcttggtaa 2040
   aaacgtatcc tgctcttata agaagcagct taaagagcaa attctgggtc aatgaacaga 2100
   ggtatggagg aatttccatt ggaggaaagc tcccagtcgt ccccatcacg ggggaagcac 2160
   ttgttgggtt tttaagcgac cttggccgga tcatgaatgt gagcgggggc cctatcacta 2220
   gagaggcctc taaagaaata cctgatttcc ttaaacatct agaaactgaa gacaacatta 2280
   aggtgtggtt taataacaaa ggctggcatg ccctggtcag ctttctcaat gtggcccaca 2340
   acgccatctt acgggccagc ctgcctaagg acagaagccc cgaggagtat ggaatcaccg 2400
   tcattagcca acccctgaac ctgaccaagg agcagctctc agagattaca gtgctgacca 2460
   cttcagtgga tgctgtggtt gccatctgcg tgattttctc catgtccttc gtcccagcca 2520
   gctttgtcct ttatttgatc caggagcggg tgaacaaatc caagcacctc cagtttatca 2580
   gtggagtgag ccccaccacc tactgggtaa ccaacttcct ctgggacatc atgaattatt 2640
   ccgtgagtgc tgggctggtg gtgggcatct tcatcgggtt tcagaagaaa gcctacactt 2700
   ctccagaaaa ccttcctgcc cttgtggcac tgctcctgct gtatggatgg gcggtcattc 2760
   ccatgatgta cccagcatcc ttcctgtttg atgtccccag cacagcctat gtggctttat 2820
   cttgtgctaa tctgttcatc ggcatcaaca gcagtgctat taccttcatc ttggaattat 2880
   ttgagaataa ccggacgctg ctcaggttca acgccgtgct gaggaagctg ctcattgtct 2940
   tcccccactt ctgcctgggc cggggcctca ttgaccttgc actgagccag gctgtgacag 3000
   atgtctatgc ccggtttggt gaggagcact ctgcaaatcc gttccactgg gacctgattg 3060
   ggaagaacct gtttgccatg gtggtggaag gggtggtgta cttcctcctg accctgctgg 3120
   tccagcgcca cttcttcctc tcccaatgga ttgccgagcc cactaaggag cccattgttg 3180
   atgaagatga tgatgtggct gaagaaagac aaagaattat tactggtgga aataaaactg 3240
   acatcttaag gctacatgaa ctaaccaaga tttatccagg cacctccagc ccagcagtgg 3300
   acaggctgtg tgtcggagtt cgccctggag agtgctttgg cctcctggga gtgaatggtg 3360
   ccggcaaaac aaccacattc aagatgctca ctggggacac cacagtgacc tcaggggatg 3420
   ccaccgtagc aggcaagagt attttaacca atatttctga agtccatcaa aatatgggct 3480
   actgtcctca gtttgatgca atcgatgagc tgctcacagg acgagaacat ctttaccttt 3540
   atgcccggct tcgaggtgta ccagcagaag aaatcgaaaa ggttgcaaac tggagtatta 3600
   agagcctggg cctgactgtc tacgccgact gcctggctgg cacgtacagt gggggcaaca 3660
   agcggaaact ctccacagcc atcgcactca ttggctgccc accgctggtg ctgctggatg 3720
   agcccaccac agggatggac ccccaggcac gccgcatgct gtggaacgtc atcgtgagca 3780
   tcatcagaga agggagggct gtggtcctca catcccacag catggaagaa tgtgaggcac     3840
   tgtgtacccg gctggccatc atggtaaagg gcgcctttcg atgtatgggc accattcagc     3900
   atctcaagtc caaatttgga gatggctata tcgtcacaat gaagatcaaa tccccgaagg     3960
   acgacctgct tcctgacctg aaccctgtgg agcagttctt ccaggggaac ttcccaggca     4020
   gtgtgcagag ggagaggcac tacaacatgc tccagttcca ggtctcctcc tcctccctgg     4080
   cgaggatctt ccagctcctc ctctcccaca aggacagcct gctcatcgag gagtactcag     4140
   tcacacagac cacactggac caggtgtttg taaattttgc taaacagcag actgaaagtc     4200
   atgacctccc tctgcaccct cgagctgctg gagccagtcg acaagcccag gacgactaca     4260
   aagaccatga cggtgattat aaagatcatg acatcgacta caaggatgac gatgacaagt     4320
   gagcggccgc ttcgagcaga catgataaga tacattgatg agtttggaca aaccacaact     4380
   agaatgcagt gaaaaaaatg ctttatttgt gaaatttgtg atgctattgc tttatttgta     4440
   accattataa gctgcaataa acaagttaac aacaacaatt gcattcattt tatgtttcag     4500
   gttcaggggg agatgtggga ggttttttaa agcaagtaaa acctctacaa atgtggtaaa     4560
   atcgataagg atcttcctag agcatggcta cgtagataag tagcatggcg ggttaatcat     4620
   taactacaag gaacccctag tgatggagtt ggccactccc tctctgcgcg ctcgctcgct     4680
   cactgaggcc gggcgaccaa aggtcgcccg acgcccgggc tttgcccggg cggcctcagt     4740
   gagcgagcga gcgcgcag                                                  4758
<210> 76
   <211> 4844
   <212> DNA
   <213> Artificial Sequence
<220>
   <223> synthetic
<400> 76
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatccggga tttttccgat ttcggcctat tggttaaaaa atgagctgat     180
   ttaacaaaaa tttaacgcga attttaacaa aatattaacg tttataattt caggtggcat     240
   ctttcaagct tatgcacagc tggaacttca agctgtacgt catgggcagc ggcggggtac     300
   catgcacagc tggaacttca agctgtacgt catgggcagc ggcggatgca cagctggaac     360
   ttcaagctgt acgtcatggg cagcggcgat aggcacctat tggtcttact gacatccact     420
   ttgcctttct ctccacaggt ccatcctgac gggtctgttg ccaccaacct ctgggactgt     480
   gctcgttggg ggaagggaca ttgaaaccag cctggatgca gtccggcaga gccttggcat     540
   gtgtccacag cacaacatcc tgttccacca cctcacggtg gctgagcaca tgctgttcta     600
   tgcccagctg aaaggaaagt cccaggagga ggcccagctg gagatggaag ccatgttgga 660
   ggacacaggc ctccaccaca agcggaatga agaggctcag gacctatcag gtggcatgca 720
   gagaaagctg tcggttgcca ttgcctttgt gggagatgcc aaggtggtga ttctggacga 780
   acccacctct ggggtggacc cttactcgag acgctcaatc tgggatctgc tcctgaagta 840
   tcgctcaggc agaaccatca tcatgtccac tcaccacatg gacgaggccg acctccttgg 900
   ggaccgcatt gccatcattg cccagggaag gctctactgc tcaggcaccc cactcttcct 960
   gaagaactgc tttggcacag gcttgtactt aaccttggtg cgcaagatga aaaacatcca 1020
   gagccaaagg aaaggcagtg aggggacctg cagctgctcg tctaagggtt tctccaccac 1080
   gtgtccagcc cacgtcgatg acctaactcc agaacaagtc ctggatgggg atgtaaatga 1140
   gctgatggat gtagttctcc accatgttcc agaggcaaag ctggtggagt gcattggtca 1200
   agaacttatc ttccttcttc caaataagaa cttcaagcac agagcatatg ccagcctttt 1260
   cagagagctg gaggagacgc tggctgacct tggtctcagc agttttggaa tttctgacac 1320
   tcccctggaa gagatttttc tgaaggtcac ggaggattct gattcaggac ctctgtttgc 1380
   gggtggcgct cagcagaaaa gagaaaacgt caacccccga cacccctgct tgggtcccag 1440
   agagaaggct ggacagacac cccaggactc caatgtctgc tccccagggg cgccggctgc 1500
   tcacccagag ggccagcctc ccccagagcc agagtgccca ggcccgcagc tcaacacggg 1560
   gacacagctg gtcctccagc atgtgcaggc gctgctggtc aagagattcc aacacaccat 1620
   ccgcagccac aaggacttcc tggcgcagat cgtgctcccg gctacctttg tgtttttggc 1680
   tctgatgctt tctattgtta tccctccttt tggcgaatac cccgctttga cccttcaccc 1740
   ctggatatat gggcagcagt acaccttctt cagcatggat gaaccaggca gtgagcagtt 1800
   cacggtactt gcagacgtcc tcctgaataa gccaggcttt ggcaaccgct gcctgaagga 1860
   agggtggctt ccggagtacc cctgtggcaa ctcaacaccc tggaagactc cttctgtgtc 1920
   cccaaacatc acccagctgt tccagaagca gaaatggaca caggtcaacc cttcaccatc 1980
   ctgcaggtgc agcaccaggg agaagctcac catgctgcca gagtgccccg agggtgccgg 2040
   gggcctcccg cccccccaga gaacacagcg cagcacggaa attctacaag acctgacgga 2100
   caggaacatc tccgacttct tggtaaaaac gtatcctgct cttataagaa gcagcttaaa 2160
   gagcaaattc tgggtcaatg aacagaggta tggaggaatt tccattggag gaaagctccc 2220
   agtcgtcccc atcacggggg aagcacttgt tgggttttta agcgaccttg gccggatcat 2280
   gaatgtgagc gggggcccta tcactagaga ggcctctaaa gaaatacctg atttccttaa 2340
   acatctagaa actgaagaca acattaaggt gtggtttaat aacaaaggct ggcatgccct 2400
   ggtcagcttt ctcaatgtgg cccacaacgc catcttacgg gccagcctgc ctaaggacag 2460
   aagccccgag gagtatggaa tcaccgtcat tagccaaccc ctgaacctga ccaaggagca 2520
   gctctcagag attacagtgc tgaccacttc agtggatgct gtggttgcca tctgcgtgat 2580
   tttctccatg tccttcgtcc cagccagctt tgtcctttat ttgatccagg agcgggtgaa 2640
   caaatccaag cacctccagt ttatcagtgg agtgagcccc accacctact gggtaaccaa 2700
   cttcctctgg gacatcatga attattccgt gagtgctggg ctggtggtgg gcatcttcat 2760
   cgggtttcag aagaaagcct acacttctcc agaaaacctt cctgcccttg tggcactgct 2820
   cctgctgtat ggatgggcgg tcattcccat gatgtaccca gcatccttcc tgtttgatgt 2880
   ccccagcaca gcctatgtgg ctttatcttg tgctaatctg ttcatcggca tcaacagcag 2940
   tgctattacc ttcatcttgg aattatttga gaataaccgg acgctgctca ggttcaacgc 3000
   cgtgctgagg aagctgctca ttgtcttccc ccacttctgc ctgggccggg gcctcattga 3060
   ccttgcactg agccaggctg tgacagatgt ctatgcccgg tttggtgagg agcactctgc 3120
   aaatccgttc cactgggacc tgattgggaa gaacctgttt gccatggtgg tggaaggggt 3180
   ggtgtacttc ctcctgaccc tgctggtcca gcgccacttc ttcctctccc aatggattgc 3240
   cgagcccact aaggagccca ttgttgatga agatgatgat gtggctgaag aaagacaaag 3300
   aattattact ggtggaaata aaactgacat cttaaggcta catgaactaa ccaagattta 3360
   tccaggcacc tccagcccag cagtggacag gctgtgtgtc ggagttcgcc ctggagagtg 3420
   ctttggcctc ctgggagtga atggtgccgg caaaacaacc acattcaaga tgctcactgg 3480
   ggacaccaca gtgacctcag gggatgccac cgtagcaggc aagagtattt taaccaatat 3540
   ttctgaagtc catcaaaata tgggctactg tcctcagttt gatgcaatcg atgagctgct 3600
   cacaggacga gaacatcttt acctttatgc ccggcttcga ggtgtaccag cagaagaaat 3660
   cgaaaaggtt gcaaactgga gtattaagag cctgggcctg actgtctacg ccgactgcct 3720
   ggctggcacg tacagtgggg gcaacaagcg gaaactctcc acagccatcg cactcattgg 3780
   ctgcccaccg ctggtgctgc tggatgagcc caccacaggg atggaccccc aggcacgccg 3840
   catgctgtgg aacgtcatcg tgagcatcat cagagaaggg agggctgtgg tcctcacatc 3900
   ccacagcatg gaagaatgtg aggcactgtg tacccggctg gccatcatgg taaagggcgc 3960
   ctttcgatgt atgggcacca ttcagcatct caagtccaaa tttggagatg gctatatcgt 4020
   cacaatgaag atcaaatccc cgaaggacga cctgcttcct gacctgaacc ctgtggagca 4080
   gttcttccag gggaacttcc caggcagtgt gcagagggag aggcactaca acatgctcca 4140
   gttccaggtc tcctcctcct ccctggcgag gatcttccag ctcctcctct cccacaagga 4200
   cagcctgctc atcgaggagt actcagtcac acagaccaca ctggaccagg tgtttgtaaa 4260
   ttttgctaaa cagcagactg aaagtcatga cctccctctg caccctcgag ctgctggagc 4320
   cagtcgacaa gcccaggacg actacaaaga ccatgacggt gattataaag atcatgacat 4380
   cgactacaag gatgacgatg acaagtgagc ggccgcttcg agcagacatg ataagataca 4440
   ttgatgagtt tggacaaacc acaactagaa tgcagtgaaa aaaatgcttt atttgtgaaa 4500
   tttgtgatgc tattgcttta tttgtaacca ttataagctg caataaacaa gttaacaaca 4560
   acaattgcat tcattttatg tttcaggttc agggggagat gtgggaggtt ttttaaagca 4620
   agtaaaacct ctacaaatgt ggtaaaatcg ataaggatct tcctagagca tggctacgta 4680
   gataagtagc atggcgggtt aatcattaac tacaaggaac ccctagtgat ggagttggcc 4740
   actccctctc tgcgcgctcg ctcgctcact gaggccgggc gaccaaaggt cgcccgacgc 4800
   ccgggctttg cccgggcggc ctcagtgagc gagcgagcgc gcag 4844
<210> 77
   <211> 4944
   <212> DNA
   <213> Artificial Sequence
<220>
   <223> synthetic
<400> 77
   ctgcgcgctc gctcgctcac tgaggccgcc cgggcaaagc ccgggcgtcg ggcgaccttt      60
   ggtcgcccgg cctcagtgag cgagcgagcg cgcagagagg gagtggccaa ctccatcact     120
   aggggttcct ggatccggga tttttccgat ttcggcctat tggttaaaaa atgagctgat     180
   ttaacaaaaa tttaacgcga attttaacaa aatattaacg tttataattt caggtggcat     240
   ctttcaagct tatgcagatc ttcgtgaaga ctctgactgg taagaccatc accctcgagg     300
   tggagcccag tgacaccatc gagaatgtca aggcaaagat ccaagataag gaaggcattc     360
   ctcctgatca gcagaggttg atctttgccg gaaaacagct ggaagatggt cgtaccctgt     420
   ctgactacaa catccagaaa gagtccacct tgcacctggt actccgtctc agaggtgggc     480
   gaagcttgat aggcacctat tggtcttact gacatccact ttgcctttct ctccacaggt     540
   ccatcctgac gggtctgttg ccaccaacct ctgggactgt gctcgttggg ggaagggaca     600
   ttgaaaccag cctggatgca gtccggcaga gccttggcat gtgtccacag cacaacatcc     660
   tgttccacca cctcacggtg gctgagcaca tgctgttcta tgcccagctg aaaggaaagt     720
   cccaggagga ggcccagctg gagatggaag ccatgttgga ggacacaggc ctccaccaca     780
   agcggaatga agaggctcag gacctatcag gtggcatgca gagaaagctg tcggttgcca     840
   ttgcctttgt gggagatgcc aaggtggtga ttctggacga acccacctct ggggtggacc     900
   cttactcgag acgctcaatc tgggatctgc tcctgaagta tcgctcaggc agaaccatca     960
   tcatgtccac tcaccacatg gacgaggccg acctccttgg ggaccgcatt gccatcattg    1020
   cccagggaag gctctactgc tcaggcaccc cactcttcct gaagaactgc tttggcacag    1080
   gcttgtactt aaccttggtg cgcaagatga aaaacatcca gagccaaagg aaaggcagtg 1140
   aggggacctg cagctgctcg tctaagggtt tctccaccac gtgtccagcc cacgtcgatg 1200
   acctaactcc agaacaagtc ctggatgggg atgtaaatga gctgatggat gtagttctcc 1260
   accatgttcc agaggcaaag ctggtggagt gcattggtca agaacttatc ttccttcttc 1320
   caaataagaa cttcaagcac agagcatatg ccagcctttt cagagagctg gaggagacgc 1380
   tggctgacct tggtctcagc agttttggaa tttctgacac tcccctggaa gagatttttc 1440
   tgaaggtcac ggaggattct gattcaggac ctctgtttgc gggtggcgct cagcagaaaa 1500
   gagaaaacgt caacccccga cacccctgct tgggtcccag agagaaggct ggacagacac 1560
   cccaggactc caatgtctgc tccccagggg cgccggctgc tcacccagag ggccagcctc 1620
   ccccagagcc agagtgccca ggcccgcagc tcaacacggg gacacagctg gtcctccagc 1680
   atgtgcaggc gctgctggtc aagagattcc aacacaccat ccgcagccac aaggacttcc 1740
   tggcgcagat cgtgctcccg gctacctttg tgtttttggc tctgatgctt tctattgtta 1800
   tccctccttt tggcgaatac cccgctttga cccttcaccc ctggatatat gggcagcagt 1860
   acaccttctt cagcatggat gaaccaggca gtgagcagtt cacggtactt gcagacgtcc 1920
   tcctgaataa gccaggcttt ggcaaccgct gcctgaagga agggtggctt ccggagtacc 1980
   cctgtggcaa ctcaacaccc tggaagactc cttctgtgtc cccaaacatc acccagctgt 2040
   tccagaagca gaaatggaca caggtcaacc cttcaccatc ctgcaggtgc agcaccaggg 2100
   agaagctcac catgctgcca gagtgccccg agggtgccgg gggcctcccg cccccccaga 2160
   gaacacagcg cagcacggaa attctacaag acctgacgga caggaacatc tccgacttct 2220
   tggtaaaaac gtatcctgct cttataagaa gcagcttaaa gagcaaattc tgggtcaatg 2280
   aacagaggta tggaggaatt tccattggag gaaagctccc agtcgtcccc atcacggggg 2340
   aagcacttgt tgggttttta agcgaccttg gccggatcat gaatgtgagc gggggcccta 2400
   tcactagaga ggcctctaaa gaaatacctg atttccttaa acatctagaa actgaagaca 2460
   acattaaggt gtggtttaat aacaaaggct ggcatgccct ggtcagcttt ctcaatgtgg 2520
   cccacaacgc catcttacgg gccagcctgc ctaaggacag aagccccgag gagtatggaa 2580
   tcaccgtcat tagccaaccc ctgaacctga ccaaggagca gctctcagag attacagtgc 2640
   tgaccacttc agtggatgct gtggttgcca tctgcgtgat tttctccatg tccttcgtcc 2700
   cagccagctt tgtcctttat ttgatccagg agcgggtgaa caaatccaag cacctccagt 2760
   ttatcagtgg agtgagcccc accacctact gggtaaccaa cttcctctgg gacatcatga 2820
   attattccgt gagtgctggg ctggtggtgg gcatcttcat cgggtttcag aagaaagcct 2880
   acacttctcc agaaaacctt cctgcccttg tggcactgct cctgctgtat ggatgggcgg 2940
   tcattcccat gatgtaccca gcatccttcc tgtttgatgt ccccagcaca gcctatgtgg 3000
   ctttatcttg tgctaatctg ttcatcggca tcaacagcag tgctattacc ttcatcttgg 3060
   aattatttga gaataaccgg acgctgctca ggttcaacgc cgtgctgagg aagctgctca 3120
   ttgtcttccc ccacttctgc ctgggccggg gcctcattga ccttgcactg agccaggctg 3180
   tgacagatgt ctatgcccgg tttggtgagg agcactctgc aaatccgttc cactgggacc 3240
   tgattgggaa gaacctgttt gccatggtgg tggaaggggt ggtgtacttc ctcctgaccc 3300
   tgctggtcca gcgccacttc ttcctctccc aatggattgc cgagcccact aaggagccca 3360
   ttgttgatga agatgatgat gtggctgaag aaagacaaag aattattact ggtggaaata 3420
   aaactgacat cttaaggcta catgaactaa ccaagattta tccaggcacc tccagcccag 3480
   cagtggacag gctgtgtgtc ggagttcgcc ctggagagtg ctttggcctc ctgggagtga 3540
   atggtgccgg caaaacaacc acattcaaga tgctcactgg ggacaccaca gtgacctcag 3600
   gggatgccac cgtagcaggc aagagtattt taaccaatat ttctgaagtc catcaaaata 3660
   tgggctactg tcctcagttt gatgcaatcg atgagctgct cacaggacga gaacatcttt 3720
   acctttatgc ccggcttcga ggtgtaccag cagaagaaat cgaaaaggtt gcaaactgga 3780
   gtattaagag cctgggcctg actgtctacg ccgactgcct ggctggcacg tacagtgggg 3840
   gcaacaagcg gaaactctcc acagccatcg cactcattgg ctgcccaccg ctggtgctgc 3900
   tggatgagcc caccacaggg atggaccccc aggcacgccg catgctgtgg aacgtcatcg 3960
   tgagcatcat cagagaaggg agggctgtgg tcctcacatc ccacagcatg gaagaatgtg 4020
   aggcactgtg tacccggctg gccatcatgg taaagggcgc ctttcgatgt atgggcacca 4080
   ttcagcatct caagtccaaa tttggagatg gctatatcgt cacaatgaag atcaaatccc 4140
   cgaaggacga cctgcttcct gacctgaacc ctgtggagca gttcttccag gggaacttcc 4200
   caggcagtgt gcagagggag aggcactaca acatgctcca gttccaggtc tcctcctcct 4260
   ccctggcgag gatcttccag ctcctcctct cccacaagga cagcctgctc atcgaggagt 4320
   actcagtcac acagaccaca ctggaccagg tgtttgtaaa ttttgctaaa cagcagactg 4380
   aaagtcatga cctccctctg caccctcgag ctgctggagc cagtcgacaa gcccaggacg 4440
   actacaaaga ccatgacggt gattataaag atcatgacat cgactacaag gatgacgatg 4500
   acaagtgagc ggccgcttcg agcagacatg ataagataca ttgatgagtt tggacaaacc 4560
   acaactagaa tgcagtgaaa aaaatgcttt atttgtgaaa tttgtgatgc tattgcttta 4620
   tttgtaacca ttataagctg caataaacaa gttaacaaca acaattgcat tcattttatg 4680
   tttcaggttc agggggagat gtgggaggtt ttttaaagca agtaaaacct ctacaaatgt 4740
   ggtaaaatcg ataaggatct tcctagagca tggctacgta gataagtagc atggcgggtt 4800
   aatcattaac tacaaggaac ccctagtgat ggagttggcc actccctctc tgcgcgctcg 4860
   ctcgctcact gaggccgggc gaccaaaggt cgcccgacgc ccgggctttg cccgggcggc 4920
   ctcagtgagc gagcgagcgc gcag 4944
<210> 78
   <211> 228
   <212> DNA
   <213> Artificial Sequence
<220>
   <223> synthetic
<400> 78
   atgcagatct tcgtgaagac tctgactggt aagaccatca ccctcgaggt ggagcccagt 60
   gacaccatcg agaatgtcaa ggcaaagatc caagataagg aaggcattcc tcctgatcag 120
   cagaggttga tctttgccgg aaaacagctg gaagatggtc gtaccctgtc tgactacaac 180
   atccagaaag agtccacctt gcacctggta ctccgtctca gaggtggg 228

## Description

### TECHNICAL FIELD

The present invention relates to constructs, vectors, relative host cells and pharmaceutical compositions which allow an effective gene therapy, in particular of genes larger than 5Kb.

### BACKGROUND OF THE INVENTION

Sight-restoring therapy for many inherited retinal degenerations (IRDs) is still a major unmet medical need. Gene therapy with adeno-associated viral (AAV) vectors represents, to date, the most promising approach for treatment of many IRDs. Indeed, years of pre-clinical research and a number of clinical trials for different IRDs have defined AAV's ability to efficiently deliver therapeutic genes to diseased retinal layers [photoreceptors (PR) and retinal pigment epithelium (RPE)] ^{1,2} and have underlined their excellent safety and efficacy profiles in humans ³⁻⁷. Despite this, one of the main obstacles to expand this success to other blinding condition is the packaging capacity of AAV vectors (~5 kb). This has become a limiting factor for the development of gene replacement therapy for common IRDs due to mutations in genes with a coding sequence (CDS) larger than 5 kb (herein referred to also as large genes).

Therefore, considerable interest has been directed in recent years towards the identification of strategies to increase the carrying capacity of AAV. Dual AAV vectors, based on the ability of AAV genomes to concatamerize via intermolecular recombination, have been successfully exploited to address this issue ¹⁴⁻¹⁶. Dual AAV vectors are generated by splitting a large transgene expression cassette in two separate halves each packaged in a single normal size (NS; < 5 kb) AAV vector. The reconstitution of the full-length expression cassette is achieved upon co-infection of the same cell by both dual AAV vectors followed by either: i) inverted terminal repeat (ITR)-mediated tail-to-head concatemerization of the two vector genomes followed by splicing (dual AAV trans-splicing, TS)¹⁵, ii) homologous recombination between overlapping regions contained in the two vector genomes (dual AAV overlapping, OV)¹⁵, iii) a combination of the two (dual AAV hybrid)¹⁶. Others and the inventors have recently shown the potential of dual AAV vectors in the retina ^{14, 17-19}. The most used recombinogenic regions used in the context of dual AAV hybrid vectors derive from the 872 bp sequence of the middle one-third of the human alkaline phosphatase cDNA that has been shown to confer high levels of dual AAV hybrid vectors reconstitution ¹⁶. The inventors showed that dual AAV hybrid vectors including the AK sequence outperform those including the sense alkaline phosphatase head region sequence ¹⁴, which the inventors generated based on the description provided in Ghosh et al ²². Additional studies have shown that either the head or tail of this alkaline phosphatase region confers levels of transgene reconstitution similar to those achieved with the full-length middle one-third of the alkaline phosphatase cDNA ²². The inventors found that dual AAV trans-splicing and hybrid AK vectors (that contain the short AK recombinogenic sequence from the F1 phage) transduce efficiently the mouse and pig retina and rescue mouse models of Stargardt disease (STGD) and Usher 1B (USH1B) ^{14,19}. The levels of PR transduction achieved with dual AAV TS and hybrid AK vectors resulted in significant improvement of the retinal phenotype of mouse models of IRDs and may be effective for treating inherited blinding conditions. Furthermore, vectors with heterologous ITR from serotypes 2 and 5 (ITR2 and ITR5, respectively), which are highly divergent (58% of homology 23), show both reduced ability to form circular monomers and increased directional tail-to-head concatamerization than vectors with homologous ITR ²⁴. Based on this, Yan et al have shown that dual AAV vectors with heterologous ITR2 and ITR5 reconstitute transgene expression more efficiently than dual AAV vectors with homologous ITR ^{24,25}.

Although these studies have highlighted the potential of dual AAV vectors for large gene reconstitution in the tissue of interest, such as the retina, they have also underlined critical issues that need to be addressed before considering further clinical translation of this strategy.

The production of truncated protein products from the 5'-half vector that contains the promoter sequence and/or from the 3'-half vector due to the low promoter activity of the ITR ^{14, 17, 20, 21} , still remains a major issue associated with the use of dual vectors . No formal toxicity studies have been so far performed to evaluate the potential detrimental effects of these truncated products *in vivo,* thus raising safety concern. Therefore, reduction or abolishment of their production is highly desirable. The present invention is thus aimed to solve this major issue associated with the use of dual vector systems.

### SUMMARY OF THE INVENTION

The present invention relates to constructs, vectors, relative host cells and pharmaceutical compositions which allow an effective gene therapy, in particular of genes larger than 5Kb. Large genes include, among others:

| **DISEASE** | **CAUSATIVE GENE** | **CELL AFFECTED** | **CDS SIZE (kb)** |
|---|---|---|---|
| USH1F | Protocadherin-related 15 **(PCDH15)** | Neurosensory retina | 5.9 |
| CSNB2 | Calcium channel, voltage-dependent, L type, alpha 1F subunit **(CACNA1)** | Photoreceptors | 5.9 |
| ad RP | Small nuclear ribonucleoprotein 200 kDa **(SNRNP200)** | Photoreceptors and RPE | 6.4 |

| | | | |
|---|---|---|---|
| ad or ar RP | Retinitis pigmentosa 1 **(RP1)** | Photoreceptors | 6.5 |
| USH1B | Myosin IIVA (**MYO7A**) | Photoreceptors and RPE | 6.7 |
| STGD1 | ATP-binding cassette, sub-family A, member 4 **(ABCA4)** | Photoreceptors | 6.8 |
| ad RP | Pre-mRNA processing factor 8 homologue **(PRPF8)** | Photoreceptors and RPE | 7.0 |
| Occult macular dystrophy | Retinitis pigmentosa 1-like 1 **(RP1L1)** | Photoreceptors | 7.2 |
| LCA10 | Centrosomal protein 290 kDa **(CEP290)** | Photoreceptors | 7.5 |
| RP | **EYS** | Photoreceptors and extracellular matrix | 9,4 |
| USH1D | Cadherin 23 **(CDH23)** | Neurosensory retina | 10 |
| Alstrom Syndrome | **ALMS1** | Photoreceptors | 12,5 |
| USH2A and RP | Usherin **(USH2A)** | Neurosensory retina | 15.6 |
| ad macular dystrophy | Hemicentin 1 **(HMCN1)** | Photoreceptors and RPE | 17 |
| USH2C | G-coupled receptor 98 **(GPR98)** | Neurosensory retina | 18.9 |

Stargardt disease (STGD1; MIM#248200) is the most common form of inherited macular degeneration caused by mutations in *ABCA4* (CDS: 6822 bp), which encodes the photoreceptor-specific all-trans retinal transporter ^{8, 9}. Cone-rod dystrophy type 3, fundus flavimaculatus, age-related macular degeneration type 2, Early-onset severe retinal dystrophy, and Retinitis pigmentosa type 19 are also associated with ABCA4 mutations (ABCA4-associated diseases). Usher syndrome type IB (USH1B; MIM#276900) is the most severe combined form of retinitis pigmentosa and deafness caused by mutations in *MYO7A* (CDS: 6648 bp)¹⁰, which encodes for an actin-based motor expressed in both PR and RPE within the retina ¹¹⁻¹³.

Furthermore, many other genetic diseases, not necessarily causing retinal symptoms, are due to mutations in large genes. These include, among others: Duchenne muscular dystrophy due to mutations in *DMD,* cystic fibrosis due to mutations in *CFTR,* hemophilia A due to mutations in *F8* and dysferlinopathies due to mutations in the *DYSF* gene.

In particular, the present invention is aimed to decreasing expression of a truncated protein product associated with multiple AAV vector systems, by use of signals that mediate the degradation of proteins or avoid their translation (hereinafter degradation signals). Degradation signals have never been used in the context of multiple viral vectors. In the present invention it was surprisingly found that when a degradation signal is present in at least one vector of a multiple vector system, expression of protein in truncated form is significantly decreased, leading to a higher yield of full length protein

In a first aspect therefore the present invention provides an AAV multiple vector system to express the coding sequence of a gene of interest in a cell, said coding sequence comprising a first portion and a second portion, said vector system comprising:
a) a first vector comprising:
   - said first portion of said coding sequence (CDS1),
   - a first reconstitution sequence; and
b) a second vector comprising:
   - said second portion of said coding sequence (CDS2),
   - a second reconstitution sequence,

wherein said first and second reconstitution sequences are selected from the group of:
   i] the first reconstitution sequence consists of the 3' end of said first portion of the coding sequence and the second reconstitution sequence consists of the 5'end of said second portion of the coding sequence, said first and second reconstitution sequences being overlapping sequences; or
   ii] the first reconstitution sequence comprises a splicing donor signal (SD) and the second reconstitution sequence comprises a splicing acceptor signal (SA), optionally each one of first and second reconstitution sequence further comprises a recombinogenic sequence,
characterized by the fact that either one or both of the first and second vector further comprises a nucleotide sequence of a degradation signal said sequence being located in case of i) at the 3' end of the CDS1 and/or at the 5' end of the CDS2 and in case of ii) in 3' position relative to the SD and/or in 5' position relative to the SA, wherein
   - the recombinogenic sequence is selected from the group consisting of:
      GGGATTTTGCCGATTTCGGCCTATTGGTTAAAAAATGAGCTGATTTAACAAAAATTT AACGCGAATTTTAACAAAAT (SEQ ID No 22, AK) or
      GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO. 25 (AP2), and SEQ ID NO. 26 (AP) and
   - the nucleotide sequence of the degradation signal comprises or consists of a sequence encoding SEO ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16), or the nucleotide sequence of a degradation signal comprises or consists of SEQ ID No. 16.

Preferably both of the first and second vector further comprise said nucleotide sequence of a degradation signal, wherein the nucleotide sequence of the degradation signal in the first vector is identical to or differs from that in the second vector.

Preferably the first reconstitution sequence comprises a splicing donor signal (SD) and a recombinogenic region in 3' position relative to said SD, the second reconstitution sequence comprises a splicing acceptor signal (SA) and a recombinogenic sequence in 5' position relative to the SA; wherein said nucleotide sequence of a degradation signal is localized at the 5' end and/or at the 3' end of the nucleotide sequence of the recombinogenic region of either one or both of the first and second vector.

Preferably the first vector further comprises a promoter sequence operably linked to the 5'end portion of said first portion of the coding sequence (CDS1).

Preferably both of the first vector and the second vector further comprise a 5'-terminal repeat (5'-TR) nucleotide sequence and a 3'-terminal repeat (3'-TR) nucleotide sequence, preferably the 5'-TR is a 5'-inverted terminal repeat (5'-ITR) nucleotide sequence and the 3'-TR is a 3'-inverted terminal repeat (3'-ITR) nucleotide sequence, preferably the ITRs derive from the same virus serotype or from different virus serotypes.

Preferably the coding sequence is split into the first portion and the second portion at a natural exon-exon junction.

Preferably the splicing donor signal comprises or consists essentially of a sequence that is at least 70%, 75%, 80%, 85%, 90%, 95% or 100 % identical to

Preferably the splicing acceptor signal comprises or consists essentially of a sequence that is at least 70%, 75%, 80%, 85%, 90%, 95% or 100 % identical to
GATAGGCACCTATTGGTCTTACTGACATCCACTTTGCCTTTCTCTCCACAG (SEQ ID No. 28)

Preferably the first vector further comprises at least one enhancer nucleotide sequence, operably linked to the coding sequence.

Preferably the coding sequence encodes a protein able to correct a retinal degeneration. Preferably the coding sequence encodes a protein able to correct Duchenne muscular dystrophy, cystic fibrosis, hemophilia A and dysferlinopathies.

In case of retinal degradation, preferably the coding sequence is the coding sequence of a gene selected from the group consisting of: ABCA4, MYO7A, CEP290, CDH23, EYS, PCDH15, CACNA1, SNRNP200, RP1, PRPF8, RP1L1, ALMS1, USH2A, GPR98, HMCN1.

In case of Duchenne muscular dystrophy, cystic fibrosis, hemophilia A and dysferlinopathies, preferably the coding sequence is the coding sequence of a gene selected from the group consisting of: DMD, CFTR, F8 and DYSF,

Preferably the first vector does not comprise a poly-adenylation signal nucleotide sequence.

Preferably the vector system comprises:
a) a first vector comprising in a 5'-3' direction:
   - a 5'-inverted terminal repeat (5'-ITR) sequence;
   - a promoter sequence;
   - a 5' end portion of a coding sequence of a gene of interest (CDS1), said 5'end portion being operably linked to and under control of said promoter;
   - a nucleotide sequence of a splicing donor signal;
   - a nucleotide sequence of a recombinogenic region; and
   - a 3'-inverted terminal repeat (3'-ITR) sequence; and
b) a second vector comprising in a 5'-3' direction:
   - a 5'-inverted terminal repeat (5'-ITR) sequence;
   - a nucleotide sequence of a recombinogenic region;
   - a nucleotide sequence of a splicing acceptor signal;
   - the 3'end of the coding sequence (CDS2);
   - a poly-adenylation signal nucleotide sequence; and
   - a 3'-inverted terminal repeat (3'-ITR) sequence,
      characterized by further comprising a nucleotide sequence of a degradation signal, said sequence being localized at 5' end or 3' end of the nucleotide sequence of the recombinogenic region of either one or both of the first and second vector
      wherein
         - the recombinogenic sequence is selected from the group consisting of:
            GGGATTTTGCCGATTTCGGCCTATTGGTTAAAAAATGAGCTGATTTAACAAAAATTT AACGCGAATTTTAACAAAAT (SEQ ID No 22, AK) or
            GGGATTTTTCCGATTTCGGCCTATTGGTTAAAAAATGAGCTGATTTAACAAAAATTT AACGCGAATTTTAACAAAAT (SEQ ID NO.23, AK), SEQ ID NO. 24 (AP1), SEQ ID NO 25 (AP2), and SEQ ID NO. 26 (AP) and
         - the nucleotide sequence of the degradation signal comprises or consists of a sequence encoding SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16), or the nucleotide sequence of a degradation signal comprises or consists of SEQ ID No. 16.

Preferably said first and second adeno-associated viral (AAV) vectors are selected from the same or different AAV serotypes, preferably the adeno-associated virus is selected from the serotype 2, the serotype 8, the serotype 5, the serotype 7 or the serotype 9.

Preferably the vector system of the invention further comprises a third vector comprising a third portion of said coding sequence (CDS3) and a reconstitution sequence, wherein the second vector comprises two reconstitution sequences, each reconstitution sequence located at each end of CDS2.

Prefererably the reconstitution sequence of the first vector consists of the 3' end of CDS1, the two reconstitution sequences of the second vector consist each respectively of the 5'end and of the 3' end of CDS2, the reconstitution sequence of the third vector consists of the 5' end of CDS3;
wherein said reconstitution sequence of the first vector and said reconstitution sequence of the second vector consisting of the 5'end of CDS2 are overlapping sequences, and
wherein said reconstitution sequence of the second vector consisting of the 3'end of CDS2 and said reconstitution sequence of said third vector are overlapping sequences;
wherein said second vector further comprises a degradation signal, said degradation signal being located at the 5' end and/or at the 3' end of the CDS2.

Preferably the third vector further comprises at least one nucleotide sequence of a degradation signal.

Preferably the second vector further comprises a poly-adenylation signal nucleotide sequence linked to the 3'end portion of said coding sequence (CDS2).

The present invention provides a host cell transformed with the vector system as defined above. Preferably the vector system or the host cell of the invention is for medical use. Preferably for use in gene therapy. Preferably for use in the treatment and/or prevention of a pathology or disease characterized by a retinal degeneration or for use in the prevention and/or treatment of Duchenne muscular dystrophy, cystic fibrosis, hemophilia A and dysferlinopathies.

Preferably the retinal degeneration is inherited.

Preferably the pathology or disease is selected from the group consisting of: retinitis pigmentosa (RP), Leber congenital amaurosis (LCA), Stargardt disease (STGD), Usher disease (USH), Alstrom syndrome, congenital stationary night blindness (CSNB), macular dystrophy, occult macular dystrophy, a disease caused by a mutation in the *ABCA4* gene.

The invention provides a pharmaceutical composition comprising the vector system or the host cell as defined above and pharmaceutically acceptable vehicle.

The invention provides a nucleotide sequence of a degradation signal that comprises or consists of a sequence encoding SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16), or that comprises or consists of SEQ lD No.16 in a AAV multiple vector system for use in a method to decrease expression of a protein in truncated form or for use in a method for decreasing expression of a protein in truncated form comprising inserting said nucleotide sequence of a degradation signal in one or more vector of a AAV multiple vector system.

According to preferred embodiments of the invention, the vector system to express the coding sequence of a gene of interest in a cell comprises two vectors, each vector comprising a different portion of said coding sequence and a reconstitution sequence; preferably, the reconstitution sequence of the first vector is a sequence comprising a splicing donor, while the reconstitution sequence of the second vector is a sequence comprising a splicing acceptor.

According to a further preferred embodiments of the invention, the vector system to express the coding sequence of a gene of interest in a cell comprises three vectors, each vector comprising a different portion of said coding sequence and at least one reconstitution sequence; preferably, the first vector comprises a reconstitution sequence comprising a splicing donor in 3' position relative to the first portion of the coding sequence, the second vector comprises a reconstitution sequence comprising a splicing acceptor in 5' position relative to the second portion coding sequence and a reconstitution sequence comprising a splicing donor in 3' position relative to the second portion of the coding sequence, the third vector comprises a reconstitution sequence comprising a splicing acceptor in 5' position relative to the third portion coding sequence. Preferably, the reconstitution sequences of the first and the second vector or the reconstitution sequences of the first, the second and the third vector further comprise a recombinogenic region, preferably located in 3' position relative to the splicing donor and in 5' position relative to the splicing acceptor.

Preferably all the vectors of the vector system of the invention further comprise a nucleotide sequence of a degradation signal.

According to preferred embodiments of the invention, wherein the vectors comprise reconstitution sequences that comprise a recombinogenic region, a degradation signals is localized at the 5' end or at the 3' end of the sequence of said recombinogenic region. According to preferred embodiments of the invention, the vector system to express the coding sequence of a gene of interest in a cell comprises two vectors; the first vector of the vector system comprising in a 5'-3' direction:
- the 5'end portion of the coding sequence of a gene of interest,
- the nucleic acid sequence of a splicing donor signal,
- the nucleic acid sequence of a recombinogenic region, and
- the nucleic acid sequence of a degradation signal.

According to preferred embodiments of the invention, the vector system to express the coding sequence of a gene of interest in a cell comprises two vectors, the second vector of the vector system comprising in a 5'-3' direction:
- the nucleic acid sequence of the recombinogenic region,
- the nucleic acid sequence of the degradation signal,
- the nucleic acid sequence of the splicing acceptor signal, and
- the 3'end portion of the coding sequence of a gene of interest
wherein
- the recombinogenic sequence is selected from the group consisting of:
   GGGATTTTGCCGATTTCGGCCTATTGGTTAAAAAATGAGCTGATTTAACAAAAATTT AACGCGAATTTTAACAAAAT (SEQ ID No. 22, AK) or
   GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO 25 (AP2) and SEQ ID NO. 26 (AP) and
- the nucleotide sequence of the degradation signal comprises or consists of a sequence encoding SEQ ID No. 1 (CL1), SEQ ID No. 2 (CL2), SEQ ID No. 3 (CL6), SEQ ID No . 4 (CL9), SEQ ID No. 5 (CL10), SEQ ID No. 6 (CL11), SEQ ID No. 7 (CL12), SEQ ID No . 8 (CL15), SEQ ID No. 9 (CL16), or the nucleotide sequence of a degradation signal comprises or consists of SEQ ID No. 16.

Preferably, the first vector of a vector system according to the invention further comprises a promoter sequence, more preferably said promoter sequence is operably linked to the 5'end of the first portion of the coding sequence of a gene of interest.

Preferably, the second vector of a vector system consisting of two vectors further comprises a poly-adenylation signal nucleic acid sequence, more preferably said poly-adenylation signal nucleic acid sequence is linked to the 3'end of the second portion of the coding sequence of a gene of interest. Preferably the first vector of a vector system according to the invention does not comprise a poly-adenylation signal nucleic acid sequence.

Preferably, the third vector of a vector system consisting of three vectors further comprises a poly-adenylation signal nucleic acid sequence, more preferably said poly-adenylation signal nucleic acid sequence is linked to the 3'end of the third portion of the coding sequence of a gene of interest.

Preferably, at least one of the vectors of the vector system of the invention, more preferably the first vector of the vector system of the invention, comprises a degradation signal of sequence comprising or consisting of a sequence encoding CL1 SEQ ID No. 1; preferably, said sequence encoding CL1 SEQ ID No. 1 comprises or consists of SEQ ID No. 16.

According to a preferred embodiment of the invention, the vector system comprises:
a) a first vector comprising in a 5'-3' direction:
   - a 5'-inverted terminal repeat (5'-ITR) sequence;
   - a promoter sequence;
   - a first portion of a coding sequence of a gene of interest, preferably being the 5'end portion of said coding sequence, preferably said first portion being operably linked to and under control of said promoter;
   - a nucleic acid sequence of a splicing donor signal;
   - a nucleic acid sequence of a recombinogenic region; and
   - a 3'-inverted terminal repeat (3'-ITR) sequence; and
b) a second vector comprising in a 5'-3' direction:
   - a 5'-inverted terminal repeat (5'-ITR) sequence;
   - a nucleic acid sequence of a recombinogenic region;
   - a nucleic acid sequence of a splicing acceptor signal;
   - a second portion of a coding sequence of a gene of interest, preferably being the 3'end portion of said coding sequence;
   - a poly-adenylation signal nucleic acid sequence; and
   - a 3'-inverted terminal repeat (3'-ITR) sequence,
      said first and/or second vector further comprising a nucleic acid sequence of a degradation signal, said sequence being localized at the 5' end or 3' end of the nucleic acid sequence of the recombinogenic region wherein
   - the recombinogenic sequence is selected from the group consisting of:
      GGGATTTTGCCGATTTCGGCCTATTGGTTAAAAAATGAGCTGATTTAACAAAAATTT AACGCGAATTTTAACAAAAT (SEQ ID No. 22, AK) or
      GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO. 25 (AP2) and SEQ ID NO. 26 (AP) and
   - the nucleotide sequence of the degradation signal comprises or consists of a sequence encoding SEQ ID No. 1 (CL1), SEQ ID No. 2 (CL2), SEQ ID No. 3 (CL6), SEQ ID No 4 (CL9), SEQ ID No. 5 (CL10), SEQ ID No. 6 (CL11), SEQ ID No. 7 (CL12), SEQ ID No. 8 (CL15), SEQ ID No. 9 (CL16), or the nucleotide sequence of a degradation signal comprises or consists of SEQ ID No. 16.

According to a further preferred embodiment of the invention, the vector system comprises:
a) a first vector comprising in a 5'-3' direction:
   - a 5'-inverted terminal repeat (5'-ITR) sequence;
   - a promoter sequence;
   - a first portion of a coding sequence of a gene of interest, preferably being operably linked to and under control of said promoter;
   - a nucleic acid sequence of a splicing donor signal;
   - a nucleic acid sequence of a recombinogenic region; and
   - a 3'-inverted terminal repeat (3'-ITR) sequence;
b) a second vector comprising in a 5'-3' direction:
   - a 5'-inverted terminal repeat (5'-ITR) sequence;
   - a nucleic acid sequence of a recombinogenic region;
   - a nucleic acid sequence of a splicing acceptor signal;
   - a second portion of a coding sequence of a gene of interest;
   - a nucleic acid sequence of a splicing donor signal;
   - a nucleic acid sequence of a recombinogenic region;
   - a 3'-inverted terminal repeat (3'-ITR) sequence; and
c) a third vector comprising in a 5'-3' direction:
   - a 5'-inverted terminal repeat (5'-ITR) sequence;
   - a nucleic acid sequence of a recombinogenic region;
   - a nucleic acid sequence of a splicing acceptor signal;
   - a third portion of a coding sequence of a gene of interest;
   - a poly-adenylation signal nucleic acid sequence; and
   - a 3'-inverted terminal repeat (3'-ITR) sequence,
      said first and/or second and /or third vector further comprising a nucleic acid sequence of a degradation signal, said sequence being localized at the 5' end or 3' end of the nucleic acid sequence of the recombinogenic region(s)
      wherein
         - the recombinogenic sequence is selected from the group consisting of:
            GGGATTTTGCCGATTTCGGCCTATTGGTTAAAAAATGAGCTGATTTAACAAAAATTT AACGCGAATTTTAACAAAAT(SEQ ID No. 22, AK) or
            GGGATTTTTCCGATTTCGGCCTATTGGTTAAAAAATGAGCTGATTTAACAAAAATTT AACGCGAATTTTAACAAAAT (SEQ 10 NO. 23, AK), SEQ ID NO. 24 (AP1), SEQ ID NO. 25 (AP2) and SEQ ID NO. 26 (AP) and
         - the nucleotide sequence of the degradation signal comprises or consists of a sequence encoding SEQ ID No. 1 (CL1), SEQ ID No. 2 (CL2), SEQ ID No. 3 (CL6), SEQ ID No . 4 (CL9), SEQ ID No. 5 (CL10), SEQ ID No. 6 (CL11), SEQ ID No. 7 (CL12), SEQ ID No. 8 (CL15), SEQ ID No. 9 (CL16), or the nucleotide sequence of a degradation signal comprises or consists of SEQ ID No 16.

Preferably the pathology or disease is selected from: Usher type IF (USH1F), congenital stationary night blindness (CSNB2), autosomal dominant (ad) and/or autosomal recessive (ar) Retinitis Pigmentosa (RP), USH1B, STGD1, Leber Congenital Amaurosis type 10 (LCA10), RP, Usher type 1D (USH1D), Usher type 2A (USH2A), autosomal dominant macular dystrophy, Usher type 2C (USH2C), Occult macular dystrophy, Alstrom Syndrome.

In the present invention the vector system means a construct system, a plasmid system and also viral particles.

In the present invention the construct or vector system may include more than two vectors.

In particular the construct system may include a third vector comprising a third portion of the sequence of interest.

In the present invention the full length coding sequence reconstitutes or is obtained when the various (2, 3 or more) vectors are introduced in the cell.

The coding sequence may be split in two. The portions may be equal or different in length. The full length coding sequence is obtained when the vectors of the vector system are introduced into the cell. The first portion may be the 5' end portion of the coding sequence. The second portion may be the 3'end of the coding sequence. Still, the coding sequence may be split in three portions. The portions may be equal or different in length. The full length coding sequence is obtained when the vectors of the vector system are introduced into the cell. The first portion being the 5' portion of a coding sequence, the second portion being a middle portion of the coding sequence, the third portion being the 3' portion of a coding sequence.

In the present invention the cell is preferably a mammal cell, preferably a human cell.

In the present invention the presence of one degradation signal in any of the vectors is sufficient to decrease the production of the protein in truncated form.

The term degradation signal means a sequence (either nucleotidic or amminoacidic), which can mediate the degradation of the mRNA/protein in which it is included.

The term "protein in truncated form" or a "truncated protein" is a protein which is not produced in its full-length form, since it presents deletions ranging from single to many aminoacids (as an example from 1 to 10, 1 to 20, 1 to 50, 100, 200, ect...).

In the present invention a "reconstitution sequence" is a sequence allowing for the reconstitution of the full length coding sequence with the correct frame, therefore allowing the expression of a functional protein.

The term "splicing donor/acceptor signal" means nucleotidic sequences involved in the splicing of the mRNA.

In the present invention any splicing donor or acceptor signal sequence from any intron may be used. The skilled person knows how to recognizes and select the appropriate splicing donor or acceptor signal sequence by routine experiments.

In the present invention two sequences are overlapping when at least a portion of each of said sequences is homologous one to the other. The sequences may be overlapping for at least 1, at least 2, at least 5, at least 10, at least 20, at least 50, at least 100 , at least 200 nucleotides.

The term "recombinogenic region or sequence" means a sequence which mediates the recombination between two different sequences. "Recombinogenic region or sequence" and "region of homology" are used herein interchangeably.

The term "terminal repeat" means sequences which are repeated at both ends of a nucleotide sequence.

The term "inverted terminal repeat" means sequences which are repeated at both ends of a nucleotide sequence in the opposite orientation (reverse complementary).

A protein ubiquitination signal is a signal that mediates protein degradation by the proteasome. In the present invention when a degradation signal comprises repeated sequences, being the same sequence or different sequences, said repeated sequences are preferably linked by a linker of at least 1 nucleotide.

An artificial stop codon is a nucleotide sequence purposely included in a transcript to induce the premature termination of the translation of a protein.

An enhancer sequence is a sequence that increases the transcription of a gene. Degradation signals may include: (i) the short degron CL1, a C-terminal destabilizing peptide that shares structural similarities with misfolded proteins and is thus recognized by the ubiquitination system ^{31, 32}, (ii) ubiquitin, whose fusion at the N-terminal of a donor protein mediates both direct protein degradation or degradation via the N-end rule pathway ^{33, 34} and (iii) the N-terminal PB29 degron which is a 9 aminoacid-long peptide which, similarly to the CL1 degron, is predicted to fold in structures that are recognized by enzymes of the ubiquitination pathway ³⁵. The inventors have found that inclusion of degradation sequences or signals in multiple vector systems mitigate the expression of truncated proteins. In one instance, the inventors have found that including a CL1 degradation signal results in the selective degradation of truncated proteins from the 5'-half without affecting full-length protein production both *in vitro* and in the large pig retina.

Additionally, artificial stop codons can be inserted to cause the early termination of an mRNA. MicroRNA (miR) target sequences, artificial stop codons or protein ubiquitination signals can be exploited to mediate the degradation of truncated protein products. A degradation signal sequence can comprise repeated sequences, such as more than one microRNA (miR) target sequence, artificial stop codon or protein ubiquitination signal, said repeated sequences being the same sequence or different sequences repeated at least twice; preferably, the repeated sequences are linked by a linker of at least 1 nucleotide.

Among the miR expressed in the retina, miR-let7b or -26a are expressed at high levels ²⁶⁻²⁹ while miR-204 and -124 have been shown to restrict AAV-mediated transgene expression to either RPE or photoreceptors ³⁰. Karali et al³⁰ tested the efficacy of the miR target sites in modulating the expression of a gene included in a single AAV vector in specific cell types. In Karali et al, miR target sites were included in a canonical expression cassette (coding for the entire reporter gene), downstream of a coding sequence and before the polyadenylation signal (polyA). Karali et al used miR target sites for either miR-204 or miR-124 and used 4 tandem copies of each miR. miR may also be miR mimics (Xiao, et al. J Cell Physiol 212:285-292, 2007; Wang Z Methods Mol Biol 676:211-223, 2011*).* For the first time, the inventors applied these strategies to multiple vector constructs and were able to silence the expression of truncated proteins generated from such vectors.

During the past decade, gene therapy has been applied to the treatment of disease in hundreds of clinical trials. Various tools have been developed to deliver genes into human cells. The delivery vehicles may be administered to a patient. A skilled worker would be able to determine appropriate dosage range. The term "administered" includes delivery by viral or non-viral techniques. Non-viral delivery mechanisms include but are not limited to lipid mediated transfection, liposomes, immunoliposomes, lipofectin, cationic facial amphiphiles (CFAs) and combinations thereof. Among viral delivery, genetically engineered viruses, including adeno-associated viruses, are currently amongst the most popular tool for gene delivery. The concept of virus-based gene delivery is to engineer the virus so that it can express the gene(s) of interest or regulatory sequences such as promoters and introns. Depending on the specific application and the type of virus, most viral vectors contain mutations that hamper their ability to replicate freely as wild-type viruses in the host. Viruses from several different families have been modified to generate viral vectors for gene delivery. These viruses include retroviruses, lentiviruses, adenoviruses, adeno-associated viruses, herpes viruses, baculoviruses, picornaviruses, and alphaviruses. The present invention employs adeno-associated viruses. Most of the systems contain vectors that are capable of accommodating genes of interest and helper cells that can provide the viral structural proteins and enzymes to allow for the generation of vector-containing infectious viral particles. Adeno-associated virus is a family of viruses that differs in nucleotide and amino acid sequence, genome structure, pathogenicity, and host range. This diversity provides opportunities to use viruses with different biological characteristics to develop different therapeutic applications. As with any delivery tool, the efficiency, the ability to target certain tissue or cell type, the expression of the gene of interest, and the safety of adeno-associated viral-based systems are important for successful application of gene therapy. Significant efforts have been dedicated to these areas of research in recent years. Various modifications have been made to adeno-associated virus-based vectors and helper cells to alter gene expression, target delivery, improve viral titers, and increase safety. The present invention represents an improvement in this design process in that it acts to efficiently deliver genes of interest into such viral vectors.

An ideal adeno-associated virus-based vector for gene delivery must be efficient, cell-specific, regulated, and safe. The efficiency of delivery is important because it can determine the efficacy of the therapy. Current efforts are aimed at achieving cell-type-specific infection and gene expression with adeno-associated viral vectors. In addition, adeno-associated viral vectors are being developed to regulate the expression of the gene of interest, since the therapy may require long-lasting or regulated expression. Safety is a major issue for viral gene delivery because most viruses are either pathogens or have a pathogenic potential. It is important that during gene delivery, the patient does not also inadvertently receive a pathogenic virus that has full replication potential.

**Adeno-associated virus (AAV)** is a small virus which infects humans and some other primate species. AAV is not currently known to cause disease and consequently the virus causes a very mild immune response. Gene therapy vectors using AAV can infect both dividing and quiescent cells and persist in an extrachromosomal state without integrating into the genome of the host cell. These features make AAV a very attractive candidate for creating viral vectors for gene therapy, and for the creation of isogenic human disease models.

Wild-type AAV has attracted considerable interest from gene therapy researchers due to a number of features. Chief amongst these is the virus's apparent lack of pathogenicity. It can also infect non-dividing cells and has the ability to stably integrate into the host cell genome at a specific site (designated AAVS1) in the human chromosome 19. The feature makes it somewhat more predictable than retroviruses, which present the threat of a random insertion and of mutagenesis, which is sometimes followed by development of a cancer. The AAV genome integrates most frequently into the site mentioned, while random incorporations into the genome take place with a negligible frequency. Development of AAVs as gene therapy vectors, however, has eliminated this integrative capacity by removal of the *rep* and *cap* from the DNA of the vector. The desired gene together with a promoter to drive transcription of the gene is inserted between the ITRs that aid in concatamer formation in the nucleus after the single-stranded vector DNA is converted by host cell DNA polymerase complexes into double-stranded DNA. AAV-based gene therapy vectors form episomal concatamers in the host cell nucleus. In non-dividing cells, these concatemers remain intact for the life of the host cell. In dividing cells, AAV DNA is lost through cell division, since the episomal DNA is not replicated along with the host cell DNA. Random integration of AAV DNA into the host genome is detectable but occurs at very low frequency. AAVs also present very low immunogenicity, seemingly restricted to generation of neutralizing antibodies, while they induce no clearly defined cytotoxic response. This feature, along with the ability to infect quiescent cells present their dominance over adenoviruses as vectors for the human gene therapy.

### AAV genome, transcriptome and proteome

The AAV genome is built of single-stranded deoxyribonucleic acid (ssDNA), either positive- or negative-sensed, which is about 4.7 kilobase long. The genome comprises inverted terminal repeats (ITRs) at both ends of the DNA strand, and two open reading frames (ORFs): *rep* and *cap.* The former is composed of four overlapping genes encoding Rep proteins required for the AAV life cycle, and the latter contains overlapping nucleotide sequences of capsid proteins: VP1, VP2 and VP3, which interact together to form a capsid of an icosahedral symmetry.

### ITR sequences

The Inverted Terminal Repeat (ITR) sequences received their name because of their symmetry, which was shown to be required for efficient multiplication of the AAV genome. Another property of these sequences is their ability to form a hairpin, which contributes to so-called self-priming that allows primase-independent synthesis of the second DNA strand. The ITRs were also shown to be required for both integration of the AAV DNA into the host cell genome (19th chromosome in humans) and rescue from it, as well as for efficient encapsidation of the AAV DNA combined with generation of a fully assembled, deoxyribonuclease-resistant AAV particles.

With regard to gene therapy, ITRs seem to be the only sequences required *in cis* next to the therapeutic gene: structural (*cap*) and packaging (*rep*) genes can be delivered *in trans.* With this assumption many methods were established for efficient production of recombinant AAV (rAAV) vectors containing a reporter or therapeutic gene. However, it was also published that the ITRs are not the only elements required *in cis* for the effective replication and encapsidation.

A few research groups have identified a sequence designated *cis-acting Rep-dependent element* (CARE) inside the coding sequence of the *rep* gene. CARE was shown to augment the replication and encapsidation when present *in cis.*

As of 2006 there have been 11 AAV serotypes described, the 11th in 2004. All of the known serotypes can infect cells from multiple diverse tissue types. Tissue specificity is determined by the capsid serotype and pseudotyping of AAV vectors to alter their tropism range will likely be important to their use in therapy.

The inverted terminal repeat (ITR) sequences used in an AAV vector system of the present invention can be any AAV ITR. The ITRs used in an AAV vector can be the same or different. For example, a vector may comprise an ITR of AAV serotype 2 and an ITR of AAV serotype 5. In one embodiment of a vector of the invention, an ITR is from AAV serotype 2, 4, 5, or 8. In the present invention ITRs of AVV serotype 2 and serotype 5 are preferred. AAV ITR sequences are well known in the art (for example, see for ITR2, GenBank Accession Nos. AF043303.1 ; NC_001401.2; J01901.1 ; JN898962.1; see for ITR5, GenBank Accession No. NC_006152.1).

### Serotype 2

Serotype 2 (AAV2) has been the most extensively examined so far. AAV2 presents natural tropism towards skeletal muscles, neurons, vascular smooth muscle cells and hepatocytes.

Three cell receptors have been described for AAV2: heparan sulfate proteoglycan (HSPG), aᵥβ₅ integrin and fibroblast growth factor receptor 1 (FGFR-1). The first functions as a primary receptor, while the latter two have a co-receptor activity and enable AAV to enter the cell by receptor-mediated endocytosis. These study results have been disputed by Qiu, Handa, *et al..* HSPG functions as the primary receptor, though its abundance in the extracellular matrix can scavenge AAV particles and impair the infection efficiency.

### Serotype 2 and cancer

Studies have shown that serotype 2 of the virus (AAV-2) apparently kills cancer cells without harming healthy ones. "Our results suggest that adeno-associated virus type 2, which infects the majority of the population but has no known ill effects, kills multiple types of cancer cells yet has no effect on healthy cells," said Craig Meyers, a professor of immunology and microbiology at the Penn State College of Medicine in Pennsylvania. This could lead to a new anti-cancer agent.

### Other Serotypes

Although AAV2 is the most popular serotype in various AAV-based research, it has been shown that other serotypes can be more effective as gene delivery vectors. For instance AAV6 appears much better in infecting airway epithelial cells, AAV7 presents very high transduction rate of murine skeletal muscle cells (similarly to AAV1 and AAV5), AAV8 is superb in transducing hepatocytes and photoreceptors and AAV1 and 5 were shown to be very efficient in gene delivery to vascular endothelial cells. In the brain, most AAV serotypes show neuronal tropism, while AAV5 also transduces astrocytes. AAV6, a hybrid of AAV1 and AAV2, also shows lower immunogenicity than AAV2.

Serotypes can differ with the respect to the receptors they are bound to. For example AAV4 and AAV5 transduction can be inhibited by soluble sialic acids (of different form for each of these serotypes), and AAV5 was shown to enter cells via the platelet-derived growth factor receptor. The subject invention also concerns a viral vector system comprising a polynucleotide, expression construct, or vector construct of the invention. In one embodiment, the viral vector system is an AAV system. Methods for preparing viruses and virions comprising a heterologous polynucleotide or construct are known in the art. In the case of AAV, cells can be coinfected or transfected with adenovirus or polynucleotide constructs comprising adenovirus genes suitable for AAV helper function. Examples of materials and methods are described, for example, in U.S. Patent Nos. 8,137,962 and 6,967,018. An AAV virus or AAV vector of the invention can be of any AAV serotype, including, but not limited to, serotype AAV1 , AAV2, AAV3, AAV4, AAV5, AAV6, AAV7, AAV8, AAV9, AAV10, and AAV11. In a specific embodiment, an AAV2 or an AAV5 or an AAV7 or an AAV8 or an AAV9 serotype is utilized. In one embodiment, the AAV serotype provides for one or more tyrosine to phenylalanine (Y-F) mutations on the capsid surface. In a specific embodiment, the AAV is an AAV8 serotype having a tyrosine to phenylalanine mutation at position 733 (Y733F).

The delivery of one or more therapeutic genes or regulatory sequences such as promoters or introns by a vector system according to the present invention may be used alone or in combination with other treatments or components of the treatment.

The subject invention also concerns a host cell comprising the construct system or the viral vector system of the invention. The host cell can be a cultured cell or a primary cell, i.e., isolated directly from an organism, e.g., a human. The host cell can be an adherent cell or a suspended cell, i.e., a cell that grows in suspension. Suitable host cells are known in the art and include, for instance, DH5α, E. coli cells, Chinese hamster ovarian cells, monkey VERO cells, COS cells, HEK293 cells, and the like. The cell can be a human cell or from another animal. In one embodiment, the cell is a photoreceptor cell or an RPE cell. In a specific embodiment, the cell is a cone cell. The cell may also be a muscle cell, in particular a skeletal muscle cell, a lung cell, a pancreas cell, a liver cell, a kidney cell, an intestine cell, a blood cell. In a specific embodiment, the cell is a human cone cell or rod cell. The selection of an appropriate host is deemed to be within the scope of those skilled in the art from the teachings herein. Preferably, said host cell is an animal cell, and most preferably a human cell. The cell can express a nucleotide sequence provided in the viral vector system of the invention.

The man skilled in the art is well aware of the standard methods for incorporation of a polynucleotide or vector into a host cell, for example transfection, lipofection, electroporation, microinjection, viral infection, thermal shock, transformation after chemical permeabilisation of the membrane or cell fusion. The construct or vector system of the invention can also be introduced in vivo as naked DNA using methods known in the art, such as transfection, microinjection, electroporation, calcium phosphate precipitation, and by biolistic methods.

As used herein, the term "host cell or host cell genetically engineered" relates to host cells which have been transduced, transformed or transfected with the construct system or with the viral vector system of the invention

As used herein, the terms "nucleic acid" and "polynucleotide sequence" and "construct" refer to a deoxyribonucleotide or ribonucleotide polymer in either single- or double-stranded form.

The subject invention also concerns a construct system that can include regulatory elements that are functional in the intended host cell in which the construct is to be expressed. A person of ordinary skill in the art can select regulatory elements for use in appropriate host cells, for example, mammalian or human host cells. Regulatory elements include, for example, promoters, transcription termination sequences, translation termination sequences, enhancers, signal peptides, degradation signals and polyadenylation elements. A construct of the invention can comprise a promoter sequence operably linked to a nucleotide sequence encoding a desired polypeptide.

Promoters contemplated for use in the subject invention include, but are not limited to, native gene promoters, cytomegalovirus (CMV) promoter (KF853603.1, bp 149-735), chimeric CMV/chicken beta-actin promoter (CBA) and the truncated form of CBA (smCBA) promoter (US8298818 and Light-Driven Cone Arrestin Translocation in Cones of Postnatal Guanylate Cyclase-1 Knockout Mouse Retina Treated with AAVGC1), Rhodopsin promoter (NG_009115, bp 4205-5010), Interphotoreceptor retinoid binding protein promoter (NG_029718.1, bp 4777-5011), vitelliform macular dystrophy 2 promoter (NG_009033.1, bp 4870-5470), PR-specific human G protein-coupled receptor kinase 1 (hGRK1; AY327580.1 bp1793-2087 or bp 1793-1991) (Haire et al. 2006; U.S. Patent No. 8,298,818). However any suitable promoter known in the art may be used. In a specific embodiment, the promoter is a CMV or hGRK1 promoter. In one embodiment, the promoter is a tissue- specific promoter that shows selective activity in one or a group of tissues but is less active or not active in other tissue. In one embodiment, the promoter is a photoreceptor- specific promoter. In a further embodiment, the promoter is a cone cell-specific and/or rod cell-specific promoter.

Preferred promoters are CMV, GRK1, CBA and IRBP promoters. Still preferred promoters are hybrid promoter which combine regulatory elements from various promoters (as example the chimeric CBA promoter which combines an enhancer from the CMV promoter, the CBA promoter and the Sv40 chimeric intron, herein called CBA hybrid promoter.

Promoters can be incorporated into a construct using standard techniques known in the art. Multiple copies of promoters or multiple promoters can be used in a vector of the invention. In one embodiment, the promoter can be positioned about the same distance from the transcription start site as it is from the transcription start site in its natural genetic environment. Some variation in this distance is permitted without substantial decrease in promoter activity. In the system of the invention a transcription start site is typically included in the 5' construct but not in the 3' construct. In further embodiment a transcription start site may be included in the 3'construct upstream of the degradation signal.

A construct of the invention may optionally contain a transcription termination sequence, a translation termination sequence, signal peptide sequence, internal ribosome entry sites (IRES), enhancer elements, and/or post-trascriptional regulatory elements such as the Woodchuck hepatitis virus (WHV) posttranscriptional regulatory element (WPRE). Transcription termination regions can typically be obtained from the 3' untranslated region of a eukaryotic or viral gene sequence. Transcription termination sequences can be positioned downstream of a coding sequence to provide for efficient termination. In the system of the invention a transcription termination site is typically included in the 3' construct but not in the 5' construct.

Signal peptide sequence is an amino terminal sequence that encodes information responsible for the relocation of an operably linked polypeptide to a wide range of post-translational cellular destinations, ranging from a specific organelle compartment to sites of protein action and the extracellular environment. Enhancers are cis-acting elements that increase gene transcription and can also be included in a vector. Enhancer elements are known in the art, and include, but are not limited to, the CaMV 35S enhancer element, cytomegalovirus (CMV) early promoter enhancer element, and the SV40 enhancer element. DNA sequences which direct polyadenylation of the mRNA encoded by the structural gene can also be included in a vector. Preferably, in the present invention, the coding sequence is split into a first and a second fragment or portion (5' end portion and 3' end portion) at a natural exon-exon junction. Preferably each fragment or portion of the coding sequence should not exceed a size of 60 kb, preferably each fragment or portion of the coding sequence should not exceed a size of 50 Kb, 40 Kb, 30 Kb, 20 Kb, 10 Kb . Preferably each fragment or portion of the coding sequence may have a size of about 2Kb, 2.5Kb, 3Kb, 3.5Kb, 4Kb, 4.5Kb, 5Kb, 5.5 Kb, 6Kb, 6.5 Kb, 7kb, 7.5 Kb, 8 Kb, 8.5 Kb, 9Kb, 9.5 Kb or a smaller size.

Spliceosomal introns often reside within the sequence of eukaryotic protein-coding genes. Within the intron, a donor site (5' end of the intron), a branch site (near the 3' end of the intron) and an acceptor site (3' end of the intron) are required for splicing. The splice donor site includes an almost invariant sequence GU at the 5' end of the intron, within a larger, less highly conserved region. The splice acceptor site at the 3' end of the intron terminates the intron with an almost invariant AG sequence. Upstream (5'-ward) from the AG there is a region high in pyrimidines (C and U), or polypyrimidine tract. Upstream from the polypyrimidine tract is the branchpoint, which includes an adenine nucleotide. The spicing acceptor signal and the splicing donor signal may also be chosen by the skilled person in the art among sequences known in the art.

Signals that mediate the degradation of proteins and that have never been used before in the context of a multiple viral system include but are not limited to: short degrons as CL1, CL2, CL6, CL9, CL10, CL11, CL12, CL15, CL16, SL17, a C-terminal destabilizing peptide that shares structural similarities with misfolded proteins and is thus recognized by the ubiquitination system, ubiquitin, whose fusion at the N-terminal of a donor protein mediates both direct protein degradation or degradation via the N-end rule pathway, the N-terminal PB29 degron which is a 9 aminoacid-long peptide which, similarly to the CL1 degron, is predicted to fold in structures that are recognized by enzymes of the ubiquitination pathway, artificial stop codons that cause the early termination of an mRNA, microRNA (miR) target sequences.

As those skilled in the art can readily appreciate, there can be a number of variant sequences of a protein found in nature, in addition to those variants that can be artificially created by the skilled artisan in the lab. disclosure further includes nucleotide sequences which encode the polypeptides disclosed herein. These nucleotide sequences can be readily constructed by those skilled in the art having the knowledge of the protein and amino acid sequences which are presented herein. As would be appreciated by one skilled in the art, the degeneracy of the genetic code enables the artisan to construct a variety of nucleotide sequences that encode a particular polypeptide or protein. The choice of a particular nucleotide sequence could depend, for example, upon the codon usage of a particular expression system or host cell. For example, non-natural amino acids can be substituted for the amino acids of a polypeptide so long as the polypeptide having substituted amino acids retains substantially the same activity as the polypeptide in which amino acids have not been substituted. Examples of non-natural amino acids include, but are not limited to, ornithine, citrulline, hydroxyproline, homoserine, phenylglycine, taurine, iodotyrosine, 2,4-diaminobutyric acid, a-amino isobutyric acid, 4-aminobutyric acid, 2- amino butyric acid, γ-amino butyric acid, ε-amino hexanoic acid, 6-amino hexanoic acid, 2-amino isobutyiic acid, 3 - amino propionic acid, norleucine, norvaline, sarcosine, homocitrulline, cysteic acid, τ-butylglycine, τ-butylalanine, phenylglycine, cyclohexylalanine, β-alanine, fluoro-amino acids, designer amino acids such as β-methyl amino acids, C-methyl amino acids, N-methyl amino acids, and amino acid analogues in general. Non-natural amino acids also include amino acids having derivatized side groups. Furthermore, any of the amino acids in the protein can be of the D (dextrorotary) form or L (levorotary) form. Amino acids can be generally categorized in the following classes: non-polar, uncharged polar, basic, and acidic. Conservative substitutions whereby a polypeptide having an amino acid of one class is replaced with another amino acid of the same class are disclosed herein of the so long as the polypeptide having the substitution still retains substantially the same biological activity as a polypeptide that does not have the substitution. Table 1 provides a listing of examples of amino acids belonging to each class.

| Table 1. | |
|---|---|
| Class of Amino Acid | Examples of Amino Acids |
| Nonpolar | Ala, Val, Leu, Ile, Pro, Met. Phe, Trp |
| Uncharged Polar | Gly, Ser, Thr, Cys, Tyr, Asn, Gln |
| Acidic | Asp, Glu |
| Basic | Lys, Arg. His |

Polynucleotides described herein can also be defined in terms of more particular identity and/or similarity ranges with those exemplified herein. The sequence identity will typically be greater than 60%, preferably greater than 75%, more preferably greater than 80%, even more preferably greater than 90%, and can be greater than 95%. The identity and/or similarity of a sequence can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 , 92, 93, 94, 95, 96, 97, 98, or 99% or greater as compared to a sequence exemplified herein. Unless otherwise specified, as used herein percent sequence identity and/or similarity of two sequences can be determined using the algorithm of Karlin and Altschul (1990), modified as in Karlin and Altschul (1993). Such an algorithm is incorporated into the NBLAST and XBLAST programs of Altschul et al. (1990). BLAST searches can be performed with the NBLAST program, score = 100, wordlength = 12, to obtain sequences with the desired percent sequence identity. To obtain gapped alignments for comparison purposes, Gapped BLAST can be used as described in Altschul et al. (1997). When utilizing BLAST and Gapped BLAST programs, the default parameters of the respective programs (NBLAST and XBLAST) can be used. See NCBI/N1H website.

The present invention also concerns pharmaceutical compositions comprising the vector system or the viral vector system or the host cells of the invention optionally in combination with a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system). The construct or vector can be administered in vivo or ex vivo.

Pharmaceutical compositions adapted for topical or parenteral administration, comprising an amount of a compound, constitute a preferred embodiment of the invention. For parenteral administration, the compositions may be best used in the form of a sterile aqueous solution which may contain other substances, for example enough salts or monosaccharides to make the solution isotonic with blood. The pharmaceutical composition of the present invention may be delivered to the retina preferentially via the subretinal injection or it can also be prepared in the form of injectable suspension, eye lotion or ophthalmic ointment that can be delivered to the retina with a non-invasive procedure.

The dose administered to a patient, particularly a human, in the context of the present invention should be sufficient to achieve a therapeutic response in the patient over a reasonable time frame, without lethal toxicity, and preferably causing no more than an acceptable level of side effects or morbidity. One skilled in the art will recognize that dosage will depend upon a variety of factors including the condition (health) of the subject, the body weight of the subject, kind of concurrent treatment, if any, frequency of treatment, therapeutic ratio, as well as the severity and stage of the pathological condition.

The methods described herein can be used with humans and other animals. As used herein, the terms "patient" and "subject" are used interchangeably and are intended to include such human and non-human species. Likewise, in vitro methods can be earned out on cells of such human and non- human species.

The disclosure also concerns kits comprising the construct system or viral vector system or the host cells of the invention in one or more containers. Kits can optionally include pharmaceutically acceptable carriers and/or diluents. In one embodiment, a kit includes one or more other components, adjuncts, or adjuvants as described herein. In one embodiment, a kit includes instructions or packaging materials that describe how to administer a vector system of the kit. Containers of the kit can be of any suitable material, e.g., glass, plastic, metal, etc., and of any suitable size, shape, or configuration. In one embodiment, the construct system or viral vector system or the host cells of the invention is provided in the kit as a solid. In another embodiment, the construct system or viral vector system or the host cells of the invention is provided in the kit as a liquid or solution. In one embodiment, the kit comprises an ampoule or syringe containing the construct system or viral vector system or the host cells of the invention in liquid or solution form.

The present invention also provides a pharmaceutical composition for treating an individual by gene therapy, wherein the composition comprises a therapeutically effective amount of the vector system or viral vector system or host cell of the present invention comprising one or more deliverable therapeutic and/or diagnostic transgenes(s) or a viral particle produced by or obtained from same. The pharmaceutical composition may be for human or animal usage. Typically, an ordinary skilled clinician will determine the actual dosage which will be most suitable for an individual subject and it will vary with the age, weight and response of the particular individual and administration route. A dose range between 1x10e10 and 1×10e15 genome copies of each vector/kg, preferentially between 1x10e11 and 1x10e13 genome copies of each vector/kg are expected to be effective in humans. A dose range between 1x10e10 and 1x10e15 genome copies of each vector/eye, preferentially between 1x10e10 and 1x10e13 are expected to be effective for ocular administration.

Dosage regimes and effective amounts to be administered can be determined by ordinarily skilled clinicians. Administration may be in the form of a single dose or multiple doses. General methods for performing gene therapy using polynucleotides, expression constructs, and vectors are known in the art (see, for example, Gene Therapy: Principles and Applications, Springer Verlag 1999; and U.S. Patent Nos. 6,461 ,606; 6,204,251 and 6,106,826). The 2. disclosure also concerns methods for expressing a selected polypeptide in a cell. In one embodiment, the method comprises incorporating in the cell the vector system of the invention that comprises polynucleotide sequences encoding the selected polypeptide and expressing the polynucleotide sequences in the cell. The selected polypeptide can be one that is heterologous to the cell. In one embodiment, the cell is a mammalian cell. In one embodiment, the cell is a human cell. In one embodiment, the cell is a photoreceptor cell or an RPE cell. The cell may also be a muscle cell, in particular a skeletal muscle cell, a lung cell, a pancreas cell, a liver cell, a kidney cell, an intestine cell, a blood cell. In a specific embodiment, the cell is a cone cell or a rod cell. In a specific embodiment, the cell is a human cone cell or rod cell.

### SEQUENCES

**AP1** (SEQ ID No. 24)
**AP2** (SEQ ID No. 25)
**AK seqA** (SEQ ID No. 22)
   AK seqB (SEQ ID No. 23)
**AP** (SEQ ID No. 26)
**Left ITR2** (SEQ ID No. 29)
**Right ITR2** (SEQ ID No. 30)
Left **ITR5** (SEQ ID No. 31)
**Right ITR5** (SEQ ID No. 32)
**CMV**
***CMV enhancer*** (SEQ ID No. 33)
***CMV promoter*** (SEQ ID No. 34)
***Chimeric intron (SV40 intron)*** (SEQ ID No. 35)
**hGRK1 promoter** (SEQ ID No. 36)
**CBA hybrid promoter**
   *CMV enhancer* (SEQ ID No. 37)
   *CBA promoter* (SEQ ID No. 38)
**IRBP** (SEQ ID No. 39)
**Splicing donor signal** (SEQ ID No. 27)
**miR-let 7b degradation signal** (SEQ ID No. 40)
**4xmiR-let 7b degradation signal** (SEQ ID No. 41)
**miR-26a degradation signal** (SEQ ID No. 13)
**4xmiR-26a degradation signal** (SEQ ID No. 18)
**miR-204** degradation signal (SEQ ID No. 11)
**miR-124 degradation signal** (SEQ ID No. 12)
**3xmiR-204+3xmiR-124 degradation signal** (SEQ ID No. 17)
**CL1 degradation signal (degron)**
   Nucleotidic sequence: (SEQ ID No. 16)
   Aminoacidic sequence: (SEQ ID No. 1)
**CL2 degradation signal (degron)**
   Nucleotidic sequence: (SEQ ID No. 42)
   Aminoacidic sequence: (SEQ ID No. 2)
**CL6 degradation signal (degron)**
   Nucleotidic sequence: (SEQ ID No. 43)
   Aminoacidic sequence: (SEQ ID No. 3)
**CL9 degradation signal (degron)**
   Nucleotidic sequence: (SEQ ID No. 44)
   Aminoacidic sequence: (SEQ ID No. 4)
**CL10 degradation signal (degron)**
   Nucleotidic sequence: (SEQ ID No. 45)
   Aminoacidic sequence: (SEQ ID No. 5)
**CL11 degradation signal (degron)**
   Nucleotidic sequence: (SEQ ID No. 46)
   Aminoacidic sequence: (SEQ ID No. 6)
**CL12 degradation signal (degron)**
   Nucleotidic sequence: (SEQ ID No. 47)
   Aminoacidic sequence: (SEQ ID No. 7)
**CL15 degradation signal (degron)**
   Nucleotidic sequence: (SEQ ID No. 48)
   Aminoacidic sequence: (SEQ ID No. 8)
**CL16 degradation signal (degron)**
   Nucleotidic sequence: (SEQ ID No. 49)
   Aminoacidic sequence: (SEQ ID No. 9)
**SL17 degradation signal (degron)**
   Nucleotidic sequence: (SEQ ID No. 50)
   Aminoacidic sequence: (SEQ ID No. 10)
**PB29 degradation signal (degron)**
   Nucleotidic sequence: (SEQ ID No. 19)
   Aminoacidic sequence: (SEQ ID No. 15)
**Short PB29 degradation signal (degron)**
   Nucleotidic sequence: (SEQ ID No. 20)
   Aminoacidic sequence: (SEQ ID No. 14)
**3x PB29 degradation signal (degron)** (SEQ ID No. 21)
**Artificial Stop codons** (SEQ ID No. 51)
**Splicing acceptor signal** (SEQ ID No. 28)
**SV40 Poly A** (SEQ ID No. 52)
**ABCA4 5'** (SEQ ID No. 53)
   **hGRK1-5' ABCA4+AK+CL1 Full length sequence** (SEQ ID No. 54) Legend:
   ITR: uppercases bold
   hGRK promoter: lowercases bold italic
   ABCA4 5': lowercase underlined
   SDS: lowercase bold
   AK: uppercase
   CL1: lowercase italic underlined
**Abca4_3'** (SEQ ID No. 55)
   **ABCA4 3'+AK_SV40 Full length sequence** (SEQ ID No. 56) Legend:
   ITR: uppercases bold underlined
   AK: uppercase
   SAS: lowercase bold
   ABCA4 3': lowercase underlined
   SV40 polyA: lowercases bold italic
**CMV 5' ABCA4-SD-AK Full length sequence** (SEQ ID No. 57)
**AK-SA-3' ABCA4-3XFLAG-SV40 Full length sequence** (SEQ ID No. 58).
**CMV 5' ABCA4-SD-AP1 Full length sequence** (SEQ ID No. 59)
**AP1-SA-3' ABCA4-3XFLAG-SV40 Full length sequence** (SEQ ID No. 60)
**CMV 5' ABCA4-SD-AP2 Full length sequence** (SEQ ID No. 61)
**AP2-SA-3' ABCA4-3XFLAG-SV40 Full length sequence** (SEQ ID No. 62)
**CMV 5' ABCA4-SD-AP Full length sequence** (SEQ ID No. 63)
**AP-SA-3' ABCA4-3XFLAG-SV40 Full length sequence** (SEQ ID No. 64)
**hGRK1 5' ABCA4-SD-AP1 Full length sequence** (SEQ ID No. 65)
**GRK1 5' ABCA4-SD-AP2 Full length sequence** (SEQ ID No. 66)
**ITR5-CMV 5' ABCA4-SD-AK-ITR2 Full length sequence** (SEQ ID No. 67)
**ITR2-AK-SA-3' ABCA4-SV40-ITR5 Full length sequence** (SEQ ID No. 68)
**ITR5-CBA 5' MYO7A-SD-AK-ITR2 Full length sequence** (SEQ ID No. 69)
**ITR2-AK-SA-3' MYO7A-HA-BGH-ITR5 Full length sequence** (SEQ ID No. 70)
**CMV 5' ABCA4-3XFLAG-SD-AK-4xmiR26a Full length sequence** (SEQ ID No. 71)
**CMV 5' ABCA4-3XFLAG-SD-AK-3xmiR204+3xmir124 Full length sequence** (SEQ ID No.72)
**CMV 5' ABCA4-3XFLAG-SD-AK-CL1 Full length sequence** (SEQ ID No. 73)
**AK-STOP-SA-3' ABCA4-3XFLAG-SV40 Full length sequence** (SEQ ID No. 74)
**AK-PB29-SA-3' ABCA4-3XFLAG-SV40 Full length sequence** (SEQ ID No. 75)
**AK-3XPB29-SA-3' ABCA4-3XFLAG-SV40 Full length sequence** (SEQ ID No. 76)
**AK-UBIQUITIN-SA-3' ABCA4-3XFLAG-SV40 Full length sequence** (SEQ ID No. 77).

The present invention will now be illustrated by means of non-limiting examples in reference to the following drawings.

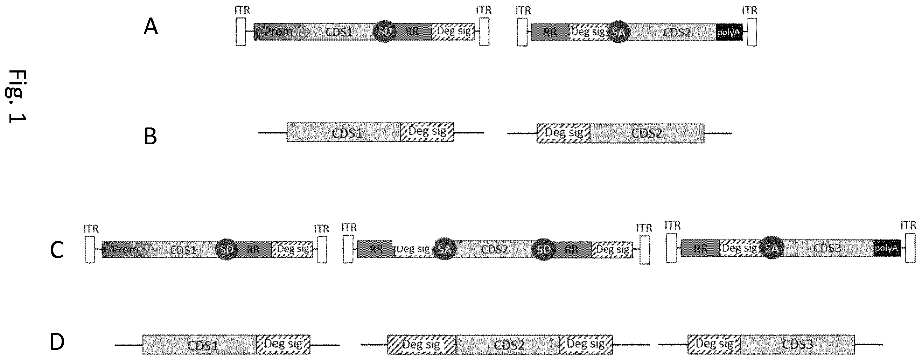

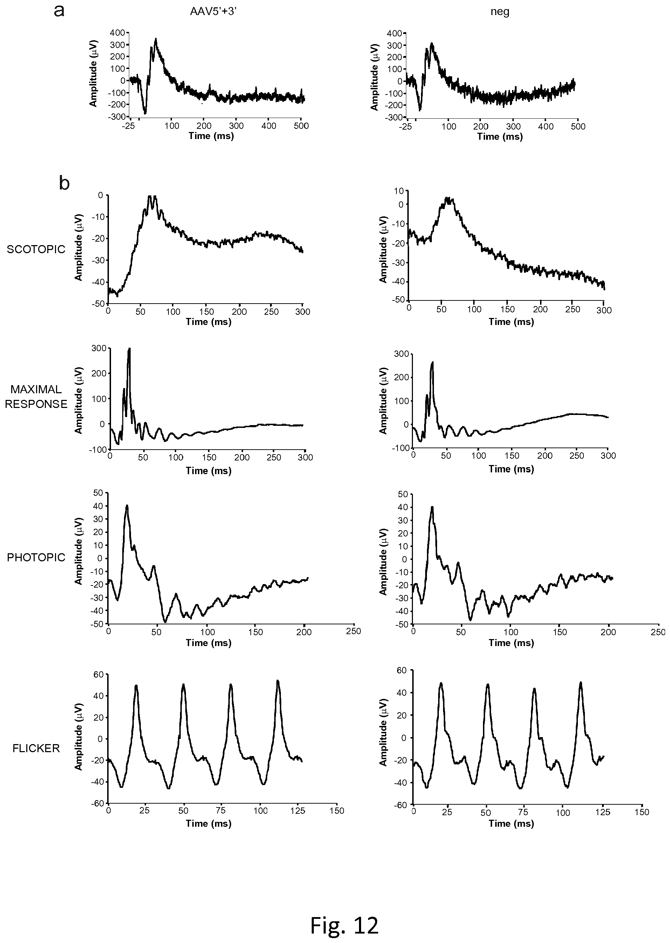

*Figure 1**. Schematic representation of multiple-vector strategies of present invention examples.* ITR: inverted terminal repeats; Prom: promoter; CDS, coding sequence; SD, splicing donor signal; RR: recombinogenic regions, AK or from alkaline phosphatase (AP1, AP2 and AP); Deg Sig; degradation signals (see Table 2); SA, splicing acceptor signal; pA, polyadenylation signal. A and C: (dual or triple) hybrid vectors strategy, including transplicing and recombinogenic regions, according to a preferred embodiment of the invention B and D: (dual or triple) vectors overlapping vectors strategy. For additional examples, see Figs. 12-14.

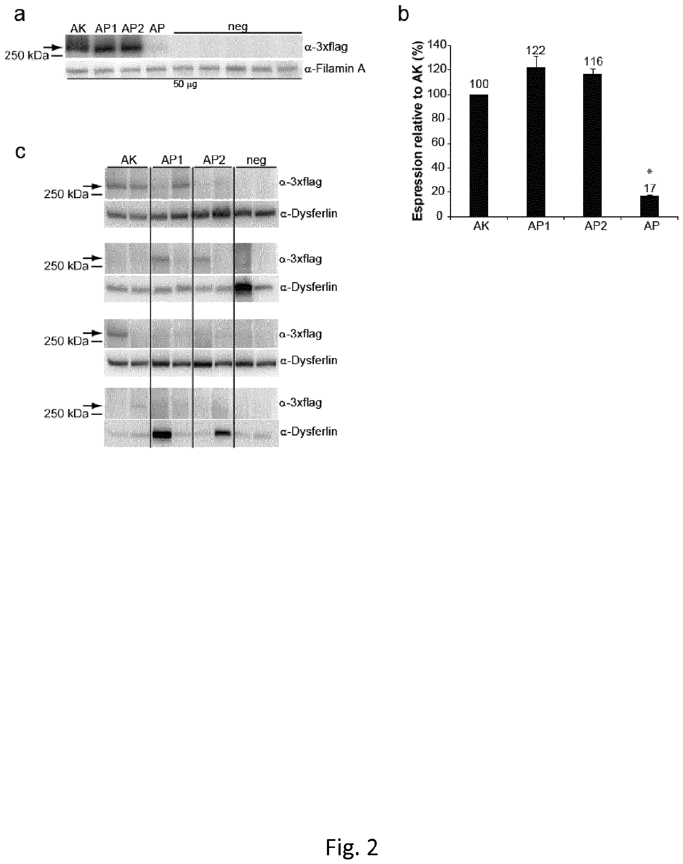

*Figure 2**. Efficient ABCA4 protein expression using the AK, AP1 and AP2 regions of homology* (a, c) Representative Western blot analysis of (a) HEK293 cells (50 micrograms/lane) infected with dual AAV2/2 (AAV serotype 2, with homologous ITR from AAV2) vectors or (c) C57BL/6 retinas (whole retinal lysates) injected with dual AAV2/8 (AAV serotype 8, with homologous ITR from AAV2) vectors encoding for *ABCA4.* The arrows indicate full-length proteins, the molecular weight ladder is depicted on the left. (b) Quantification of ABCA4 protein bands from Western blot analysis in (a). The intensity of the ABCA4 bands in (a) was divided by the intensity of the Filamin A bands. The histograms show the expression of proteins as a percentage relative to dual AAV hybrid AK vectors, the mean value is depicted above the corresponding bar. Values are represented as: mean ± s.e.m. (standard error of the mean). ^{∗}pANOVA < 0.05; the asterisk indicate significant differences with AK, AP1 and AP2. (a-c) AK: cells infected or eyes injected with dual AAV hybrid AK vectors; AP1: cells infected or eyes injected with dual AAV hybrid AP1 vectors; AP2: cells infected or eyes injected with dual AAV hybrid AP2 vectors; AP: cells infected with dual AAV hybrid AP vectors; neg: cells infected or eyes injected with either the 3'-half vectors or EGFP expressing vectors, as negative controls. α-3xflag: Western blot with anti-3xflag antibodies; α-Filamin A, Western blot with anti-Filamin A antibodies, used as loading control; α-Dysferlin, Western blot with anti-Dysferlin antibodies, used as loading control.

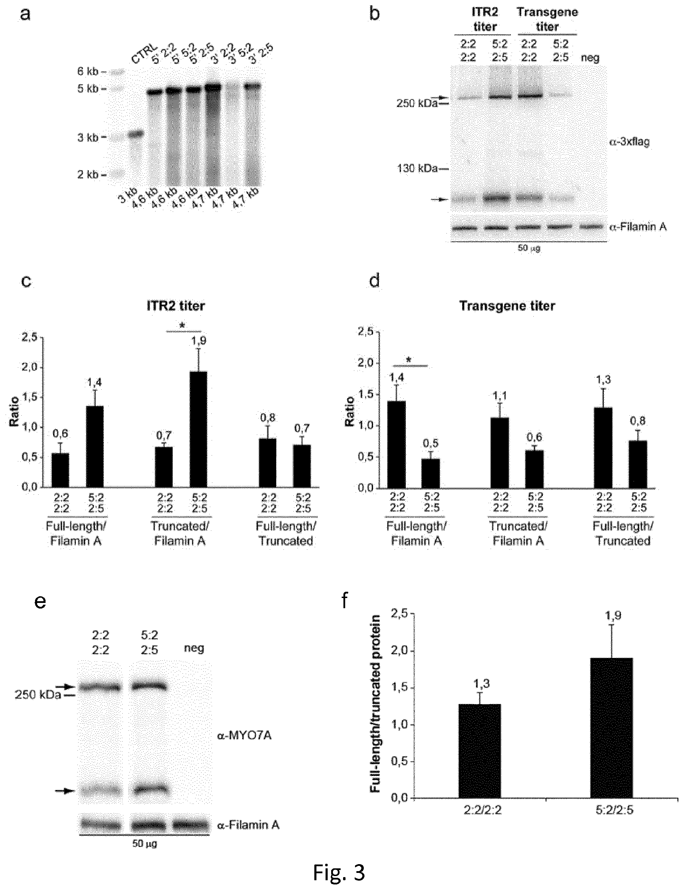

*Figure 3**. Genome and transduction efficiency of vectors with heterologous ITR2 and ITR5.* (a) Alkaline Southern blot analysis of DNA extracted from 3 × 1010 GC of both 5'- and 3'-ABCA4-half vectors with either homologous (2:2) or heterologous (5:2 or 2:5) ITR, and of a control AAV preparation with homologous ITR2 (CTRL). The expected size of each genome is depicted beloweach lane. The molecular weight marker (kb) is depicted on the left 5': 5'-half vector; 3': 3'-half vector, (b-d) Representative Western blot analysis and quantification of HEK293 cells infected with dual AAV2/2 hybrid ABCA4 vectors with either heterologous ITR2 and ITR5 or homologous ITR2 at m.o.i. based on either the ITR2 (b and c) or the transgene (b and d) titre. The Western blot images (b) are representative of n = 3 independent experiments; the quantifications (c and d) are from n = 3 independent experiments. (b) The upper arrow indicates full-length ABCA4 protein, the lower arrow indicates truncated proteins; the molecular weight ladder is depicted on the left. The micrograms of proteins loaded are depicted below the image. α-3×flag: Western blot with anti-3×flag antibodies; α-Filamin A: Western blot with anti-Filamin A antibodies, used as loading control. (c and d) Quantification of full-length and truncated ABCA4 protein bands from Western blot analysis of cells infected with a dose of vector based on either the ITR2 (c) or the transgene (d) titre. The histograms show either the intensity of the full-length and truncated protein bands divided by that of the Filamin A bands or the intensity of the full-length protein bands divided by that of the truncated protein bands in the corresponding lane. Representative Western blot analysis and quantification of HEK293 cells infected with dual AAV2 (AAV serotype 2) hybrid vectors with either heterologous ITR2 and ITR5 or homologous ITR2 encoding for *MYO7A* (e, f). the Western blot images (e) are representative of and the quantifications (f) are from n=3 independent experiments. (e) The upper arrows indicate full-length proteins, the lower arrows indicate truncated proteins, the molecular weight ladder is depicted on the left. The micrograms of proteins loaded are depicted below the image. (f) Quantification of MYO7A protein bands from Western blot analysis.

The mean value is depicted above the corresponding bar. Values are represented as: mean ± s.e.m. ^{∗}p Student's t test ≤ 0.05.

2:2 2:2: cells infected with dual AAV hybrid vectors with homologous ITR from AAV2; 5:2 2:5: cells infected with dual AAV hybrid vectors with heterologous ITR from AAV2 and AAV5; neg: cells infected with EGFP-expressing vectors, as negative controls.

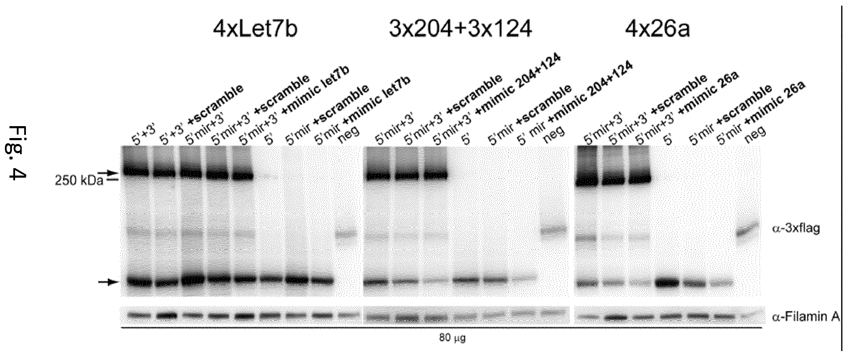

*Figure 4**. Inclusion of miR target sites in the 5 '-half vectors does not result in significant reduction of truncated protein products*
Representative Western blot analysis of HEK293 cells infected with dual AAV2/2 (AAV serotype 2) hybrid vectors encoding for *ABCA4,* containing miR target sites for either miR-let7b (left panel), miR-204+124 (central panel) or miR-26a (right panel). The upper arrow indicates full-length ABCA4 proteins, the lower arrow indicates truncated proteins; the molecular weight ladder is depicted on the left. The micrograms of proteins loaded are depicted below the image. 5'+3': cells co-infected with 5'-half vectors without miR target sites and 3'-half vectors; 5'+3'+scrumble: cells co-infected with 5'-half vectors without miR target sites and 3'-half vectors in the presence of scramble miR mimics; 5'mir+3': cells co-infected with 5*'-half* vectors containing miR target sites and 3'-half vectors; 5'mir+3'+ scramble: cells co-infected with 5'-half vectors containing miR target sites and 3'-half vectors in the presence of scramble miR mimics; 5'mir+3'+mimic let7b: cells co-infected with 5'-half vectors containing miR target sites and 3'-half vectors in the presence of mir-let7b mimics; 5' : cells infected with 5'-half vectors without miR target sites;5'mir: cells infected with 5'-half vectors containing miR target sites in the presence of scramble miR mimics; 5'mir+mimic let7b: cells infected with 5'-half vectors containing miR target sites in the presence of mir-let7b mimics; neg: control cells infected with either the 3'-half vectors or EGFP-expressing vectors; 5'mir+3'+mimic 204+124: cells co-infected with 5'-half vectors containing miR target sites and 3'-half vectors in the presence of mir-204 and 124 mimics; 5'mir+mimic 204+124: cells infected with 5'-half vectors containing miR target sites in the presence of mir-204 and 124 mimics; 5'mir+3'+mimic 26a: cells co-infected with 5'-half vectors containing miR target sites and 3'-half vectors in the presence of mir-26a mimics; 5'mir+mimic 26a: cells infected with 5'-half vectors containing miR target sites in the presence of mir-26a mimics. α-3xflag: Western blot with anti-3xflag antibodies; α-Filamin A, Western blot with anti-Filamin A antibodies, used as loading control

Scramble sequence correspond to sequence of a different miRNA, for instance in the experiment with mir-let7b mimics the scramble sequence was that of miR26a.

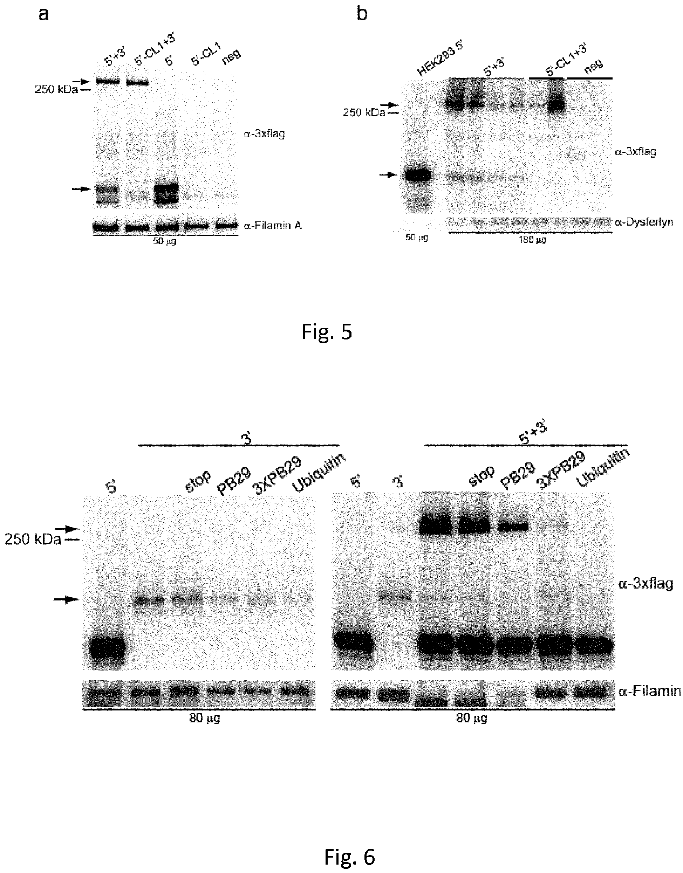

*Figure 5**. Inclusion of CL1 degradation signal in the* 5'-half *vectors results in significant reduction of truncated protein products*
Representative Western blot analysis of either (a) HEK293 cells infected with dual AAV2/2 (AAV serotype 2, with homologous ITR from AAV2) hybrid vectors or (b) pig eyes (RPE+retina) one month post-injection of dual AAV2/8 (AAV serotype 8, with homologous ITR from AAV2) hybrid vectors encoding for *ABCA4* and containing or not the CL1 degradation signal. The upper arrows indicate the full-length ABCA4 protein, the lower arrows indicate the truncated protein from the 5'-half vector; the molecular weight ladder is depicted on the left. The micrograms of proteins loaded are depicted below each image. **5'+3':** cells co-infected or eyes co-injected with 5'-half vectors without CL1 and 3'-half vectors; **5'-CL1+3':** cells co-infected or eyes co-injected with 5'-half vectors containing CL1 and 3'-half vectors; 5': cells infected with 5'-half vectors wihtout CL1; **5'-CL1:** cells infected with 5'-half vectors containing CL1; **neg:** control cells infected or control eyes injected with either the 3'-half vectors or EGFP expressing vectors, as negative controls; **α-3xflag:** Western blot with anti-3xflag antibodies; **α-Filamin A:** Western blot with anti-Filamin A antibodies, used as loading control; **α-Dysferlin:** Western blot with anti-Dysferlin antibodies, used as loading control, (a) The Western blot image is representative of n = 3 independent experiments, (b) The Western blot image is representative of n= 5 eyes injected with 5'+3' vectors, n=2 eyes injected with 5'-CL1+3' vectors and n=5 of eyes injected with either the 3'-half vectors or EGFP expressing vectors as negative controls.

*Figure 6**. Inclusion of* degradation *signals in the 3'-half vectors results in slight reduction of truncated protein products*
Representative Western blot analysis of HEK293 cells infected with dual AAV2/2 hybrid vectors encoding for *ABCA4* and containing different degradation signals. The upper arrow indicates the full-length ABCA4 protein, the lower arrow indicates truncated protein products; the molecular weight ladder is depicted on the left. The micrograms of proteins loaded are depicted below each image. **5'+3':** cells co-infected with 5'- and 3'-half vectors without degradation signals; **5':** cells infected with 5'-half vectors; **3' (no label):** cells infected with 3'-half vectors without degradation signals; **stop:** cells infected with 3'-half vectors containing stop codons; **PB29:** cells infected with 3'-half vectors containing the PB29 degradation signal; **3xPB29:** cells infected with 3'-half vectors containing 3 tandem copies of the PB29 degradation signal; **Ubiquitin:** cells infected with 3'-half vectors containing the ubiquitin degradation signal. **α-3xflag**: Western blot with anti-3xflag antibodies; **α-Filamin A:** Western blot with anti-Filamin A antibodies, used as loading control.

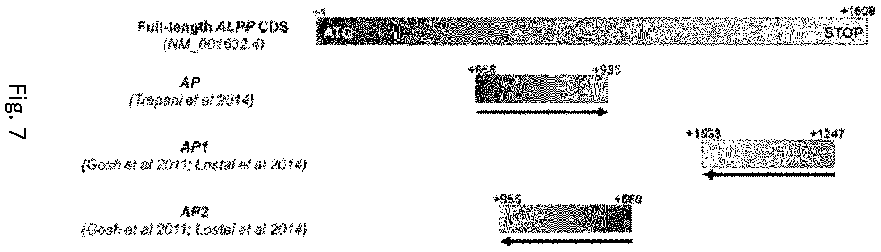

*Figure 7*: *Schematic representation of the AP, AP1 and AP2 regions of homology derived from ALPP (placental alkaline phosphatase) used in preferred embodiments of the present invention.* CDS: coding sequence

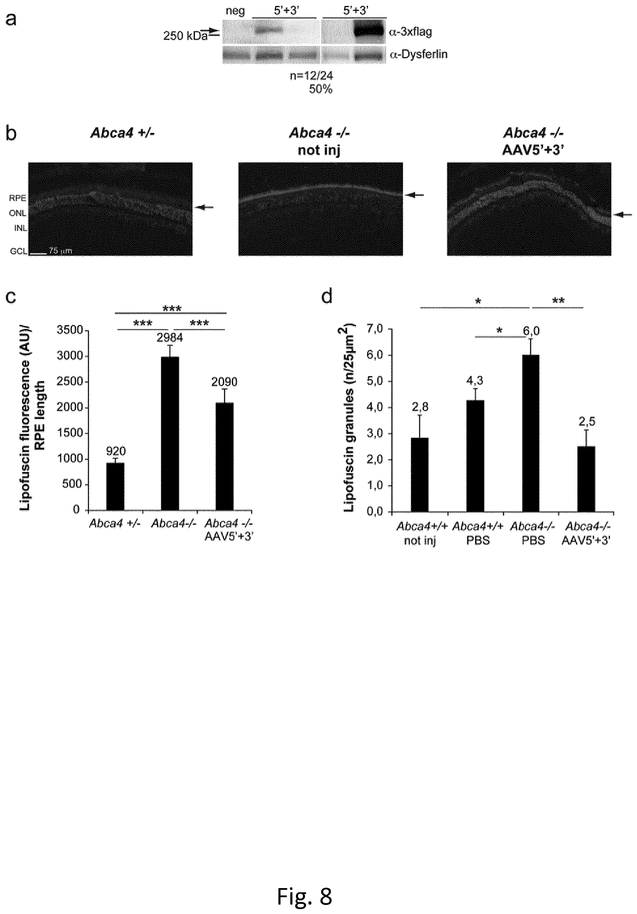

*Figure 8**: Subretinal delivery of improved dual AAV vectors results in ABCA4 expression in mouse photoreceptors and significant reduction of lipofuscin accumulation in the Abca4-*/*- mouse retina.* (a) Representative Western blot analysis of C57BL/6 retinas (whole retinal lysates) either injected with dual AAV2/8 hybrid ABCA4 vectors (5' + 3') or with negative controls (neg). The arrow indicates full-length proteins, the molecular weight ladder is depicted on the left. α-3×flag: Western blot with anti-3×flag antibodies; α-Dysferlin: Western blot with anti-Dysferlin antibodies, used as loading control. (b and c) Representative pictures (b) and quantification (c) of lipofuscin autofluorescence (red signal) in the retinas (RPE or RPE + OS) of either pigmented Abca4+/- mice not injected or injected with AAV as control (Abca4+/-) or pigmented Abca4-/- mice either not injected (Abca4-/-) or injected with dual AAV hybrid ABCA4 vectors (Abca4-/- AAV5'+3'). (b) The scale bar (75 µm) is depicted in the picture. RPE: retinal pigment epithelium; ONL: outer nuclear layer; INL: inner nuclear layer; GCL: ganglion cell layer. The arrows indicate lipofuscin signal. (c) Mean lipofuscin autofluorescence in the temporal side of three sections for each sample. Mean autofluorescence in each section was normalized for the length of the underlying RPE. The mean value is depicted above the corresponding bar. Values are represented as mean ± s.e.m. ^{∗∗∗}p ANOVA < 0.0001. n = 4 eyes for each group. (d) Mean number of RPE lipofuscin granules counted in at least 40 fields (25 µm2)/retina of albino Abca4+/+ mice either not injected (Abca4+/+ not inj) or injected with PBS (Abca4+/+ PBS), and albino Abca4-/- mice injected with either PBS (Abca4-/- PBS) or dual AAV hybrid ABCA4 vectors (Abca4-/- AAV5'+3'). The mean value is depicted above the corresponding bar. Values are represented as mean ± s.e.m. ^{∗}pANOVA ≤ 0.05; ^{∗∗}pANOVA ≤0.01. n = 4 eyes from Abca4+/+ not inj; n = 4 eyes from Abca4+/+ PBS; n = 3 eyes from Abca4-/- PBS; n = 3 eyes from Abca4-/- AAV5'+ 3'.

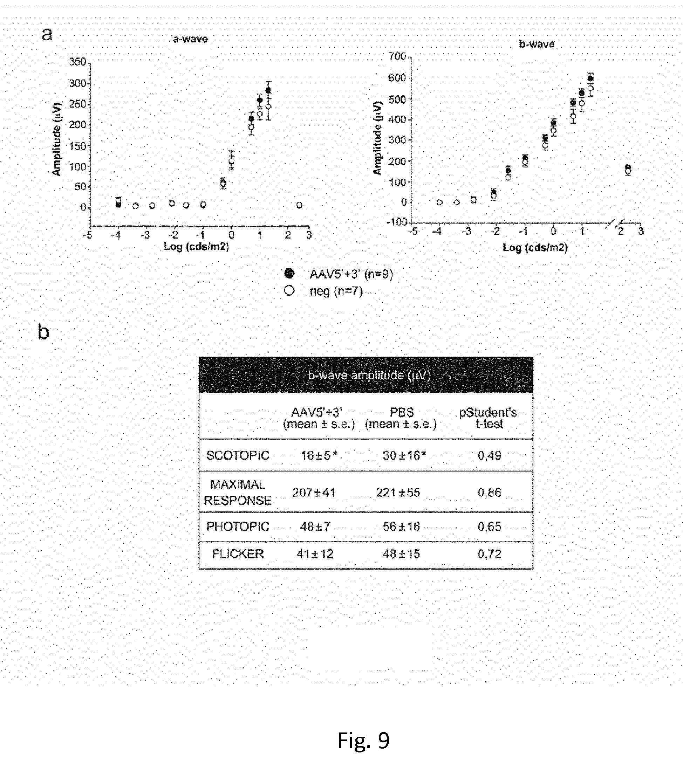

*Figure 9**: Similar electrical activity between either negative control or improved dual AAVtreated eyes of mice and pigs.* (a) Mean a-wave (left panel) and b-wave (right panel) amplitudes of C57BL/6 mice 1-month post-injection of either dualAAV hybrid ABCA4 vectors (AAV5'+ 3') or negative controls (i.e. negative control AAV vectors or PBS; neg). Data are presented as mean ± s.e.m.; n indicates the number of eyes analysed.
(b) Mean b-wave amplitudes (µV) in scotopic, maximal response, photopic and flicker ERG tests in pigs 1-month post-injection of either dual AAV hybrid ABCA4 vectors (AAV5'+ 3') or PBS. n = 5 eyes injected with dual AAV hybrid ABCA4 vectors; n = 4 injected with PBS; ^{∗}: n = 2.

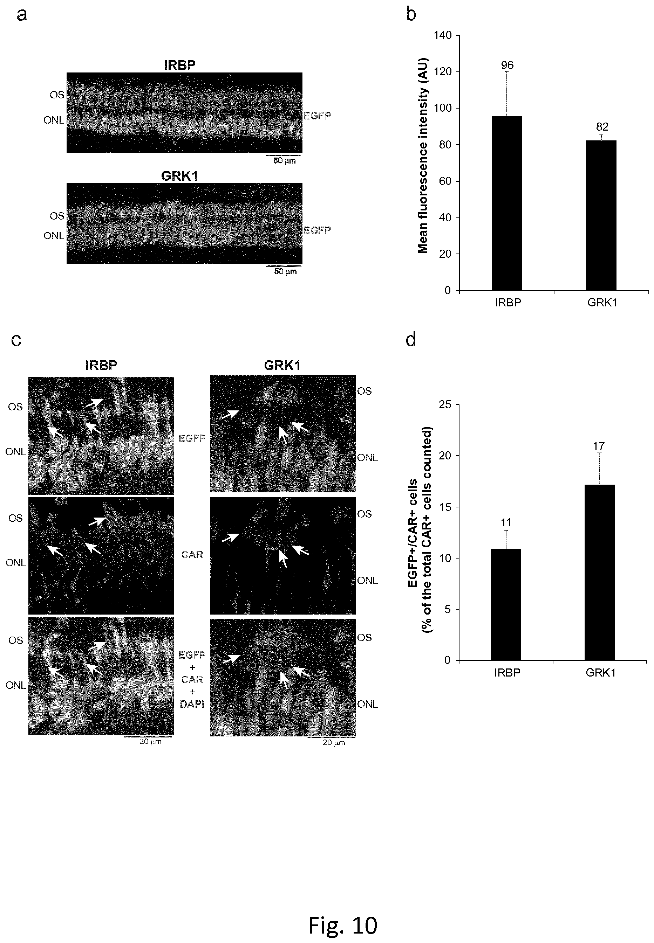

*Figure 10**: EGFP protein expression from the IRBP and GRK1 promoters in pig rod and cone photoreceptors.* Three month-old Large White pigs mice were injected subretinally with 1×10¹¹ GC/eye each of either AAV2/8-IRBP- or AAV2/8-GRK1-EGFP vectors. Retinal cryosections were obtained 4 weeks after injection and EGFP was analysed using fluorescence microscopy. (a-b) Representative images (a) and quantification (b) of fluorescence intensity in the PR layer. Fluorescence intensity was quantified for each group of animals on cryosections (six different fields/eye; 20x magnification), (c-d) Representative images (c) and quantification (d) of cone transduction efficiency. Cone transduction efficiency was evaluated on cryosections (six different fields/eye; 63x magnification) immunostained with an anti-LUMIf-hCAR antibody, and is expressed as number of cones expressing EGFP (EGFP+/CAR+) on total number of cones (CAR+) in each field. (a, c) The scale bar is depicted in the picture. (b-d) n=3 eyes injected with AAV2/8-IRBP-EGFP vectors; n=3 eyes injected with AAV2/8-GRK1-EGFP vectors. Values are represented as mean ± s.e.m. No significant differences were found using Student's t-test. OS: outer segments; ONL: outer nuclear layer; EGFP: native EGFP fluorescence; CAR: anti-cone arrestin staining; DAPI: 4',6'-diamidino-2-phénylindole staining. The arrows point at transduced cones.

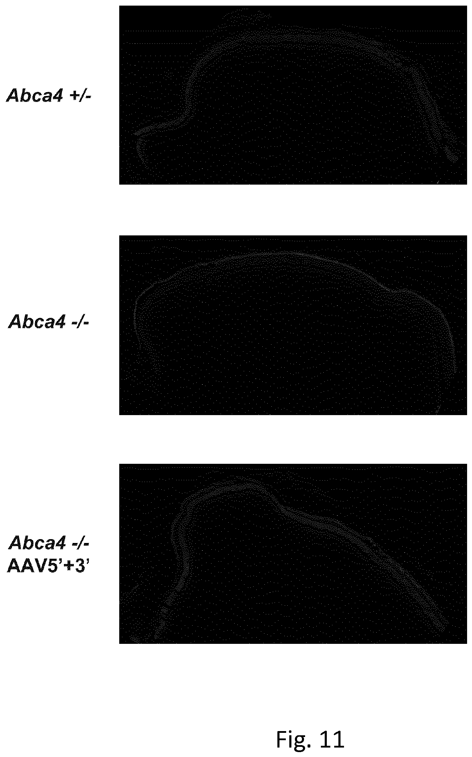

*Figure 11**: Subretinal delivery of improved dual AAV vectors results in significant reduction of lipofuscin accumulation in the Abca4-*/*- mouse retina.* Montage of images of the temporal (injected) side of retinal cross-sections showing lipofuscin auto fluorescence (red signal) in the retinas (RPE or RPE+OS) of either pigmented Abca4+/- mice not injected or injected with AAV as control (Abca4+/-) or pigmented Abca4-/- mice either not injected (Abca4-/-) or injected with dual AAV hybrid ABCA4 vectors (Abca4-/- AAV5'+3'). n= 4 eyes for each group. T: temporal side; N: nasal side.

*Figure 12**: Similar electrical activity between either negative control or improved dual AAVtreated eyes in mice and pigs.* (a) Representative ERG traces from C57BL/6 mice one month post-injection of either dual AAV hybrid ABCA4 vectors (AAV5'+3') or negative controls (i.e. negative control AAV vectors or PBS; neg). (b) Representative traces from scotopic, maximal response, photopic and flicker ERG tests in pigs one month post-injection of either dual AAV hybrid ABCA4 vectors (AAV5'+3') or PBS.

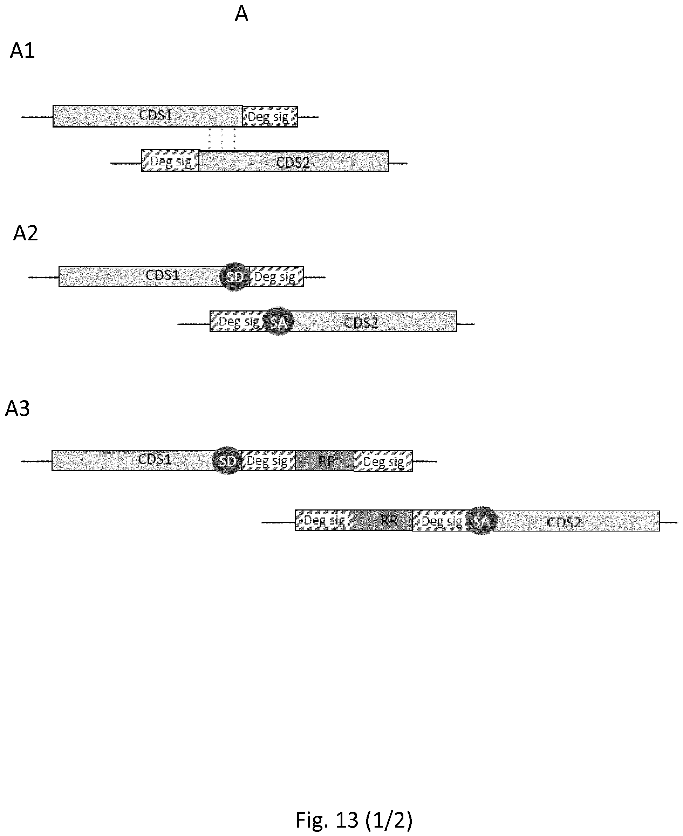

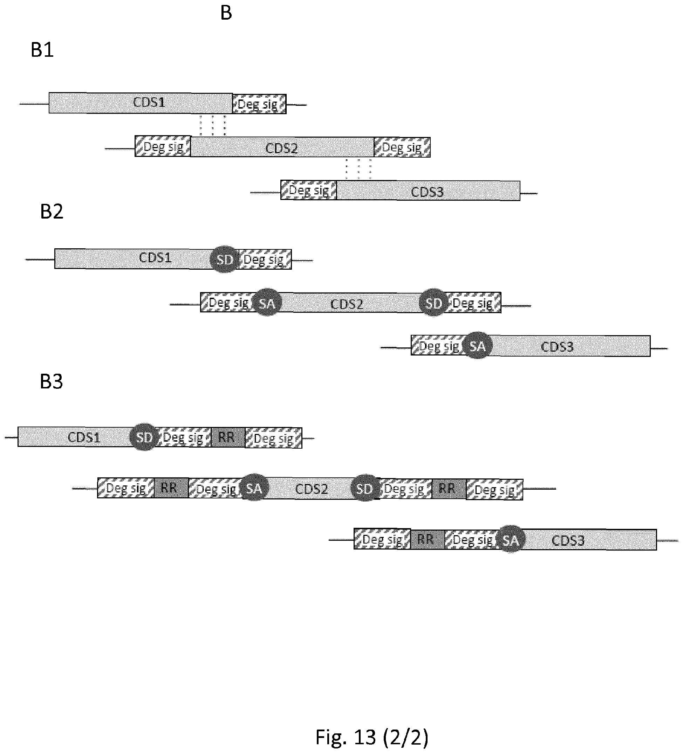

*Figure 13**. Schematic representation of vector system strategies, according to examples of the invention.* (A) Schematic representation of a vector system consisting of two vectors, according to preferred embodiments of the invention: a first vector comprises a first portion of the coding sequence (CDS1 portion), a second vector comprises a second portion (CDS2 portion) of the coding sequence. (A1) the reconstitution sequences of the vector system consist in the overlapping ends of the coding sequence portions. (A2) , the reconstitution sequences of the first and second vector consists respectively in a splicing donor and a splicing acceptor sequence. (A3) each reconstitution sequence comprises the splicing donor/acceptor, arranged as in A2 and it further comprises a recombinogenic region. A degradation signal is comprised in at least one of the vectors. The figure shows for each vector all the potential positions of the of the one or more degradation signals of the vector system, according to preferred non-limiting embodiments of the invention.
(B) Schematic representation of a vector system consisting of three vectors, according to preferred embodiments of the invention: a first vector comprises a first portion (CDS1 portion) of the coding sequence, a second vector comprises a second portion (CDS2 portion) of the coding sequence and a third vector comprises a third portion (CDS3 portion) of the coding sequence. (B1) the reconstitution sequences of the vector system consist in overlapping ends of the coding sequence portions (3' end of CDS1 overlapping with 5' end of CDS2; 3' end of CDS2 overlapping with 5' end of CDS3). (B2) the reconstitution sequence of the first vector consists in a splicing donor, the reconstitution sequence of the first vector consists in a splicing donor; the second vector comprises a first reconstitution sequence at the 5' end of CDS2 and a second reconstitution sequence at the 3' end of CDS2, the first reconstitution sequence being a splicing acceptor and the second being a splicing donor; the reconstitution sequence of the third vector consists in a splicing acceptor. (B3) each reconstitution sequence comprises the splicing donor/acceptor arranged as in B2 and further comprises a recombinogenic region. A degradation signal is comprised in at least one of the vectors. The figure shows for each vector all the potential positions of the one or more degradation signals of the vector system, according to preferred non-limiting embodiments of the invention.

CDS, coding sequence; SD, splicing donor signal; RR: recombinogenic regions; Deg Sig; degradation signals (see Table 2); SA, splicing acceptor signal.

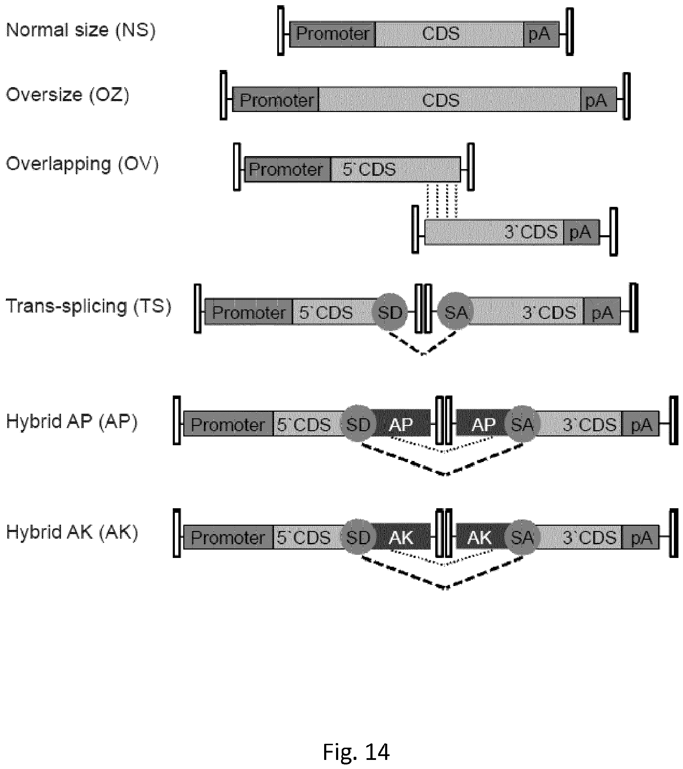

*Figure 14**. Schematic representation of prior art multiple vector-based strategies for large gene transduction.* CDS: coding sequence; pA: poly-adenilation signal; SD: splicing donor signal; SA: splicing acceptor signal; AP: alkaline phosphatase recombinogenic region; AK: F1 phage recombinogenic region. Dotted lines show the splicing occurring between SD and SA, pointed lines show overlapping regions available for homologous recombination. Normal size and oversize AAV vector plasmids contained full length expression cassettes including the promoter, the full-length transgene CDS and the poly-adenilation signal (pA). The two separate AAV vector plasmids (5' and 3') required to generate dual AAV vectors contained either the promoter followed by the N-terminal portion of the transgene CDS (5' plasmid) or the C-terminal portion of the transgene CDS followed by the pA signal (3' plasmid).

### DETAILED DESCRIPTION OF THE INVENTION

### MATERIALS AND METHODS

### Generation of plasmids

The plasmids used for AAV vector production were all derived from the dual hybrid AK vector plasmids encoding either the human *ABCA4,* the human *MYO7A* or the EGFP reporter protein containing the inverted terminal repeats (ITR) of AAV serotype 2 ¹⁴.

The AK recombinogenic sequence ¹⁴ contained in the vector plasmids encoding *ABCA4* was replaced with three different recombinogenic sequences derived from the alkaline phosphatase gene: AP (NM_001632, bp 823-1100, ¹⁴); AP1 (XM_005246439.2, bp1802-1516 ²⁰); AP2 (XM_005246439.2, bp 1225-938 ²⁰).

Dual AAV vector plasmids bearing heterologous ITR from AAV serotype 2 (ITR2) and ITR from AAV serotype 5 (ITR5) in the 5:2-2:5 configuration were generated by replacing the left ITR2 in the 5'-half vector plasmid and the right ITR2 in the 3'-half vector plasmids, respectively, with ITR5 (NC_006152.1, bp 1-175). Dual AAV vector plasmids bearing heterologous ITR2 and ITR5 in the 2:5 or 5:2 configurations were generated by replacing either the right or the left ITR2 with the ITR5, respectively. The pAAV5/2 packaging plasmid containing Rep5 (NC_006152.1, bp 171-2206) and the AAV2 Cap (AF043303 bp2203-2208) genes (Rep5Cap2), was obtained from the pAAV2/2 packaging plasmid, containing the Rep (AF043303 bp321-1993) and Cap (AF043303 bp2203-2208) genes from AAV2 (Rep2Cap2), by replacing the Rep2 gene with the Rep5 open reading frame from AAV5 (NC_006152.1, bp 171-2206).

The pZac5:5-CMV-EGFP plasmid containing the EGFP expression cassette with the ITR5 was generated from the pAAV2.1-CMV-EGFP plasmid, containing the ITR2 flanking the EGFP expression cassette ⁴⁵.

Degradation signals were cloned in dual AAV hybrid AK vectors encoding for *ABCA4* as follows: in the 5'-half vector plasmids between the AK sequence and the right ITR2; in the 3'-half vector plasmids between the AK sequence and the splice acceptor signal. Details on degradation signal sequences can be found in Table 2.

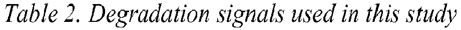

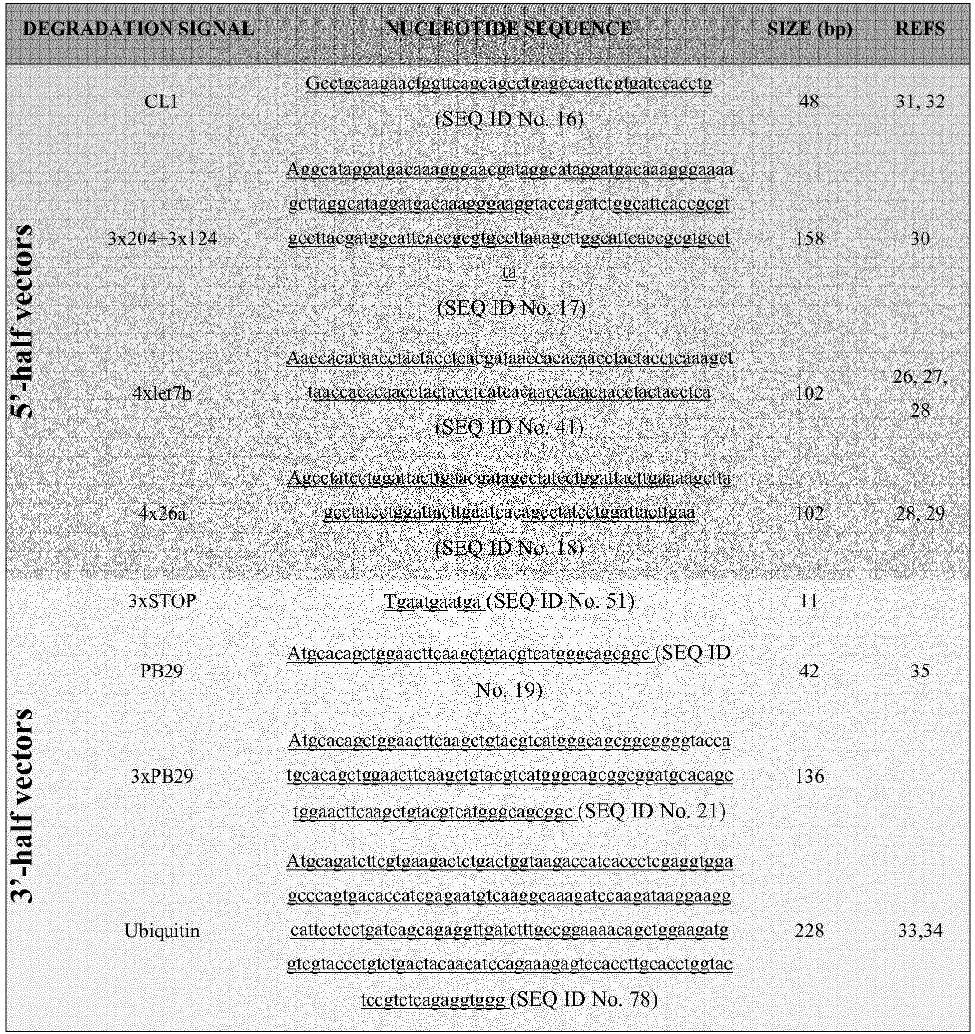

The sequences underlined correspond to the degradation signals; for degradation signals including repeated sequences, not underlined nucleotides are shown which have been included inbetween repeated sequences for cloning purposes.

The ABCA4 protein expressed from dual AAV vectors is tagged with 3xflag at both N- (amino acidic position 590) and C-termini for the experiments shown in Fig. 3 and 4 and Fig. 6, and at the C-terminus alone for the experiments in Fig. 2 and 8a.

Dual AAV hybrid vectors sets encoding for *ABCA4* used in this study included either the ubiquitous CMV ⁴⁶ or the PR-specific human G protein-coupled receptor kinase 1 (GRK1) ⁴⁷ promoters, while dual AAV hybrid vectors encoding for *MYO7A* included the ubiquitous CBA promoter ³⁹.

### AAV vector production and characterization

The AAV vector large preparations were produced by the TIGEM AAV Vector Core by triple transfection of HEK293 cells followed by two rounds of CsC12 purification. AAV vectors bearing homologous ITR2 were obtained as previously described ⁴⁸.

To obtain AAV vectors bearing heterologous ITR2 and ITR5 a suspension of 1.1×10⁹ lowpassage HEK293 cells was quadruple-transfected by calcium phosphate with 500 µg of pDeltaF6 helper plasmid which contains the Ad helper genes ⁴⁹, 260 µg of pAAV cis-plasmid and different amounts of Rep2Cap2 and Rep5 packaging constructs. The amount of Rep2Cap2 and Rep5 packaging constructs was as follows:
(i) PROTOCOL A: 130 µg of each Rep5 and Rep2Cap2 (ratio 1:1)
(ii) PROTOCOL B: 90 µg of Rep5 and 260 µg of Rep2Cap2 (ratio 1:3)
(iii) PROTOCOL C: 26 µg of Rep5 and 260 µg of Rep2Cap2 (ratio 1:10)

Each AAV preparation was then purified according to the published protocol ⁴⁸.

The protocols described below were used for the Rep competition experiments:
1- to assess Rep5 competition with Rep2 for production of AAV vectors with ITR2, HEK293 cells were either quadruple-transfected by calcium phosphate with pDeltaF6, pAAV2.1-CMV-EGFP cis, the Rep2Cap2 and Rep5Cap2 constructs at a weight ratio of 2:1:1.5:1.5 or, as a control, quadruple-transfected with the pDeltaF6, pAAV2.1-CMV-EGFP, the Rep2Cap2 packaging construct and a control irrelevant plasmid at a weight ratio of 2:1:1.5:1.5;
2- to assess Rep2 competition with Rep5 for production of AAV vectors with ITR5, HEK293 cells were either quadruple-transfected by calcium phosphate with pDeltaF6, pZac5:5-CMV-EGFP, the Rep5Cap2 and Rep2Cap2 constructs at a weight ratio of 2:1:1.5:1.5 or, as a control, quadruple-transfected with pDeltaF6, pZac5:5-CMV-EGFP, the Rep5 construct and a control irrelevant plasmid at a weight ratio of 2:1:1.5:1.5.

For the large-scale AAV vector preparations physical titres [genome copies (GC)/mL] were determined by averaging the titre achieved by PCR quantification using TaqMan (Applied Biosystems, Carlsbad, CA, USA) ⁴⁸ with a probe annealing on ITR2 and that obtained by dot-blot analysis ⁵⁰ with a probe annealing within 1 kb from ITR2. For the large-scale AAV vector preparations produced with different Rep5:Rep2Cap2 weight ratio, physical titres [genome copies (GC)/mL] were determined by PCR quantification using TaqMan with a probe annealing on ITR2. For the AAV vector preparations used in the competition experiments physical titres [genome copies (GC)] were determined by PCR quantification using TaqMan with a probe annealing on the bovine growth hormone (BGH) polyadenilation signal, included in the *EGFP*expressing cassette packaged in the AAV vectors.

### AAV infection of HEK293 cells

AAV infection of HEK293 cells was performed as previously described ¹⁴. AAV2 vectors bearing heterologous ITR2 and ITR5 and produced according to Protocol C were used to infect HEK293 cells with a multiplicity of infection (m.o.i) of 1×10⁴ GC/cell of each vector (2×10⁴ total GC/cell when the inventors used dual AAV vectors at a 1:1 ratio) calculated considering the lowest titre achieved for each viral preparation. Infections with AAV2/2 bearing recombinogenic regions and degradation signals were carried out with a m.o.i of 5×10⁴ GC/cell of each vector (1×10⁵ total GC/cell in the case of dual AAV vectors at 1:1 ratio) calculated considering the average titre between TaqMan and dot-blot.

For the experiments using 5'-half vectors containing miR target sites, cells were transfected using calcium phosphate 4 hours prior to infection with the corresponding miR mimics (50 nM; miRIDIAN microRNA mimic hsa-let-7b-5p, hsa-miR-204-5p, hsa-miR-124-3p and hsa-miR-26a-5p; Dharmacon, Lafayette, CO, USA).

### Subretinal injection of AAV vectors in mice and pigs

Mice were housed at the Institute of Genetics and Biophysics animal house (Naples, Italy), maintained under a 12-h light/dark cycle (10-50 lux exposure during the light phase). C57BL/6 mice were purchased from Harlan Italy SRL (Udine, Italy). Pigmented *Abca4-*/*-* mice were generated through successive crosses of albino *Abca4-*/*-* mice¹⁴ with Sv129 mice and maintained inbred; breeding was performed crossing heterozygous mice with homozygous mice. Albino *Abca4-*/*-* mice were generated through successive crosses and backcrossed with BALB/c mice (homozygous for Rpe65 Leu450) and maintained inbred; breeding was performed crossing heterozygous mice with homozygous mice. C57BL/6 (5 week-old), pigmented *Abca4-*/*-* (5.5 month-old) and albino *Abca4-*/*-* (2.5-3-month old) mice were anesthetized as previously described⁶¹, then 1 µl of either PBS or AAV2/8 vectors was delivered subretinally to the temporal side of the retina via a trans-scleral trans-choroidal approach as described by Liang et al⁶². AAV2/5-VMD2-human Tyrosinase⁶³ (dose: 2×10⁸ GC/eye) was added to the AAV2/8 vector solution that was subretinally delivered to albino *Abca4-*/*-* mice (Fig. 8d). This allowed us to mark the RPE within the transduced part of the eyecup, which was subsequently dissected and analyzed.

The Large White Female pigs used in this study were registered as purebred in the LW Herd Book of the Italian National Pig Breeders' Association. Pigs were housed at the Cardarelli hospital animal house (Naples, Italy) and maintained under 12-hour light/dark cycle (10-50 lux exposure during the light phase). This study was carried out in accordance with the Association for Research in Vision and Ophthalmology Statement for the Use of Animals in Ophthalmic and Vision Research and with the Italian Ministry of Health regulation for animal procedures. All procedures were submitted to the Italian Ministry of Health; Department of Public Health, Animal Health, Nutrition and Food Safety. Surgery was performed under anesthesia and all efforts were made to minimize suffering. Animals were sacrificed as previously described³⁹. Subretinal delivery of AAV vectors to 3 month-old pigs was performed as previously described³⁹. All eyes were treated with 100 µl of either PBS or AAV2/8 vector solution. The AAV2/8 dose was 1×10¹¹GC of each vector/eye therefore co-injection of dual AAV vectors at a 1:1 ratio resulted in a total dose of 2×10¹¹ GC/eye.

For the animal studies included in Figure 2c, 5b, 8, 9, 10, 11 and 12, right and left eyes were assigned randomly to the various experimental groups and the researchers conducting and quantifying the experiments were blind to the treatment received by the animals.

### Western blot analysis

For Western blot analysis HEK293 cells, mouse and pig retinas were lysed in RIPA buffer (50 mM Tris-HCl pH 8.0, 150 mM NaCl, 1% NP40, 0.5% Na-Deoxycholate, 1 mM EDTA pH 8.0, 0.1% SDS). Lysis buffers were supplemented with protease inhibitors (Complete Protease inhibitor cocktail tablets; Roche) and 1 mM phenylmethylsulfonyl. After lysis, samples of cells containing MYO7A were denatured at 99°C for 5 min in 1X Laemli sample buffer; samples containing ABCA4 were denatured at 37°C for 15 min in 1X Laemli sample buffer supplemented with 4 M urea. Lysates were separated by 6-7% (ABCA4 and MYO7A samples, respectively) or 8% (WB in Fig. 5b) SDS-polyacrylamide gel electrophoresis, The antibodies used for immuno-blotting are as follows: anti-3×flag (1:1000, A8592; Sigma-Aldrich); anti-MYO7A (1:500, polyclonal; Primm Srl, Milan, Italy) generated using a peptide corresponding to aminoacids 941-1070 of the human MYO7A protein; anti-Filamin A (1:1000, catalog #4762; Cell Signaling Technology, Danvers, MA, USA); anti-Dysferlin (1:500, Dysferlin, clone Ham1/ 7B6, MONX10795; Tebu-bio, Le Perray-en-Yveline, France). The quantification of ABCA4 and MYO7A bands detected by Western blot was performed using ImageJ software (free download available at http://rsbweb.nih.gov/ij/). For the in vitro experiments performed with AAV bearing heterologous ITR2 and ITR5, the intensity of the full-length ABCA4 and MYO7A bands was normalized to either that of the truncated protein product in the corresponding lane or to that of Filamin A bands, while the intensity of the shorter ABCA4 and MYO7A proteins bands was normalized to that of Filamin A bands. The intensity of ABCA4 bands achieved with AAV vectors bearing degradation signals or homology regions was normalized to that of Filamin A bands for the *in vitro* experiments or Dysferlin bands for the *in vivo* experiments. Quantification of the Western blot experiments has been performed as follows:
- Fig. 2a-b: the intensity of the ABCA4 band was normalized to that of Filamin A band in the corresponding lane. Normalized ABCA4 expression was then expressed as percentage relative to dual AAV hybrid AK vectors;
- Fig. 2c: the intensity of the ABCA4 band (a.u.) was calculated as fold of increase relative to the mean intensity measured at the same level in the negative control lanes of each gel (the measurement of the negative control sample in lane 7 of the lower left panel was excluded from the analysis given the exceptionally high background signal). Values for each group are represented as mean ± standard error of the mean (s.e.m.);
- Fig. 3b-d: the full-length ABCA4 and truncated protein band intensities were divided by those of the Filamin A bands or the intensity of the full-length ABCA4 protein bands was divided by that of the truncated protein bands in the corresponding lane. Values are represented as: mean ± s.e.m.;
- Table 5: full-length ABCA4 and truncated protein band intensities were measured in cells co-infected with 5'- and 3'-half vectors. The ratio between the intensity of full-length ABCA4 and truncated protein bands in the presence of either the corresponding mimic or a scramble mimic was calculated. Values represent mean ± s.e.m. of the ratios from three independent experiments;
- Table 6: full-length ABCA4 and truncated protein band intensities were measured in cells co-infected with 5'- and 3'-half vectors. The ratio between the intensity of the full-length ABCA4 and truncated bands from vectors either with or without the degradation signals was calculated. Values represent mean ± s.e.m. of the ratios from three independent experiments.
- Fig. 8a: the intensity of the ABCA4 band (a.u.) was calculated as fold of increase relative to the mean background intensity measured in the negative control lanes of the corresponding gel. Values are expressed as mean ± s.e.m.

### Southern blot analysis

Three ×10¹⁰ GC of viral DNA were extracted from AAV particles. To digest unpackaged genomes, the vector solution was resuspended in 240 µl of PBS pH 7.4 19 (GIBCO; Invitrogen S.R.L., Milan, Italy) and then incubated with 1 U/ul of DNase I (Roche) in a total volume of 300 µl containing 40 mM TRIS-HCl, 10 mM NaCl, 6 mM MgC12, 1 mM CaC12 pH 7.9 for 2 h at 37°C. The DNase I was then inactivated with 50 mM EDTA, followed by incubation with proteinase K and 2.5% N-lauroyl-sarcosil solution at 50°C for 45 min to lyse the capsids. The DNA was extracted twice with phenol-chloroform and precipitated with two volumes of ethanol 100 and 10% sodium acetate (3 M, pH 7). Alkaline agarose gel electrophoresis and blotting were performed as previously described (Sambrook & Russell, 2001 Molecular Cloning). Ten microlitres of the 1 kb DNA ladder (N3232L; New England Biolabs, Ipswich, MA, USA) were loaded as molecular weight marker. Two different double strand DNA fragments were labelled with digoxigenin-dUTP using the DIG high prime DNA labelling and detection starter kit (Roche) and used as probes. The 5' probe (768 bp) was generated by double digestion of the pZac2.1-CMV-ABCA4_5' plasmid with SpeI and NotI; the 3' probe (974 bp) was generated by double digestion of the pZac2.1-ABCA4_3'_3xflag_SV40 plasmid with ClaI and MfeI. Prehybridization and hybridization were performed at 65°C in Church buffer (Sambrook & Russel, 2001 Molecular cloning) for 1 h and overnight, respectively. Then, the membrane (Whatman Nytran N, charged nylon membrane; Sigma-Aldrich, Milan, Italy) was first washed for 30 min in SSC 29-0.1% SDS, then for 30 min in SSC 0.59-0.1% SDS at 65°C, and then for 30 min in SSC 0.19-0.1% SDS at 37°C. The membrane was then analyzed by chemiluminescence detection by enzyme immunoassay using the DIG DNA Labeling and Detection Kit (Roche).

### Histological analysis

Mice were euthanized, and their eyeballs were then harvested and fixed overnight by immersion in 4% paraformaldehyde (PFA). Before harvesting the eyeballs, the temporal aspect of the sclerae was marked by cauterization, in order to orient the eyes with respect to the injection site at the moment of the inclusion. The eyeballs were cut so that the lens and vitreous could be removed while leaving the eyecup intact. Mice eyecups were infiltrated with 30% sucrose for cryopreservation and embedded in tissue-freezing medium (O.C.T. matrix; Kaltek, Padua, Italy). For each eye, 150-200 serial sections (10 µm thick) were cut along the horizontal plane and the sections were progressively distributed on 10 slides so that each slide contained 15 to 20 sections, each representative of the entire eye at different levels. The sections were stained with 4',6'-diamidino-2-phenylindole (Vectashield; Vector Lab, Peterborough, United Kingdom) and were monitored with a Zeiss Axiocam (Carl Zeiss, Oberkochen, Germany) at different magnifications.

Pigs were sacrificed, and their eyeballs were harvested and fixed overnight by immersion in 4% PFA. The eyeballs were cut so that the lens and vitreous could be removed, leaving the eyecups in place. The eyecups were gradually dehydrated by progressively infiltrating them with 10%, 20%, and 30% sucrose. Tissue-freezing medium (O.C.T. matrix; Kaltek) embedding was performed. Before embedding, the swine eyecups were analyzed with a fluorescence stereomicroscope (Leica Microsystems GmbH, Wetzlar, Germany) in order to localize the transduced region whenever an EGFP-encoding vector was administered. For each eye, 200-300 serial sections (12 µm thick) were cut along the horizontal meridian and the sections were progressively distributed on glass slides so that each slide contained 6-10 sections. Section staining and image acquisition were performed as described for mice.

### Cone immunofluorescence staining

Frozen retinal sections were washed once with PBS and then permeabilized for 1 hr in PBS containing 0.1% Triton X-100. Blocking solution containing 10% normal goat serum (Sigma-Aldrich) was applied for 1 hr. Primary antibody [anti-human CAR^{66,67}, which also recognises the porcine CAR ("Luminaire founders"-hCAR, 1:10,000; kindly provided by Dr. Cheryl M. Craft, Doheny Eye Institute, Los Angeles, CA)] was diluted in PBS and incubated overnight at 4°C. The secondary antibody (Alexa Fluor 594, anti-rabbit, 1:1,000; Molecular Probes, Invitrogen, Carlsbad, CA) was incubated for 45 min. Sections stained with the anti-CAR antibodies were analyzed at 63x magnification using a Leica Laser Confocal Microscope System (Leica Microsystems GmbH), as previously described⁶⁴. Briefly, for each eye six different z-stacks from six different transduced regions were taken. For each z-stack, images from single plans were used to count CAR+ /EGFP + cells. In doing this, the inventors carefully moved along the Z-axis to distinguish one cell from another and thus to avoid to count twice the same cell. For each retina the inventors counted the CAR-positive (CAR+)/EGFP-positive (EGFP+) cells on total CAR+ cells. The inventors then calculated the average number of CAR+ /EGFP + cells of the three eyes of each experimental group.

### EGFP quantification

Fluorescence intensity in PR was rigorously and reproducibly quantified in an unbiased manner as previously described⁶⁴. Individual color channel images were taken using a Leica microscope (Leica Microsystems GmbH). TIFF images were gray-scaled with image analysis software (LAS AF lite; Leica Microsystems GmbH). Six images of each eye were analyzed at 20x magnification by a masked observer. PR (outer nuclear layer + OS) were selectively outlined in every image, and the total fluorescence for the enclosed area was calculated in an unbiased manner using the image analysis software. The fluorescence in PR was then averaged from six images collected from separate retinal sections from each eye. The inventors then calculated the average fluorescence of the three eyes of each experimental group.

### Quantification of lipofuscin autofluorescence

For lipofuscin fluorescence analysis, eyes were harvested from pigmented *Abca4*+/*-* and *Abca4-*/- mice at 3 months after AAV injection. Mice were dark-adapted over-night and sacrificed under dim red-light. For each eye, four overlapping pictures from the temporal side of three sections from different regions of the eye were taken using a Leica DM5000B microscope equipped with a TX2 filter (excitation: 560±40 nm; emission: 645±75) ⁷¹⁻⁷⁵ and under a 20X objective. The four images for each section were then combined in a single montage used for further fluorescence analysis. Intensity of lipofuscin fluorescence (red signal) in each section was automatically calculated using the ImageJ software and was then normalized for the length of the RPE underlying the area of fluorescence.

### Transmission electron microscopy

For electron microscopy analyses eyes were harvested from albino *Abca4-*/*-* and *Abca4*+/+ mice at 3 months after AAV injection. Eyes were fixed in 0.2% glutaraldehyde-2% paraformaldehyde in 0.1 M PHEM buffer pH 6.9 (240 mM PIPES, 100 mM HEPES, 8 mM MgC12, 40 mM EGTA) overnight and then rinsed in 0.1 M PHEM buffer. Eyes were then dissected under light microscope to select the tyrosinase-positive portions of the eyecups. The transduced portion of the eyecups were subsequently embedded in 12% gelatin, infused with 2.3 M sucrose and frozen in liquid nitrogen. Cryosections (50 nm) were cut using a Leica Ultramicrotome EM FC7 (Leica Microsystems) and extreme care was taken to align PR connecting cilia longitudinally. To avoid bias in the attribution of morphological data to the various experimental groups, counts of lipofuscin granules were performed by a masked operator (Dr. Roman Polishchuk) using the iTEM software (Olympus SYS, Hamburg, Germany). The 'Touch count' module of the iTEM software was used to count the number of lipofuscin granules in 25 µm² areas (at least 40) distributed randomly across the RPE layer. The granule density was expressed as number of granules per 25 µm².

### Electroretinogram Recordings

Electrophysiological recordings in mice and pigs were performed as detailed in (68) and in (69), respectively.

### Statistical analysis

p-values ≤ 0.05 were considered statistically significant. One-way ANOVA (R statistical software) with post-hoc Multiple Comparison Procedure was used to compare data depicted in Figure 2b (pANOVA=1,2 × 10⁻⁶), 2c (pANOVA= 0,326), 8c (pANOVA=1,5 × 10⁻¹⁰), 8d (pANOVA= 0,034) and 9a (pANOVA a-wave: 0,5; pANOVA b-wave: 0,8) and Table 6 (pANOVA= 0,0135). As the counts of lipofuscin granules (Fig. 8d) are expressed as discrete numbers, these were analyzed by deviance from a Negative Binomial generalized linear models⁶⁵. The statistically significant differences between groups determined with the post-hoc Multiple Comparison Procedure are the following: Fig. 2b: AP vs AK: 1,08 × 10⁻⁵; AP1 vs AK: 0,05; AP2 vs AK: 0,17; AP1 vs AP: 1,8 × 10⁻⁶; AP2 vs AP: 2,8 × 10⁻⁶; AP2 vs AP1: 0,82. Fig. 8c: *Abca4*+/- not inj vs *Abca4-*/*-* not inj: 0,00; *Abca4-*/*-* not inj vs *Abca4-*/*-* AAV5'+3': 9,3 × 10⁻⁵; *Abca4*+/- not inj vs *Abca4-*/*-* AAV5'+3': 4 × 10⁻⁶. Fig. 8d: *Abca4-*/*-* PBS vs *Abca4-*/*-* AAV5'+3': 0,01; *Abca4*+/+ PBS vs *Abca4-*/*-* AAV5'+3': 0,37; *Abca4*+/+ not inj vs *Abca4-*/*-* AAV5'+3': 0,53; *Abca4*+/+ PBS vs *Abca4-*/*-* PBS: 0,05; *Abca4*+/+ not inj vs *Abca4-*/*-* PBS: 0,03; *Abca4*+/+ not inj vs *Abca4*+/+ PBS: 0,76. Table 6: 3xSTOP vs no degradation signal: 0,97; 3xSTOP vs PB29: 1,0; 3xSTOP vs 3xPB29: 0,15; 3xSTOP vs ubiquitin: 0,10; PB29 vs no degradation signal: 1,0; PB29 vs 3xPB29: 0,1; PB29 vs ubiquitin: 0,07; 3xPB29 vs no degradation signal: 0,06; 3xPB29 vs ubiquitin: 1,0; ubiquitin vs no degradation signal: 0,04.

The Student's t-test was used to compare data depicted in Figure 3c, d and f.

### RESULTS

### Dual AAV hybrid vectors which include the AP1, AP2 or AK recombinogenic regions show efficient transduction

The inventors evaluated several multiple vector strategies as depicted in Fig. 1 and 13.

In particular, they evaluated in parallel the transduction efficacy of dual AAV hybrid vectors with different regions of homology. For this purpose the inventors generated dual AAV2/2 hybrid vectors that include the *ABCA4*-3xflag coding sequence, under the control of the ubiquitous CMV promoter, and either the AK ¹⁴, AP ¹⁴, AP1 or AP2 ²⁰ regions of homology (Fig. 7). The inventors used these vectors to infect HEK293 cells [multiplicity of infection, m.o.i.: 5×10⁴ genome copies (GC)/cell of each vector]. Cell lysates were analysed by Western blot with anti-3xflag antibodies to detect ABCA4-3xflag (Fig. 2). Each of the dual AAV hybrid vectors sets resulted in expression of full-length proteins of the expected size that were not detected in the lanes loaded with negative controls (Fig. 2a). Quantification of ABCA4 expression (Fig. 2b) showed that infection with dual AAV hybrid AP1 and AP2 vectors resulted in slightly higher levels of transgene expression than with dual AAV hybrid AK vectors and all significantly outperformed dual AAV hybrid AP vectors ¹⁴. The inventors have previously found that the efficiency of dual AAV vectors which rely on homologous recombination is lower in terminally-differentiated cells as PR than in cell culture ¹⁴. The inventors therefore evaluated PR-specific transduction levels in C57BL/6 mice following subretinal administration of dual AAV AK, AP1 and AP2 vectors which include the PR-specific human G protein-coupled receptor kinase 1 (GRK1) promoter (dose of each vector/eye: 1.9x10⁹ GC; Fig. 2c). One month after vector administration the inventors detected ABCA4 protein expression more consistently in retinas treated with dual AAV hybrid AK than AP1 or AP2 vectors (Fig. 2c).

### Inclusion of heterologous ITR in AAV vectors affects their production yields and does not reduce levels of truncated protein products

To test if the use of heterologous ITR improve the productive directional concatemerization of dual AAV vectors, the inventors generated dual AAV2/2 hybrid AK vectors that included either *ABCA4-3xflag* or *MYO7A*-HA coding sequences with heterologous ITR2 and ITR5 in either the 5:2 (left ITR from AAV5 and right ITR from AAV2) or the 2:5 (left ITR from AAV2 and right ITR from AAV5) configuration (Fig. 1). The production of dual AAV vectors bearing heterologous ITR2 and ITR5 requires the simultaneous expression of the Rep proteins from AAV serotypes 2 and 5 which cannot cross-complement virus replication ²³. Indeed, it has been shown that Rep2 and Rep5 can bind interchangeably to ITR2 or ITR5, although less efficiently than to homologous ITR, however they cannot cleave the terminal resolution sites of the ITR from the other serotype ³⁶. Therefore, before generating dual AAV hybrid AK vectors with heterologous ITR2 and ITR5, the inventors assessed the potential competition of (i) Rep5 with Rep2 in the production of AAV2/2-CMV-EGFP vectors (i.e. vectors with homologous ITR2) and (ii) Rep2 with Rep5 in the production of AAV5/2-CMV-EGFP vectors (i.e. vectors with homologous ITR5), using the same amount of the Rep5Cap2 and Rep2Cap2 packaging constructs (ratio1:1). Indeed, when the Rep5Cap2 packaging construct is provided in addition to Rep2Cap2, the total yields of AAV2/2-CMV-EGFP vectors are reduced to 42% of those of control preparations obtained when only Rep2Cap2 is provided as packaging construct (average of 4 independent preps of each type, p Student's t-test <0.05). Conversely, no significant differences were found in the total yields of AAV5/2-CMV-EGFP preps obtained when Rep2Cap2 was added to Rep5Cap2, which were 83% of those obtained when Rep5Cap2 was the only packaging construct transfected (average of 4 independent preps of each type, no significant differences were found using Student's t-test). Given the competition of Rep5 with Rep2 in the production of vectors with ITR2, the inventors tested three different ratios between Rep5 and the Rep2Cap2 packaging constructs in the production of AAV with heterologous ITR2 and ITR5 (Protocol A with 1:1, Protocol B with 1:3 and Protocol C with 1:10 Rep5/Rep2Cap2 ratio). As shown in Table 3, viral titres determined by PCR quantification using a probe annealing to ITR2 progressively increased when the amount of Rep5 was decreased, with the best titre obtained with Protocol C.

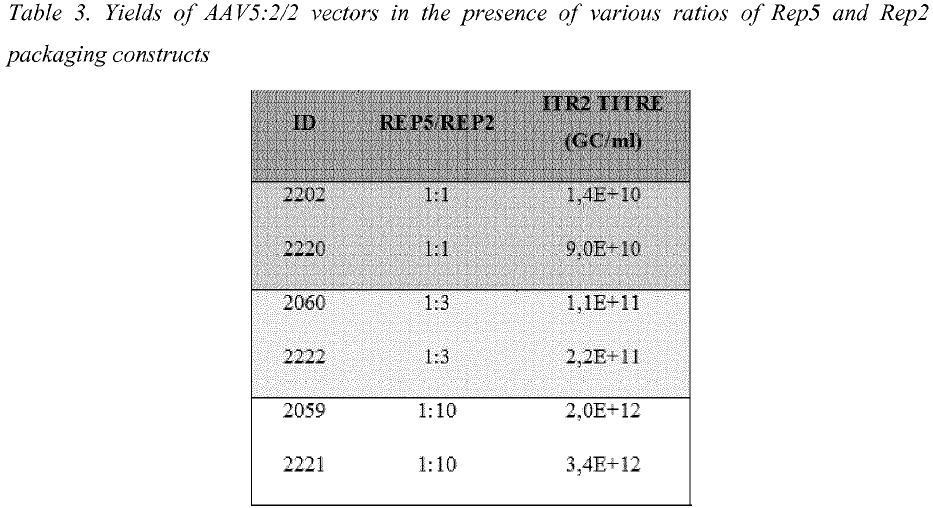

ID: identification number of AAV5:2/2 vectors; GC: genome copies.

These results confirmed the competition of Rep5 with Rep2 during the production of vectors with ITR2 and led us to follow Protocol C for the production of AAV vectors with heterologous ITR2 and ITR5. However, several AAV preparations obtained with this strategy revealed: (i) up to 6-fold lower titres determined on ITR2 than titres determined on a transgenic sequence in between the ITR (Table 4) which could suggest that the integrity of ITR2 is compromised and (ii) a mean reduction of about 6-fold in the total yields of AAV vectors with heterologous ITR2 and ITR5 compared to those containing homologous ITR2 (Table 4).

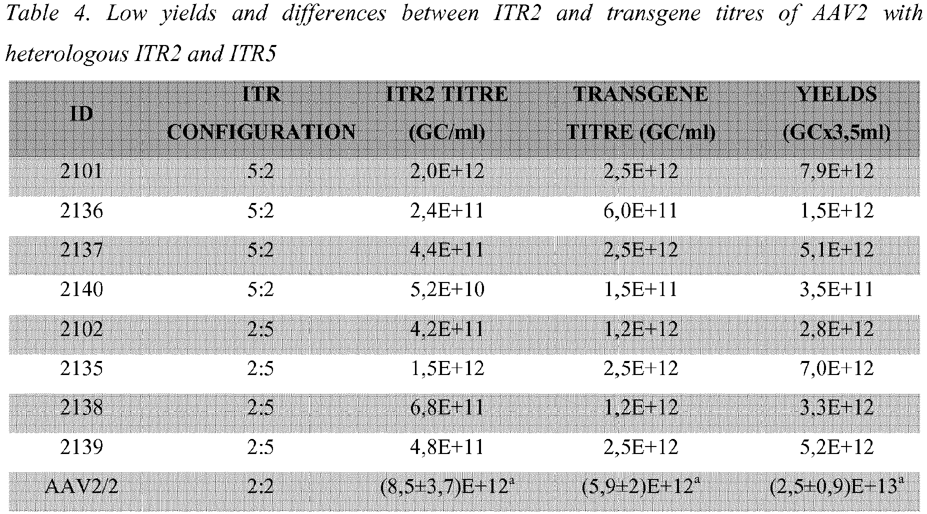

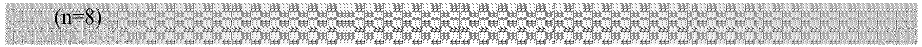

ID: identification number of AAV vectors; GC: genome copies.^{a} Values represent mean±SEM.

However, Southern blot analysis of AAV preparation with heterologous ITR revealed no evident alteration of genome integrity (Fig. 3a).

To test if the inclusion of heterologous ITR in dual AAV hybrid AK vectors enhanced the formation of tail-to-head productive concatemers and full-length protein transduction while reducing the production of truncated proteins, the inventors infected HEK293 cells with dual AAV hybrid vectors encoding for either *ABCA4* or *MYO7A* with either heterologous ITR2 and ITR5 (in the 5:2/2:5 configuration) or homologous ITR2 (Fig. 3b, 3e).

Given the difference between the ITR2 and transgene titres for vectors with heterologous but not homologous ITR (Table 4), the inventors infected cells with 10⁴ genome copies (GC)/cell of each vector based on either ITR2 or transgene titres. Western blot analysis of HEK293 cells infected with dual AAV vectors based on ITR2 titers, using anti-3×flag (to detect ABCA4-3xflag, Fig. 3b) or anti-Myo7a (Fig. 3e) antibodies, showed that the inclusion of heterologous ITR2 and ITR5 resulted in higher levels of both full-length and truncated protein than homologous ITR2 (Fig. 3b, c, d, f). However this was not observed when HEK293 cells were infected with the same dual AAV vector preps based on the transgene titre (Fig. 3b, d). In conclusion, the ratio between full-length and truncated protein expression was similar regardless of the ITR included in the vectors (Fig. 3 c, d, f) and of the vector titre used to dose cells (Fig. 3b, c, d).

### CL1 degron in the 5'-half vector decreases the production of truncated protein products

To selectively reduce the levels of truncated protein products produced by each 5'- and 3'-half of dual AAV hybrid vectors ¹⁴, the inventors placed putative degradation sequences in the 5'-half vector after the splicing donor signal between AK and the right ITR, and in the 3'-half vector between AK and the splicing acceptor signal (Fig. 1). Thus, the degradation signal will be included in the truncated but not in the full-length protein which results from a spliced mRNA. As degradation signals in the 5'-half vectors the inventors have included: (i) the CL1 degron (CL1), (ii) 4 copies of the miR-let7b target site (4xLet7b), (iii) 4 copies of the miR-26a target site (4x26a) or (iv) the combination of 3 copies each of miR-204 and miR-124 target sites (3x204+3x124) (Table 2). As degradation signals in the 3'-half vectors the inventors have included: (i) 3 stop codons (STOP), (ii) PB29 either in a single (PB29) or in three tandem copies (3xPB29) or (iii) ubiquitin (Table 2). The inventors generated dual AAV2/2 hybrid AK vectors encoding for ABCA4 including the various degradation signals and evaluated their efficacy after infection of HEK293 cells [m.o.i.: 5 × 10⁴ genome copies (GC)/cell of each vector]. Since miRlet7b, miR-26a, miR-204 and miR-124 are poorly expressed or completely absent in HEK293 cells (Ambion miRNA Research Guide and ³⁷), to test the silencing of the construct containing target sites for these miR, the inventors transfected cells with miR mimics (i.e. small, chemically modified double-stranded RNAs that mimic endogenous miR ³⁸) prior to infection with the AAV2/2 vectors containing the corresponding target sites. To define the concentration of miR mimics required to achieve silencing of a gene containing the corresponding miR target sites, the inventors used a plasmid encoding for the reporter EGFP protein and containing the miR target sites before the polyadenylation signal (data not shown). The same experimental settings were used for further evaluation of the miR target sites in the context of dual AAV hybrid AK vectors. The inventors found that inclusion of miR-204+124 and 26a target sequences in the 5'-half of dual AAV hybrid AK vectors reduced albeit did not abolish the expression of the truncated protein products without affecting full-length protein expression (Fig. 4). Differently, the inclusion of miR-let7b target sites was not effective in reducing truncated protein expression (Fig. 4).

Notably, as shown in figure 5a, the inventors found that the inclusion of the CL1 degradation signal in the 5'-half vector reduced truncated protein expression to undetectable levels without affecting full-length protein expression (Fig. 5a). Since differences in the tissue-specific expression of enzymes of the ubiquitination pathway that mediate CL1 degradation ³¹ may account for changes in CL1 efficacy, the inventors further evaluated the efficacy of the CL1 degron in the pig retina, which has a size and structure similar to human ^{19, 30, 39, 40} and is therefore an excellent pre-clinical large animal model to evaluate vector safety and efficiency.

To this aim, the inventors injected subretinally in Large White pigs AAV2/8 dual AAV hybrid AK vectors (of which the 5'-half vector included or not the CL1 sequence) encoding for *ABCA4* (dose of each vector/eye: 1 × 10¹¹ GC). Notably, the inventors found that the inclusion of the CL1 degradation signal in the 5'-half vector resulted in a significant reduction of truncated protein expression below the detection limit of the Western blot analysis without affecting full-length protein expression (Fig. 5b). Among the degradation signals tested in the 3'-half vector the inventors found that STOP codons did not affect truncated protein production. Differently, PB29 (either in a single or in three tandem copies) and Ubiquitin were all effective in reducing truncated protein expression. However, while Ubiquitin abolished also full-length protein expression, PB29 affected full-length protein production to a lesser extent (Fig. 6).

Among the degradation signals tested in the 3'-half vector the inventors identified three (PB29, 3xPB29 and ubiquitin) that reduced both the levels of truncated protein products and of full-length proteins (Fig. 6 and Tables 5 and 6).

**Table 5. Quantification of full-length ABCA4 relative to truncated protein expression from Western blot analysis of HEK293 cells infected with dual AAV hybrid vectors including miR target sites in the 5'-half vector.**

| **miR TARGET SITES** | **FULL-LENGTH ABCA4 / TRUNCATED PROTEIN** | |
|---|---|---|
| | **+SCRAMBLE** | **+miR** |
| 5'-miR-let7b + 3' | 1.2 ± 0,3 | 0,8 ± 0.3 |
| 5'-miR-204+124 + 3' | 1.8 ± 0.5 | 2.7 ± 0.9 |
| 5'-miR-26a + 3' | 1,9 ± 0,8 | 2.5 ± 1,1 |

| | | |
|---|---|---|
| Values represent mean ± s.e.m. of the ratios (from three independent experiments) between the intensity of full-length ABCA4 and truncated protein bands in the presence of either the corresponding mimic or a scramble mimic. Ratios in the presence of either the scramble or the corresponding mimic for each pair of vectors were compared using Student's ttest and no significant differences were found. | | |

**Table 6: Quantification of full-length ABCA4 and truncated protein expression from Western blot analysis of HEK293 cells infected with dual AAV hybrid vectors including degradation signals in the 3'-half vector.**

| **DEGRADATION SIGNALS** | **FULL-LENGTH ABCA4 / TRUNCATED PROTEIN** | |
|---|---|---|
| | **5'+3' NO DEGRADATION SIGNAL** | **5'+3' + DEGRADATIO N SIGNAL** |
| 3xSTOP | 5,9 ± 1,8 | 4.9 ± 1,1 |
| PB29 | | 5,3 ± 1,1 |
| 3xPB29 | | 1 ± 0,3 |
| ubiquitin | | 0,6 ± 0,2 |

| | | |
|---|---|---|
| Values represent mean ± s.e.m. of the ratios (from three independent experiments) between the intensity of the full-length ABCA4 and truncated protein bands from vectors either with or without the degradation signals. More details on the statistical analysis including specific statistical values can be found in the Statistical analysis paragraph of the Materials and Methods section | | |

### Subretinal administration of improved dual AAV vectors reduces lipofuscin accumulation in the Abca4-/- retina

Based on our findings improved dual AAV *hybrid-ABCA4* vectors should include homologous ITR2, the AK region of homology and the CL1. As *ABCA4* is expressed in both rod and cone photoreceptors in humans⁷⁰, the inventors identified a suitable promoter for *ABCA4* delivery by comparing the PR transduction properties of single AAV2/8 vectors encoding *EGFP* from either the human GRK1 (G protein-coupled receptor kinase 1) or IRBP (interphotoreceptor retinoid binding protein) promoters, which have been both described to drive high levels of combined rod and cone PR transduction in various species⁵³⁻⁵⁵. Taking advantage of the pig retinal architecture which include a streak-like region with a cone:rod = 1:3⁵⁶ similar to the human macula, the inventors injected subretinally 1×10¹¹GC/eye of either AAV2/8-GRK1- or IRBP-*EGFP* vectors in 3 month-old Large White pigs. Four weeks after the injection, the inventors analysed the corresponding retinal cryosections under a fluorescence microscope. EGFP fluorescence quantification in the PR cell layer (Fig. 10a-b) showed that both promoters give comparable levels of PR transduction (predominantly rods in this region). However, when the inventors counted the number of cones labelled with an antibody raised against cone arrestin (CAR)⁵⁷ that were also EGFP positive, they found higher although not statistically significant levels of cone PR transduction with the GRK1 promoter (Material, Fig. 10c-d). Based on this, the inventors included the GRK1 promoter in our improved dual AAV hybrid *ABCA4* vectors, and investigated their ability to both express *ABCA4* and decrease the abnormal content of A2E-containing autofluorescent lipofuscin material in the RPE of *Abca4-*/*-* mice. The inventors initially injected subretinally one month-old C57/BL6 mice with improved dual AAV vectors (dose of each vector/eye: 2×10⁹ GC) and found that 12 out of 24 (50%) injected eyes had detectable albeit variable levels of full-length ABCA4 protein by Western blot [Fig. 8a; ABCA4 protein levels in the ABCA4-positive eyes: 2,8 ± 0,7 a.u. (mean ± standard error of the mean)].

This is similar to our previous finding that a different version of the dual AAV platform resulted in 50% ABCA4-expressing eyes¹⁴. The inventors then injected 5.5 month-old pigmented *Abca4-*/- mice subretinally in the temporal region of the eye with the improved dual AAV vectors (dose of each vector/eye: 1.8×10⁹ GC). Three months later the inventors harvested the eyes and measured the levels of lipofuscin fluorescence (excitation: 560±40 nm; emission: 645±75) on retinal cryosections [in either the RPE alone or in RPE +outer segments (OS)] in the temporal region of the eye (Fig. 8b-c and Fig. 11). The inventors found that lipofuscin fluorescence intensity in this region of the eye was significantly higher in untreated *Abca4-*/*-* than in both *Abca4*+/- and -/- mice injected with the therapeutic dual AAV hybrid *ABCA4* vectors (Fig. 8b, c and Fig. 11). Then, using transmission electron microscopy the inventors counted the number of RPE lipofuscin granules. These were increased in 5.5-6-month old albino *Abca4-*/*-* mice injected with PBS compared to age-matched *Abca4*+/+ controls (Fig. 8d), at levels similar to those the inventors have independently measured in *Abca4-*/*-* mice either uninjected or injected with a control AAV vector (data not shown). The number of lipofuscin granules in *Abca4-*/*-* RPE was normalized 3 months post subretinal injection of improved dual AAV hybrid *ABCA4* vectors (dose of each vector/eye: 1×10⁹ GC, Fig. 8d).

### Improved dual AAV vectors are safe upon subretinal administration to the mouse and pig retina

To investigate the safety of improved dual AAV2/8 hybrid *ABCA4* vectors, the inventors injected them subretinally in both wild-type C57BL/6 mice and Large White pigs (dose of each vector/eye: 3×10⁹ and 1×10¹¹GC, respectively). One month post-injection the inventors measured retinal electrical activity by Ganzfeld electroretinogram (ERG) and found that both the a- and b-wave amplitudes were not significantly different between mouse eyes that were injected with dual AAV hybrid *ABCA4* vectors and eyes injected with either negative control AAV vectors or PBS (Fig. 9a and Material, Fig. 12a). Similarly, the b-wave amplitude in both scotopic, photopic, maximum response and flicker ERG tests was comparable in pig eyes that were injected with dual AAV hybrid *ABCA4* vectors to those of control eyes injected with PBS (Fig. 9b and Material, Fig. 12b).

### DISCUSSION

AAV restricted packaging capacity represents one of the main obstacles to the widespread application of AAV for gene therapy of IRDs. However, recently, several groups have independently reported that dual AAV vectors effectively expand AAV cargo capacity in both the mouse and pig retina ^{14, 17, 19, 41} thus extending AAV applicability to IRDs due to mutations in genes that would not fit in a single canonical AAV vector. Here the inventors set-up to overcome some limitations associated with the use of dual AAV vectors, namely their relatively low efficiency when compared to a single vector, and the production of truncated proteins which may raise safety concerns.

Strategies aiming at increasing dual AAV genome tail-to-head concatemerization should in theory increase the levels of full-length and reduce those of truncated proteins from free single half-vectors. The inventors set to improve tail-to-head dual AAV hybrid genome concatemerization by including either optimal regions of homology or heterologous ITR. In a side-by-side evaluation of previously described regions of homology, the inventors have found that the AP1 and AP2 sequences recently published by Lostal et al. ²⁰ and the AK sequence from the Fl phage ¹⁴ drive overall similar levels of protein expression *in vitro* with dual AAV hybrid AK vectors driving more consistent ABCA4 expression in the mouse retina. Independently, the availability of different regions of homology is useful to direct proper concatemerization of triple AAV vectors to further expand AAV cargo capacity ^{20, 42}. Heterologous ITR2 and ITR5 have been successfully included in dual ^{24, 25} and triple ⁴² AAV vectors. The inventors found that the yields of AAV vectors with heterologous ITR2 and ITR5 are lower than those with homologous ITR2. The inventors also detected less vector genomes with heterologous ITR when the inventors probe their ITR2 than when the inventors probe a different region of their genome. As the inventors show that Rep5 interferes with production of vectors with ITR2, this suggests anomalies at the level of ITR2 included in AAV vectors with heterologous ITR, which are produced in the presence of Rep5, but not in AAV vectors with homologous ITR2, which are produced only in the presence of Rep2 and that showed similar titres whether the inventors probe ITR2 or a different region of the genome. These results partly differ from those previously reported where dual AAV vectors with heterologous ITR2 and ITR5 had higher transduction efficiency than vectors with homologous ITRs and apparently no production issues ^{24, 25}. Besides the different packaging constructs and production protocols, in this study the inventors used dual AAV hybrid vectors which included regions of homology between the two half-vectors as opposed to the trans-splicing system used in the previous reports which simply relies on the ITR for concatemerization ^{24, 25}. As in dual AAV hybrid vectors the reconstitution of the full-length gene is mainly mediated by the region of homology included in the vectors ¹⁶ which direct concatemer formation, this may account for the smaller increase in transgene expression the inventors observed with vectors with heterologous ITR compared to the previous studies that used trans-splicing vectors ^{24, 25}. In addition, the inventors may have overestimated the efficiency of the vectors with heterologous ITR as the inventors used them based on a titre calculated on ITR2 which is 3-6-fold lower than the one calculated on the transgenic sequence for MYO7A- and ABCA4-expressing vectors, respectively. As both titres calculated on ITR2 and on the transgenic sequence are similar between the corresponding dual AAV vectors with homologous ITR2, the inventors have used them at a 3-6-fold lower volume than those with the heterologous ITR2 and ITR5. This may explain the apparently higher levels of both full-length and truncated protein products from dual AAV vector with heterologous than with homologous ITR.

In the inventors' previous studies the inventors did not observe signs of local toxicity up to 8 months after subretinal administration of dual AAV vectors ¹⁴, however, the production of truncated protein products from single half-vectors of dual AAV might raise safety concerns.

The inclusion of miR target sites in the transcript of a gene has been shown to be an effective strategy to restrict transgene expression in various tissues, including the retina ³⁰. However *in vitro* the inventors achieved a partial reduction of truncated protein production only when the inventors included target sites for miR-204+124 and 26a. Indeed, features of the mRNA external to the miR target sites may affect the efficiency of the silencing ^{43, 44}. Along this line, since the truncated protein products that derive from the 5'-half is produced from a vector that is not endowed with a canonical polyadenilation signal, it may be possible that the resulting mRNA can not undergo an efficient miR-mediated silencing. Importantly, the inventors achieved complete degradation of the truncated protein product from the 5'-half vector by inclusion of the CL1 degron. The inventors showed that this signal is effective both *in vitro* and in the pig retina, indicating that the enzymes of the degradative pathway required for CL1 activity are expressed in various cell types. As the truncated protein product from the 3'-half vector is less abundant than that produced by the 5'-half vector (Fig. 6), its presence should raise less safety concerns. Data presented here in the mouse and pig retina support the safety of improved dual AAV vectors.

Notably, the inventors found that subretinal administration of improved dual AAV vectors, under the control of the GRK1 promoter, which provides high levels of combined rod and cone transduction, results in effective ABCA4 delivery in mice, although at variable levels. This could be due to both the inherent variability of the subretinal injection in the small murine eye and the overall lower efficacy of the dual AAV system compared to a single AAV vector¹⁴.

Despite this variability, the inventors found that dual AAV mediated ABCA4 delivery results in significant lipofuscin reduction in the *Abca4-*/*-* retina suggesting that a wide range of transgene expression levels can similarly contribute to therapeutic efficacy. This was observed using two independent techniques, however, more pronounced improvement of the phenotype was observed when the inventors dissected and analysed the AAV transduced area of the retina that indeed showed normalization of the number of lipofuscin granules. In conclusion, the invention provides multiple vectors with improved features suitable for clinical application, in particular for the therapy of retinal diseases. In addition, the invention improves the safety and efficacy of multiple vectors which further expand cargo capacity ^{20, 42}.

### REFERENCES

1. Trapani, I, et al (2014). Progress in retinal and eye research 43: 108-128.
2. Boye, SE, Boye, SL, Lewin, AS, and Hauswirth, WW (2013). Molecular therapy : the journal of the American Society of Gene Therapy 21: 509-519.
3. Bainbridge, JW, et al. (2008). The New England journal of medicine 358: 2231-2239.
4. Maguire, AM, et al. (2009). Lancet 374: 1597-1605.
5. Maguire, AM, et al. (2008). The New England journal of medicine 358: 2240-2248.
6. Cideciyan, AV, et al. (2009). Human gene therapy 20: 999-1004.
7. Simonelli, F, et al. (2010). Molecular therapy : the journal of the American Society of Gene Therapy 18: 643-650.
8. Allikmets, R, et al. (1997). Nature genetics 15: 236-246.
9. Molday, RS, and Zhang, K (2010). Progress in lipid research 49: 476-492.
10. Millan, JM, et al. (2011). Journal of ophthalmology 2011: 417217.
11. Hasson, T, et al. (1995). PNAS 92: 9815-9819.
12. Liu, X, Ondek, B, and Williams, DS (1998). Nature genetics 19: 117-118.
13. Gibbs, D, et al. (2010). Investigative ophthalmology & visual science 51: 1130-1135.
14. Trapani, I, Colella, P, Sommella, A, Iodice, C, Cesi, G, de Simone, S, et al. (2014). Effective delivery of large genes to the retina by dual AAV vectors. EMBO molecular medicine 6: 194-211.
15. Duan, D, Yue, Y, and Engelhardt, JF (2001). Molecular therapy : the journal of the American Society of Gene Therapy 4: 383-391.
16. Ghosh, A, Yue, Y, Lai, Y, and Duan, D (2008). Molecular therapy : the journal of the American Society of Gene Therapy 16: 124-130.
17. Dyka, FM, et al., (2014). Human gene therapy methods 25: 166-177.
18. Lopes, VS, et al. (2013). Gene Ther.
19. Colella, P, et al. (2014). Gene Ther 21: 450-456.
20. Lostal, W, Kodippili, K, Yue, Y, and Duan, D (2014). Human gene therapy 25: 552-562.
21. Flotte, TR, et al. (1993). The Journal of biological chemistry 268: 3781-3790.
22. Ghosh, A, Yue, Y, and Duan, D (2011). Human gene therapy 22: 77-83.
23. Chiorini, JA, et al., (1999). Journal of virology 73: 1309-1319.
24. Yan, Z, Zak, R, Zhang, Y, and Engelhardt, JF (2005). Journal of virology 79: 364-379.
25. Yan, Z, et al. (2007). Human gene therapy 18: 81-87.
26. Karali, et al. (2010). BMC genomics 11: 715.
27. Kutty, RK, et al. (2010). Molecular vision 16: 1475-1486.
28. Ragusa, M, et al. (2013). Molecular vision 19: 430-440.
29. Sundermeier, TR, and Palczewski, K (2012). Cellular and molecular life sciences : CMLS 69: 2739-2750.
30. Karali, M, et al. (2011). PloS one 6: e22166.
31. Gilon, T, Chomsky, O, and Kulka, RG (1998). The EMBO journal 17: 2759-2766.
32. Bence, NF, Sampat, RM, and Kopito, RR (2001). Science 292: 1552-1555.
33. Bachmair, A, Finley, D, and Varshavsky, A (1986). Science 234: 179-186.
34. Johnson, ES, et al., (1992). The EMBO journal 11: 497-505.
35. Sadis, S, et al., (1995). Molecular and cellular biology 15: 4086-4094.
36. Chiorini, JA, Afione, S, and Kotin, RM (1999). Journal of virology 73: 4293-4298.
37. Tian, W, et al. (2012). PloS one 7: e29551.
38. Wang, Z (2011). Methods in molecular biology 676: 211-223.
39. Mussolino, C, et al. (2011). Gene Ther 18: 637-645.
40. Hendrickson, A, and Hicks, D (2002). Experimental eye research 74: 435-444.
41. Reich, SJ, et al. (2003). Human gene therapy 14: 37-44.
42. Koo, T, et al., (2014). Human gene therapy 25: 98-108.
43. Walters, RW, Bradrick, SS, and Gromeier, M (2010). Rna 16: 239-250.
44. Ricci, EP, et al. (2011). Nucleic acids research 39: 5215-5231.
45. Auricchio, et al. (2001). Human molecular genetics 10: 3075-3081.
46. Gao, G, et al. (2000). Human gene therapy 11: 2079-2091.
47. Young, JE, et al., (2003). Investigative ophthalmology & visual science 44: 4076-4085.
48. Doria, M, et al., (2013). Human gene therapy methods 24: 392-398.
49. Zhang, Y, et al., (2000). Journal of virology 74: 8003-8010.
50. Drittanti, L, et al., (2000). Gene Ther 7: 924-929.
51. Gargiulo, S, et al. (2012). ILAR journal / National Research Council, Institute of Laboratory Animal Resources 53: E70-81.
52. Liang, FQ, et al., (2001). Methods in molecular medicine 47: 125-139.
53. Beltran, et al. (2012) Proc. Natl. Acad. Sci. U. S. A., 109, 2132-2137.
54. Boye, S.E., et al. (2012) Hum. Gene Ther., 23, 1101-1115.
55. Khani, S.C., et al., (2007) Invest. Ophthalmol. Vis. Sci., 48, 3954-3961.
56. Chandler, M.J., et al., (1999) Vet. Ophthalmol., 2, 179-184.
57. Li, A., Zhu, X. and Craft, C.M. (2002) Invest. Ophthalmol. Vis. Sci., 43, 1375-1383.
58. Allocca, M., et al. (2008) J. Clin. Invest., 118, 1955-1964.
59. Parish, C.A., et al., (1998) Proc. Natl. Acad. Sci. U. S. A., 95, 14609-14613.
60. Ben-Shabat, S., et al., (2002) J. Biol. Chem., 277, 7183-7190.
61. Gargiulo, S., et al., (2012) ILAR J, 53, E70-81.
62. Liang, F.Q., et al., (2001) Methods Mol. Med., 47, 125-139.
63. Gargiulo, A., et al. (2009) Mol. Ther., 17, 1347-1354.
64. Manfredi, A., et al. (2013) Hum. Gene Ther., 24, 982-992.
65. Venables VN and Ripley BD. (2002) Modern Applied Statistics with S. Springer Science+Business Media, New York, USA.
66. Li, A., Zhu, X., Brown, B. and Craft, C.M. (2003) Adv. Exp. Med. Biol., 533, 361-368.
67. Li, A., et al. (2003) Invest. Ophthalmol. Vis. Sci., 44, 996-1007.
68. Allocca, M., et al. (2011) Invest. Ophthalmol. Vis. Sci., 52, 5713-5719.
69. Testa, F., et al. (2011) Invest. Ophthalmol. Vis. Sci., 52, 5618-5624.
70. Molday, L.L., Rabin, A.R. and Molday, R.S. (2000) Nat. Genet., 25, 257-258.
71. Sparrow, J.R., Wu, Y., Nagasaki, T., Yoon, K.D., Yamamoto, K. and Zhou, J. (2010) Photochem Photobiol Sci, 9, 1480-1489.
72. Sparrow, J.R. and Duncker, T. (2014) J Clin Med, 3, 1302-1321.
73. Finnemann, S.C., Leung, L.W. and Rodriguez-Boulan, E. (2002) Proc. Natl. Acad. Sci. U. S. A., 99, 3842-3847.
74. Secondi, R., Kong, J., Blonska, A.M., Staurenghi, G. and Sparrow, J.R. (2012) Invest. Ophthalmol. Vis. Sci., 53, 5190-5197.
75. Delori, F.C., Dorey, C.K., Staurenghi, G., Arend, O., Goger, D.G. and Weiter, J.J. (1995) Invest. Ophthalmol. Vis. Sci., 36, 718-729.

## Claims

1. An AAV multiple vector system to express the coding sequence of a gene of interest in a cell, said coding sequence comprising a first portion and a second portion, said vector system comprising:
a) a first vector comprising:
- said first portion of said coding sequence (CDS1),
- a first reconstitution sequence; and
b) a second vector comprising:
- said second portion of said coding sequence (CDS2),
- a second reconstitution sequence,
wherein said first and second reconstitution sequences are selected from the group of:
i] the first reconstitution sequence consists of the 3' end of said first portion of the coding sequence and the second reconstitution sequence consists of the 5'end of said second portion of the coding sequence, said first and second reconstitution sequences being overlapping sequences; or
ii] the first reconstitution sequence comprises a splicing donor signal (SD) and the second reconstitution sequence comprises a splicing acceptor signal (SA), optionally each one of first and second reconstitution sequence further comprises a recombinogenic sequence,
**characterized by** the fact that either one or both of the first and second vector further comprises a nucleotide sequence of a degradation signal, said sequence being located in case of i) at the 3' end of the CDS1 and/or at the 5' end of the CDS2 and in case of ii) in 3' position relative to the SD and/or in 5' position relative to the SA, wherein
- the recombinogenic sequence is selected from the group consisting of:
GGGATTTTGCCGATTTCGGCCTATTGGTTAAAAAATGAGCTGATTTAACAAAAATTT AACGCGAATTTTAACAAAAT (SEQ ID No. 22, AK) or
GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO. 25 (AP2), and SEQ ID NO. 26 (AP) and
- the nucleotide sequence of the degradation signal comprises or consists of a sequence encoding SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16), or the nucleotide sequence of a degradation signal comprises or consists of SEQ ID No. 16.

2. The AAV multiple vector system according to claim 1, wherein both of the first and second vector further comprise said nucleotide sequence of a degradation signal, wherein the nucleotide sequence of the degradation signal in the first vector is identical to or differs from that in the second vector, or wherein the first reconstitution sequence comprises a splicing donor signal (SD) and a recombinogenic region in 3' position relative to said SD, the second reconstitution sequence comprises a splicing acceptor signal (SA) and a recombinogenic sequence in 5' position relative to the SA; wherein said nucleotide sequence of a degradation signal is localized at the 5' end and/or at the 3' end of the nucleotide sequence of the recombinogenic region of either one or both of the first and second vector.

3. The AAV multiple vector system according to any one of previous claims, wherein the first vector further comprises a promoter sequence operably linked to the 5'end portion of said first portion of the coding sequence (CDS1).

4. The AAV multiple vector system according to any one of previous claims, wherein both of the first vector and the second vector further comprise a 5'-terminal repeat (5'-TR) nucleotide sequence and a 3'-terminal repeat (3'-TR) nucleotide sequence, preferably the 5'-TR is a 5'-inverted terminal repeat (5'-ITR) nucleotide sequence and the 3'-TR is a 3'-inverted terminal repeat (3'-ITR) nucleotide sequence, preferably the ITRs derive from the same virus serotype or from different virus serotypes.

5. The AAV multiple vector system according to any one of previous claim, wherein the splicing donor signal comprises or consists of a sequence that is at least 70%, 75%, 80%, 85%, 90%, 95% or 100 % identical to
GTAAGTATCAAGGTTACAAGACAGGTTTAAGGAGACCAATAGAAACTGGGCTTGTC GAGACAGAGAAGACTCTTGCGTTTCT (SEQ ID No. 27), or wherein the splicing acceptor signal comprises or consists of a sequence that is at least 70%, 75%, 80%, 85%, 90%, 95% or 100 % identical to
GATAGGCACCTATTGGTCTTACTGACATCCACTTTGCCTTTCTCTCCACAG (SEQ ID No. 28)

6. The AAV multiple vector system according to any one of previous claim wherein the coding sequence is the coding sequence of a gene selected from the group consisting of: ABCA4, MYO7A, CEP290, CDH23, EYS, PCDH15, CACNA1, SNRNP200, RP1, PRPF8, RP1L1, ALMS1, USH2A, GPR98, HMCN1, preferably the coding sequence is the coding sequence of a gene selected from the group consisting of: DMD, CFTR, F8 and DYSF.

7. The AAV multiple vector system according to any one of previous claims, wherein the first vector does not comprise a poly-adenylation signal nucleotide sequence.

8. The AAV multiple vector system according to any one of previous claims comprising:
a) a first vector comprising in a 5'-3' direction:
- a 5'-inverted terminal repeat (5'-ITR) sequence;
- a promoter sequence;
- a 5' end portion of a coding sequence of a gene of interest (CDS1), said 5'end portion being operably linked to and under control of said promoter;
- a nucleotide sequence of a splicing donor signal;
- a nucleotide sequence of a recombinogenic region; and
- a 3'-inverted terminal repeat (3'-ITR) sequence; and
b) a second vector comprising in a 5'-3' direction:
- a 5'-inverted terminal repeat (5'-ITR) sequence;
- a nucleotide sequence of a recombinogenic region;
- a nucleotide sequence of a splicing acceptor signal;
- the 3'end of the coding sequence (CDS2);
- a poly-adenylation signal nucleotide sequence; and
- a 3'-inverted terminal repeat (3'-ITR) sequence,
**characterized by** further comprising a nucleotide sequence of a degradation signal, said sequence being localized at 5' end or 3' end of the nucleotide sequence of the recombinogenic region of either one or both of the first and second vector, wherein
- the recombinogenic sequence is selected from the group consisting of:
GGGATTTTGCCGATTTCGGCCTATTGGTTAAAAAATGAGCTGATTTAACAAAAATTT AACGCGAATTTTAACAAAAT (SEQ ID No. 22, AK) or
GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO. 25 (AP2) and SEQ ID NO. 26 (AP) and
- the nucleotide sequence of the degradation signal comprises or consists of a sequence encoding SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16), or the nucleotide sequence of a degradation signal comprises or consists of SEQ ID No. 16.

9. The AAV multiple vector system according to any one of previous claims further comprising a third vector comprising a third portion of said coding sequence (CDS3) and a reconstitution sequence, wherein the second vector comprises two reconstitution sequences, each reconstitution sequence located at each end of CDS2, preferably the third vector further comprises at least one nucleotide sequence of a degradation signal, preferably the second vector further comprises a poly-adenylation signal nucleotide sequence linked to the 3'end portion of said coding sequence (CDS2).

10. A host cell comprising the AAV multiple vector system according to any one of previous claim.

11. The AAV multiple vector system according to any one of claim 1 to 9 or the host cell according to claim 10 for medical use, preferably for use in gene therapy, preferably for use in the treatment and/or prevention of a pathology or disease **characterized by** a retinal degeneration, preferably the retinal degeneration is inherited, preferably the pathology or disease is selected from the group consisting of: retinitis pigmentosa (RP), Leber congenital amaurosis (LCA), Stargardt disease (STGD), Usher disease (USH), Alstrom syndrome, congenital stationary night blindness (CSNB), macular dystrophy, occult macular dystrophy, a disease caused by a mutation in the *ABCA4* gene, preferably for use in the prevention and/or treatment of Duchenne muscular dystrophy, cystic fibrosis, hemophilia A and dysferlinopathies.

12. A pharmaceutical composition comprising the AAV multiple vector system according to any one of claim 1 to 9 or the host cell according to claim 10 and pharmaceutically acceptable vehicle.

13. A nucleotide sequence of a degradation signal that comprises or consists of a sequence encoding SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16), or that comprises or consists of SEQ ID No. 16 in a AAV multiple vector system for use in a method to decrease expression of a protein in truncated form or for use in a method for decreasing expression of a protein in truncated form comprising inserting said nucleotide sequence of a degradation signal in one or more vector of a AAV multiple vector system.

## Patentansprüche

1. AAV-Mehrvektorsystem zur Expression der Codiersequenz eines Gens von Interesse in einer Zelle, wobei die Codiersequenz einen ersten Teil und einen zweiten Teil umfasst, wobei das Vektorsystem Folgendes umfasst:
a) einen ersten Vektor umfassend:
- den ersten Teil der Codiersequenz (CDS1),
- eine erste Rekonstitutionssequenz; und
b) einen zweiten Vektor umfassend:
- den zweiten Teil der Codiersequenz (CDS2),
- eine zweite Rekonstitutionssequenz,
wobei die erste und die zweite Rekonstitutionssequenz aus der folgenden Gruppe ausgewählt sind:
i] die erste Rekonstitutionssequenz besteht aus dem 3'-Ende des ersten Teils der Codiersequenz, und die zweite Rekonstitutionssequenz besteht aus dem 5'-Ende des zweiten Teils der Codiersequenz, wobei es sich bei der ersten und der zweiten Rekonstitutionssequenz um überlappende Sequenzen handelt; oder
ii] die erste Rekonstitutionssequenz umfasst ein Spleißdonorsignal (SD) und die zweite Rekonstitutionssequenz ein Spleißakzeptorsignal (SA), gegebenenfalls umfassen die erste und die zweite Rekonstitutionssequenz jeweils ferner eine rekombinogene Sequenz, **gekennzeichnet durch** die Tatsache, dass der erste oder bzw. und der zweite Vektor ferner eine Nukleotidsequenz eines Abbausignals umfasst bzw. umfassen, wobei die Sequenz im Fall von i) am 3'-Ende der CDS1 und/oder am 5'-Ende der CDS2 und im Fall von ii) in 3'-Position relativ zum SD und/oder in 5'-Position relativ zum SA liegt, wobei
- die rekombinogene Sequenz ausgewählt ist aus der Gruppe bestehend aus:
GGGATTTTGCCGATTTCGGCCTATTGGTTAAAAAATGAGCTGATTTAACAAAAAT TTAACGCGAATTTTAACAAAAT (SEQ ID Nr. 22, AK) oder GGGATTTTTCCGATTTCGGCCTATTGGTTAAAAAATGAGCTGATTTAACAAAAAT TTAACGCGAATTTTAACAAAAT (SEQ ID NO. 23, AK), SEQ ID NO. 24 (AP1), SEQ ID NO. 25 (AP2) und SEQ ID NO. 26 (AP) und
- die Nukleotidsequenz des Abbausignals eine Sequenz, die SEQ ID Nr. 1 (CL1), SEQ ID Nr. 2 (CL2), SEQ ID Nr. 3 (CL6), SEQ ID Nr. 4 (CL9), SEQ ID Nr. 5 (CL10), SEQ ID Nr. 6 (CL11), SEQ ID Nr. 7 (CL12), SEQ ID Nr. 8 (CL15), SEQ ID Nr. 9 (CL16) codiert, umfasst oder daraus besteht oder die Nukleotidsequenz eines Abbausignals SEQ ID Nr. 16 umfasst oder daraus besteht.

2. AAV-Mehrvektorsystem nach Anspruch 1, wobei sowohl der erste als auch der zweite Vektor ferner die Nukleotidsequenz eines Abbausignals umfassen, wobei die Nukleotidsequenz des Abbausignals im ersten Vektor mit der im zweiten Vektor identisch oder von dieser verschieden ist oder wobei die erste Rekonstitutionssequenz ein Spleißdonorsignal (SD) und eine rekombinogene Region in 3'-Position relativ zu dem SD umfasst, die zweite Rekonstitutionssequenz ein Spleißakzeptorsignal (SA) und eine rekombinogene Sequenz in 5'-Position relativ zum SA umfasst; wobei die Nukleotidsequenz eines Abbausignals am 5'-Ende und/oder am 3'-Ende der Nukleotidsequenz der rekombinogenen Region des ersten oder bzw. und des zweiten Vektors lokalisiert ist.

3. AAV-Mehrvektorsystem nach einem der vorherigen Ansprüche, wobei der erste Vektor ferner eine Promotorsequenz in operativer Verknüpfung mit dem 5'-Ende-Teil des ersten Teils der Codiersequenz (CDS1) umfasst.

4. AAV-Mehrvektorsystem nach einem der vorherigen Ansprüche, wobei sowohl der erste Vektor als auch der zweite Vektor ferner eine 5'-TR(5'-terminal repeat)-Nukleotidsequenz und eine 3'-TR(3'-terminal repeat)-Nukleotidsequenz umfassen, es sich vorzugsweise bei der 5'-TR um eine 5'-ITR(5'-inverted terminal repeat)-Nukleotidsequenz und bei der 3'-TR um eine 3'-ITR(3'-inverted terminal repeat)-Nukleotidsequenz handelt, vorzugsweise die ITRs aus dem gleichen Virus-Serotyp oder aus unterschiedlichen Virus-Serotypen stammen.

5. AAV-Mehrvektorsystem nach einem der vorherigen Ansprüche, wobei das Spleißdonorsignal eine Sequenz, die zu wenigstens 70%, 75%, 80%, 85%, 90%, 95% oder 100% mit GTAAGTATCAAGGTTACAAGACAGGTTTAAGGAGACCAATAGAAACTGGGCTTGT CGAGACAGAGAAGACTCTTGCGTTTCT (SEQ ID Nr. 27) identisch ist, umfasst bzw. daraus besteht oder wobei das Spleißakzeptorsignal eine Sequenz, die zu wenigstens 70%, 75%, 80%, 85%, 90%, 95% oder 100% mit GATAGGCACCTATTGGTCTTACTGACATCCACTTTGCCTTTCTCTCCACAG(SEQ ID Nr. 28) identisch ist, umfasst bzw. daraus besteht.

6. AAV-Mehrvektorsystem nach einem der vorherigen Ansprüche, wobei es sich bei der Codiersequenz um die Codiersequenz eines Gens, das ausgewählt ist aus der Gruppe bestehend aus ABCA4, MYO7A, CEP290, CDH23, EYS, PCDH15, CACNA1, SNRNP200, RP1, PRPF8, RPILI, ALMS1, USH2A, GPR98, HMCN1, vorzugsweise um die um die Codiersequenz eines Gens, das ausgewählt ist aus der Gruppe bestehend aus DMD, CFTR, F8 und DYSF, handelt.

7. AAV-Mehrvektorsystem nach einem der vorherigen Ansprüche, wobei der erste Vektor keine Polyadenylierungssignal-Nukleotidsequenz umfasst.

8. AAV-Mehrvektorsystem nach einem der vorherigen Ansprüche, umfassend:
a) einen ersten Vektor, der in 5'-3'-Richtung Folgendes umfasst:
- eine 5'-ITR(5'-inverted terminal repeat)-Sequenz;
- eine Promotorsequenz;
- einen 5'-Ende-Teil einer Codiersequenz eines Gens von Interesse (CDS1), wobei der 5'-Ende-Teil in operativer Verknüpfung mit dem Promotor und unter dessen Kontrolle steht;
- eine Nukleotidsequenz eines Spleißdonorsignals;
- eine Nukleotidsequenz einer rekombinogenen Region; und
- eine 3'-ITR(3'-inverted terminal repeat)-Sequenz; und
b) einen zweiten Vektor, der in 5'-3'-Richtung Folgendes umfasst:
- eine 5'-ITR(5'-inverted terminal repeat)-Sequenz;
- eine Nukleotidsequenz einer rekombinogenen Region;
- eine Nukleotidsequenz eines Spleißakzeptorsignals;
- das 3'-Ende der Codiersequenz (CDS2);
- eine Polyadenylierungssignal-Nukleotidsequenz; und
- eine 3'-ITR(3'-inverted terminal repeat)-Sequenz, **gekennzeichnet durch** weiteres Umfassen einer Nukleotidsequenz eines Abbausignals, wobei die Sequenz am 5'-Ende oder 3'-Ende der Nukleotidsequenz der rekombinogenen Region des ersten oder bzw. und des zweiten Vektors lokalisiert ist, wobei
- die rekombinogene Sequenz ausgewählt ist aus der Gruppe bestehend aus:
GGGATTTTGCCGATTTCGGCCTATTGGTTAAAAAATGAGCTGATTTAACAAAAAT TTAACGCGAATTTTAACAAAAT (SEQ ID Nr. 22, AK) oder GGGATTTTTCCGATTTCGGCCTATTGGTTAAAAAATGAGCTGATTTAACAAAAAT TTAACGCGAATTTTAACAAAAT (SEQ ID NO. 23, AK), SEQ ID NO. 24 (AP1), SEQ ID NO. 25 (AP2) und SEQ ID NO. 26 (AP) und
- die Nukleotidsequenz des Abbausignals eine Sequenz, die SEQ ID Nr. 1 (CL1), SEQ ID Nr. 2 (CL2), SEQ ID Nr. 3 (CL6), SEQ ID Nr. 4 (CL9), SEQ ID Nr. 5 (CL10), SEQ ID Nr. 6 (CL11), SEQ ID Nr. 7 (CL12), SEQ ID Nr. 8 (CL15), SEQ ID Nr. 9 (CL16) codiert, umfasst oder daraus besteht oder die Nukleotidsequenz eines Abbausignals SEQ ID Nr. 16 umfasst oder daraus besteht.

9. AAV-Mehrvektorsystem nach einem der vorherigen Ansprüche, ferner umfassend einen dritten Vektor, der einen dritten Teil der Codiersequenz (CDS3) und eine Rekonstitutionssequenz umfasst, wobei der zweite Vektor zwei Rekonstitutionssequenzen umfasst, wobei die Rekonstitutionssequenzen jeweils an einem Ende von CDS2 liegen, der dritte Vektor vorzugsweise ferner wenigstens eine Nukleotidsequenz eines Abbausignals umfasst, der zweite Vektor vorzugsweise ferner eine mit dem 3'-Ende-Teil der Codiersequenz (CDS2) verknüpfte Polyadenylierungssignal-Nukleotidsequenz umfasst.

10. Wirtszelle, umfassend das AAV-Mehrvektorsystem nach einem der vorherigen Ansprüche.

11. AAV-Mehrvektorsystem nach einem der Ansprüche 1 bis 9 oder Wirtszelle nach Anspruch 10 zur medizinischen Verwendung, vorzugsweise zur Verwendung bei der Gentherapie, vorzugsweise zur Verwendung bei der Behandlung und/oder Vorbeugung eines Krankheitsbilds bzw. einer Krankheit, das bzw. die durch eine Netzhautdegeneration gekennzeichnet ist, vorzugsweise die Netzhautdegeneration vererbt ist, vorzugsweise das Krankheitsbild bzw. die Krankheit ausgewählt ist aus der Gruppe bestehend aus: Retinitis pigmentosa (RP), Leberscher kongenitaler Amaurose (LCA), Morbus Stargardt (STGD), Usher-Syndrom (USH), Alström-Syndrom, kongenitaler stationärer Nachtblindheit (CSNB), Makuladystrophie, okkulter Makuladystrophie, einer Krankheit, die durch eine Mutation im ABCA4-Gen verursacht wird, vorzugsweise zur Verwendung bei der Vorbeugung und/oder Behandlung von Duchenne-Muskeldystrophie, Mukoviszidose, Hämophilie A und Dysferlinopathien.

12. Pharmazeutische Zusammensetzung, umfassend das AAV-Mehrvektorsystem nach einem der Ansprüche 1 bis 9 oder die Wirtszelle nach Anspruch 10 und pharmazeutisch unbedenkliches Vehikel.

13. Nukleotidsequenz eines Abbausignals, die eine Sequenz, die SEQ ID Nr. 1 (CL1), SEQ ID Nr. 2 (CL2), SEQ ID Nr. 3 (CL6), SEQ ID Nr. 4 (CL9), SEQ ID Nr. 5 (CL10), SEQ ID Nr. 6 (CL11), SEQ ID Nr. 7 (CL12), SEQ ID Nr. 8 (CL15), SEQ ID Nr. 9 (CL16) codiert, umfasst oder daraus besteht oder die SEQ ID Nr. 16 umfasst oder daraus besteht, in einem AAV-Mehrvektorsystem zur Verwendung bei einem Verfahren zum Senken der Expression eines Proteins in verkürzter Form oder zur Verwendung bei einem Verfahren für sinkende Expression eines Proteins in verkürzter Form, umfassend Inserieren der Nukleotidsequenz eines Abbausignals in einem Vektor oder mehr eines AAV-Mehrvektorsystems.

## Revendications

1. Système de vecteur multiple AAV pour exprimer la séquence codante d'un gène d'intérêt dans une cellule, ladite séquence codante comprenant une première partie et une deuxième partie, ledit système de vecteur comprenant :
a) un premier vecteur comprenant :
- ladite première partie de ladite séquence codante (CDS1),
- une première séquence de reconstitution ; et
b) un deuxième vecteur comprenant :
- ladite deuxième partie de ladite séquence codante (CDS2),
- une deuxième séquence de reconstitution,
lesdites première et deuxième séquences de reconstitution étant choisies dans le groupe composé de
i) la première séquence de reconstitution constituée de la terminaison 3' de ladite première partie de la séquence codante et la deuxième séquence de reconstitution constituée de la terminaison 5' de ladite deuxième partie de la séquence codante, lesdites première et deuxième séquences de reconstitution étant des séquences chevauchantes ; ou
ii) la première séquence de reconstitution comprend un signal donneur d'épissage (SD) et la deuxième séquence de reconstitution comprend un signal accepteur d'épissage (SA), éventuellement chacune parmi la première et la deuxième séquence de reconstitution comprend en outre une séquence recombinogénique, **caractérisé par le fait que** soit l'un soit les deux parmi le premier et le deuxième vecteur comprend/comprennent en outre une séquence nucléotidique d'un signal de dégradation, ladite séquence étant localisée dans le cas de i) au niveau de la terminaison 3' de la CDS1 et/ou au niveau de la terminaison 5' de la CDS2 et dans le cas de ii) en position 3' par rapport au SD et/ou en position 5' par rapport au SA,
- la séquence recombinogénique étant choisie dans le groupe constitué par :
GGGATTTTGCCGATTTCGGCCTATTGGTTAAAAAATGAGCTGATTTAACAAAAATTT AACGCGAATTTTAACAAAAT (SEQ ID No. 22, AK) ou
GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO. 25 (AP2), et SEQ ID NO. 26 (AP) ^{et}
- la séquence nucléotidique du signal de dégradation comprenant ou étant constituée d'une séquence codant pour
SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16),
ou la séquence nucléotidique d'un signal de dégradation comprenant ou étant constituée de la SEQ ID NO: 16.

2. Système de vecteur multiple AAV selon la revendication 1, à la fois le premier et le deuxième vecteur comprenant en outre ladite séquence nucléotidique d'un signal de dégradation, la séquence nucléotidique du signal de dégradation dans le premier vecteur étant identique ou étant différente de celle dans le deuxième vecteur, ou la première séquence de reconstitution comprenant un signal donneur d'épissage (SD) et une région recombinogénique en position 3' par rapport audit SD, la deuxième séquence de reconstitution comprenant un signal accepteur d'épissage (SA) et une séquence recombinogénique en position 5' par rapport au SA ; ladite séquence nucléotidique d'un signal de dégradation étant localisée au niveau de la terminaison 5' et/ou au niveau de la terminaison 3' de la séquence nucléotidique de la région recombinogénique de l'un ou des deux parmi le premier et le deuxième vecteur.

3. Système de vecteur multiple AAV selon l'une quelconque des revendications précédentes, le premier vecteur comprenant en outre une séquence promoteur liée de manière fonctionnelle à la partie de terminaison 5' de ladite première partie de la séquence codante (CDS1).

4. Système de vecteur multiple AAV selon l'une quelconque des revendications précédentes, à la fois le premier vecteur et le deuxième vecteur comprenant en outre une séquence nucléotidique de répétition 5'-terminale (5'-TR) et une séquence nucléotidique de répétition 3'-terminale (3'-TR), préférablement la 5'-TR étant une séquence nucléotidique de répétition 5'-terminale inversée (5'-ITR) et la 3'-TR étant une séquence nucléotidique de répétition 3'-terminale inversée (3'-ITR), préférablement les ITR étant issues du même sérotype de virus ou de différents sérotypes de virus.

5. Système de vecteur multiple AAV selon l'une quelconque des revendications précédentes, le signal donneur d'épissage comprenant ou étant constitué d'une séquence qui est au moins 70 %, 75 %, 80 %, 85 %, 90 %, 95 % ou 100 % identique à
GTAAGTATCAAGGTTACAAGACAGGTTAAGGAGACCAATAGAAACTGGGCTTGTC GAGACAGAGAAGACTCTTGCGTTTCT (SEQ ID No. 27),
ou le signal accepteur d'épissage comprenant ou étant constitué d'une séquence qui est au moins 70 %, 75 %, 80 %, 85 %, 90 %, 95 % ou 100 % identique à GATAGGCACCTATTGGTCTTACTGACATCCACTTTGCCTTTCTCTCCACAG (SEQ ID No. 28)

6. Système de vecteur multiple AAV selon l'une quelconque des revendications précédentes, la séquence codante étant la séquence codante d'un gène choisi dans le groupe constitué par : ABCA4, MYO7A, CEP290, CDH23, EYS, PCDH15, CACNA1, SNRNP200, RP1, PRPF8, RP1L1, ALMS1, USH2A, GPR98, HMCN1, préférablement la séquence codante étant la séquence codante d'un gène choisi dans le groupe constitué par : DMD, CFTR, F8 et DYSF.

7. Système de vecteur multiple AAV selon l'une quelconque des revendications précédentes, le premier vecteur ne comprenant pas de séquence nucléotidique de signal de polyadénylation.

8. Système de vecteur multiple AAV selon l'une quelconque des revendications précédentes comprenant :
a) un premier vecteur comprenant dans une direction 5'-3' :
- une séquence de répétition 5'-terminale inversée (5'-ITR) ;
- une séquence promoteur ;
- une partie terminale 5' d'une séquence codante d'un gène d'intérêt (CDS1), ladite partie terminale 5' étant liée de manière fonctionnelle à et sous contrôle dudit promoteur ;
- une séquence nucléotidique d'un signal donneur d'épissage ;
- une séquence nucléotidique d'une région recombinogénique ; et
- une séquence de répétition 3'-terminale inversée (3'-ITR) ; et
b) un deuxième vecteur comprenant dans une direction 5'-3' :
- une séquence de répétition 5'-terminale inversée (5'-ITR) ;
- une séquence nucléotidique d'une région recombinogénique ;
- une séquence nucléotidique d'un signal accepteur d'épissage ;
- la partie terminale 3' de la séquence codante (CDS2),
- une séquence nucléotidique de signal de polyadénylation ; et
- une séquence de répétition 3'-terminale inversée (3'-ITR),
**caractérisé par le fait qu'**il comprend en outre une séquence nucléotidique d'un signal de dégradation, ladite séquence étant localisée au niveau de l'extrémité 5' ou de l'extrémité 3' de la séquence nucléotidique de la région recombinogénique de l'un ou des deux parmi le premier et le deuxième vecteur,
- la séquence recombinogénique étant choisie dans le groupe constitué par :
GGGATTTTGCCGATTTCGGCCTATTGGTTAAAAAATGAGCTGATTTAACAAAAATTT AACGCGAATTTTAACAAAAT (SEQ ID No. 22, AK) ou
GGGATTTTTCCGATTTCGGCCTATTGGTTAAAAAATGAGCTGATTTAACAAAAATTT AACGCGAATTTTAACAAAAT (SEQ ID NO. 23, AK), SEQ ID NO. 24 (AP1), SEQ ID NO. 25 (AP2), et SEQ ID NO. 26 (AP) ^{et}
- la séquence nucléotidique du signal de dégradation comprenant ou étant constituée d'une séquence codant SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16),
ou la séquence nucléotidique d'un signal de dégradation comprenant ou étant constituée de la SEQ ID NO: 16.

9. Système de vecteur multiple AAV selon l'une quelconque des revendications précédentes comprenant en outre un troisième vecteur comprenant une troisième partie de ladite séquence codante (CDS3) et une séquence de reconstitution, le deuxième vecteur comprenant deux séquences de reconstitution, chaque séquence de reconstitution étant située au niveau de chaque terminaison de CDS2, préférablement le troisième vecteur comprenant en outre au moins une séquence nucléotidique d'un signal de dégradation, préférablement le deuxième vecteur comprenant en outre une séquence nucléotidique de signal de polyadénylation liée à la partie terminale 3' de ladite séquence codante (CDS2).

10. Cellule hôte comprenant le système de vecteur multiple AAV selon l'une quelconque des revendications précédentes.

11. Système de vecteur multiple AAV selon l'une quelconque des revendications 1 à 9 ou cellule hôte selon la revendication 10 pour une utilisation médicale, préférablement pour une utilisation en thérapie génique, préférablement pour une utilisation dans le traitement et/ou la prévention d'une pathologie ou maladie **caractérisée par** une dégénérescence rétinienne, préférablement la dégénérescence rétinienne étant héritée, préférablement la pathologie ou maladie étant choisie dans le groupe constitué par : la rétinite pigmentaire (RP), l'amaurose congénitale de Leber (LCA), la maladie de Stargardt (STGD), la maladie d'Usher (USH), le syndrome d'Alstrom, la cécité nocturne stationnaire congénitale (CSNB), la dystrophie maculaire, la dystrophie maculaire occulte, une maladie causée par une mutation dans le gène *ABCA4,* préférablement pour une utilisation dans la prévention et/ou le traitement de la dystrophie musculaire de Duchenne, de la fibrose kystique, de l'hémophilie A et de dysferlinopathies.

12. Composition pharmaceutique comprenant le système de vecteur multiple AAV selon l'une quelconque des revendications 1 à 9 ou la cellule hôte selon la revendication 10 et un véhicule pharmaceutiquement acceptable.

13. Séquence nucléotidique d'un signal de dégradation qui comprend ou est constituée d'une séquence codant pour
SEQ ID No. 1 (CL1), SEQ ID No. 2 (CL2), SEQ ID No. 3 (CL6), SEQ ID No. 4 (CL9), SEQ ID No. 5 (CL10), SEQ ID No. 6 (CL11), SEQ ID No. 7 (CL12), SEQ ID No. 8 (CL15), SEQ ID No. 9 (CL16),
ou qui comprend ou est constituée de la SEQ ID NO: 16 dans un système de vecteur multiple AAV pour une utilisation dans un procédé pour diminuer l'expression d'une protéine sous forme tronquée ou pour une utilisation dans un procédé pour diminuer l'expression d'une protéine sous forme tronquée comprenant l'insertion de ladite séquence nucléotidique d'un signal de dégradation dans un ou plusieurs vecteurs d'un système de vecteur multiple AAV.
